(19) Europäisches Patentamt
European Patent Office
Office européen des brevets

(11) EP 0 694 536 A1

(12) **DEMANDE DE BREVET EUROPEEN**

(43) Date de publication:
**31.01.1996 Bulletin 1996/05**

(51) Int Cl.[6]: **C07D 235/26**, A61K 31/415

(21) Numéro de dépôt: **95401599.6**

(22) Date de dépôt: **04.07.1995**

(84) Etats contractants désignés:
**AT BE CH DE DK ES FR GB GR IE IT LI LU MC NL PT SE**

(30) Priorité: **05.07.1994 FR 9408278**

(71) Demandeur: **SANOFI**
**F-75008 Paris (FR)**

(72) Inventeurs:
- **Di Malta, Alain**
**F-34980 Saint Clement de Riviere (FR)**
- **Mettefeu, Daniel**
**F-34790 Grabels (FR)**
- **Garcia, Georges**
**F-34980 Saint Gely du Fesc (FR)**
- **Roux, Richard**
**F-34570 Vailhauques (FR)**
- **Serradeil-Legal, Claudine**
**F-31570 Escalquens (FR)**

(74) Mandataire: **Gillard, Marie-Louise et al**
**F-75340 Paris Cédex 07 (FR)**

(54) **Dérivés de 1-benzyl-1,3-dihydro-2H-benzimidazol-2-one, leur préparation, et compositions pharmaceutiques les contenant**

(57) La présente invention concerne des dérivés de 1-benzyl-1,3-dihydro-*2H*-benzimidazol-2-one, de formule :

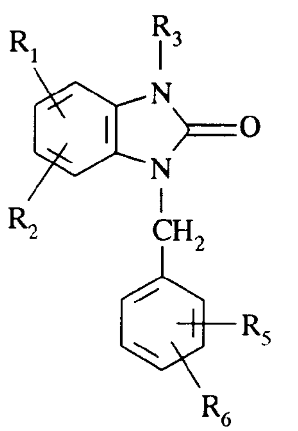

(I)

et leurs sels éventuels, leur procédé de préparation ainsi que les compositions pharmaceutiques en contenant.

Ces composés ont une affinité pour les récepteurs de la vasopressine et/ou de l'ocytocine.

EP 0 694 536 A1

## Description

La présente invention a pour objet des dérivés de 1-benzyl-1,3-dihydro-2*H*-benzimidazol-2-one, leur préparation et les compositions pharmaceutiques en contenant.

Les composés selon la présente invention ont une affinité pour les récepteurs de la vasopressine et/ou de l'ocytocine.

La vasopressine est une hormone connue pour son effet antidiurétique et son effet dans la régulation de la pression artérielle. Elle stimule plusieurs types de récepteurs : $V_1$ ($V_{1a}$, $V_{1b}$), $V_2$. Ces récepteurs sont localisés dans le foie, les vaisseaux (coronaires, rénaux, cérébraux), les plaquettes, le rein, l'utérus, les glandes surrénales, le système nerveux central, l'hypophyse. L'ocytocine a une structure peptidique proche de celle de la vasopressine. Les récepteurs de l'ocytocine se trouvent aussi sur le muscle lisse de l'utérus, ils se trouvent également sur des cellules myoépithéliales de la glande mammaire, dans le système nerveux central et dans le rein. La localisation des différents récepteurs est décrite dans : S. JARS et al., Vasopressin and oxytocin receptors : an overview, *in* Progress in Endocrinology. H. IMURA and K. SHIZURNE ed., Experta Medica, Amsterdam, 1988, 1183-1188, ainsi que dans les articles suivants : Presse Médicale, 1987, 16 (10), 481-485, J. Lab. Clin. Med., 1989, 114 (6), 617-632 et Pharmacol. Rev., 1991, 43 (1), 73-108. La vasopressine exerce ainsi des effets cardiovasculaires, hépatiques, antidiurétiques, agrégants et des effets sur le système nerveux central et périphérique, et sur la sphère utérine. L'ocytocine intervient dans la parturition, la lactation et le comportement sexuel.

Les composés selon la présente invention permettent soit de mimer les effets de l'hormone (pour les agonistes), soit de les inhiber (pour les antagonistes), de façon sélective. Les antagonistes des récepteurs de la vasopressine peuvent intervenir sur la régulation de la circulation centrale et périphérique, notamment les circulations coronaire, rénale et gastrique, ainsi que sur la régulation hydrique et la libération de l'hormone adrénocorticotrophique (ACTH). Les agonistes de la vasopressine peuvent remplacer avantageusement la vasopressine ou ses analogues dans le traitement du diabète insipide ; ils peuvent également être utilisés dans le traitement de l'énurésie, et dans la régulation de l'hémostase : traitement de l'hémophilie, du syndrome de Von Willebrand, antidote des agrégants plaquettaires (F.A. LASZLO, , Pharmacol. Rev., 1991, 43, 73-108. Drug Investigation, 1990, 2 (Suppl. 5), 1-47). Les hormones elles-mêmes : la vasopressine et l'ocytocine ainsi que certains de leurs analogues peptidiques ou non peptidiques sont utilisés en thérapeutique et ont montré leur efficacité. On peut citer plusieurs revues et de nombreux articles de la littérature : Vasopressin, P. GROSS et al., ed. John Libbey Eurotext, 1993, en particulier p. 243-257 et p. 549-562 ; F.A. LASZLO and F.A. LASZLO Jr., Clinical perspectives for vasopressin antagonists, Drug News Perspect., 1993, 6 (8) ; W.G. NORTH, J. Clin. Endocrinol., 1991, 73, 1316-1320 ; J.J. LEGROS et al., Prog. Neuro-Pharmacol. Biol. Psychiat., 1988, 12, 571-586 ; K.E. ANDERSSON et al., Drugs Today, 1988, 24 (7) 509-528 ; D.L. STUMP et al., Drugs, 1990, 39, 38-53 ; S. CALTABIANO et al., Drugs Future, 1988, 13, 25-30 ; Y. MURA et al., Clin. Nephrol. 1993, 40, 60-61 ; Faseb J., 1994, 8 (5), A 587 : 3398.

Ainsi les composés selon l'invention sont utiles notamment dans le traitement des affections du système nerveux central et périphérique, du système cardiovasculaire, de la sphère rénale, de la sphère gastrique et dans les troubles du comportement sexuel, chez l'homme et chez l'animal.

Des dérivés de 1-benzyl-1,3-dihydro-2*H*-benzimidazol-2-one ont été préparés et ont montré une activité pharmacologique. On peut citer par exemple les brevets ou demandes de brevets suivants :

- FR 1573739 ; pharmacologie non spécifiée ;
- DE 2400094, concerne des composés à activité anti-hypertensive ;
- DE 2527261, concerne des composés à activité neuroleptique ;
- DE 2714437, concerne des composés à activité antiallergique ;
- EP 260744, concerne des composés à activité inhibitrice de la synthèse d'androgène à partir de stéroïdes ;
- JP 57018674, concerne des composés à activité dépressive du système nerveux central, antihistaminique, antiagrégant plaquettaire ou inhibitrice de phospho-diesterase ;
- DE 2717439, concerne des composés à activité système nerveux central, possédant des propriétés antidépressives ;
- DE 2338813, concerne des composés à activité anti-inflammatoire ;

- DE 2626128, concerne des composés à activité anti-hypertensive ;
- JP 53009770, concerne des composés à activité analgésique et dépressive centrale ;
- EP 254627, concerne des composés à activité anti-histaminique et antispasmodique ;
- US 4209527, concerne des composés à activité inhibitrice de l'aldose réductase ;
- EP 51827, concerne des composés à activité antiallergique, anti-asthmatique et antiinflammatoire.

On peut citer également les publications suivantes :

- Eur. J. Pharmacol., Mol. Pharmacol. Sect., 1992, 226 (2), 109-120, concerne des composés antagonistes de la dopamine ;
- Eur. J. Med. Chem., 1992, 27 (8), 779-789, concerne des composés à activité inhibitrice de l'aldose réductase ;
- Eur. J. Med. Chem. - Chim Ther., 1981, 16 (4), 321-326, concerne des composés à activité antiulcéreuse et antisécrétrice.

La demande de brevet EP 454 330 décrit notamment, comme intermédiaire de synthèse, un dérivé de 1,3-dihydro-2*H*-benzimidazol-2-one de formule :

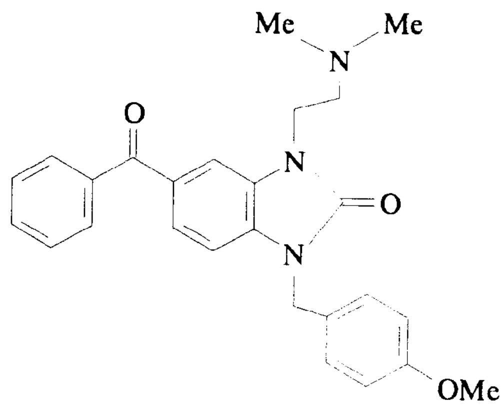

1

Récemment plusieurs demandes de brevet ont décrit des familles de composés à structure non peptidiques ayant une activité sur les récepteurs de la vasopressine et/ou de l'ocytocine. On peut citer les demandes EP 382 185, EP 444 945, EP 514 667, EP 469 984, EP 526 348, les demandes WO 91/05549 et WO 93/15051 et la demande de brevet JP-03/127732.

Selon l'un de ses aspects la présente invention a pour objet des composés de formule :

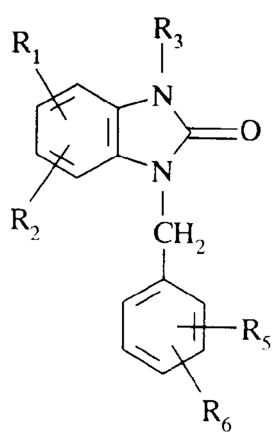

(I)

dans laquelle :

- $R_1$ représente un halogène ; un $(C_1-C_7)$alkyle ; un $(C_1-C_7)$alkylthio ; un phénylthio ; un trifluorométhyle ; un cyano ; un nitro ; un groupe $-NR_7R_8$ ; un hydroxy ; un $(C_1-C_7)$alcoxy ; un $(C_3-C_7)$cycloalkyloxy ; un $(C_3-C_7)$ cycloalkylméthoxy ; un phénoxy ; un benzyloxy ; un ω-halogéno$(C_1-C_7)$alkyloxy ; un polyhalogéno$(C_1-C_7)$alkyloxy ; un ω-hydroxy$(C_2-C_7)$alkyloxy ; un ω-méthoxy$(C_2-C_7)$alkyloxy ;

- $R_2$ représente un hydrogène, un halogène, un $(C_1-C_7)$alkyle ;

- $R_3$ représente $R_4$ ; un groupe-$(CH_2)_p$-$R_4$ ; un indanyle ; un hexahydroindanyle ; un adamantyle ; un noradamantyle ; un norbornyle ; un $(C_1-C_8)$alkyle non substitué ou substitué par un $(C_1-C_4)$alcoxy ; un cyclohexyle substitué par un di$(C_1-C_7)$alkylamino, un carboxy, un $(C_1-C_4)$alcoxycarbonyle, un hydroxy, un tetrahydropyran-2-yloxy, un $(C_1-C_4)$ alcoxy$(C_1-C_4)$alcoxy ou un phényl$(C_1-C_2)$alcoxy$(C_1-C_4)$alcoxy ;

- $R_4$ représente un groupe $-NR_9R_{10}$ ; un $(C_3-C_7)$cycloalkyle non substitué ou substitué une ou deux fois par un $(C_1-C_4)$ alkyle ou un $(C_1-C_4)$alcoxy ; un furyle ; un thiényle ; un pyrrolyle ; un triazolyle ; un tétrazolyle ; un pyridyle ; un pyridyle N-oxyde ; un pyrimidinyle ; un pyrazolyle ; un pyrazinyle ; un tétrahydropyran-4-yle ; un azétidin-3-yle substitué en position 1 par $R_{11}$ ; un pipérid-4-yle substitué en position 1 par $R_{11}$ ; un groupe Ar ;

- $R_5$ représente un hydrogène ; un $(C_1-C_7)$alkyle ; un $(C_1-C_7)$alcoxy ; un halogène ; un hydroxy ; un trifluorométhyle ;

- $R_6$ représente un cyano ; un groupe $-CH_2NR_7R_8$ ; un nitro ; un groupe $-NR_{12}R_{13}$ ; un groupe -NHOH ; un guanidino non substitué ou substitué en position 1 par un $(C_1-C_7)$alkyle et/ou en position 3 par un ou deux $(C_1-C_7)$alkyles, un groupe Ar ou un groupe $-CH_2$-Ar et/ou en position 2 par un cyano ; un groupe $-OR_{14}$ ; un groupe $-SR_{14}$ ; un $(C_1-C_7)$ alkylcarbonyle ; un groupe $-CONR_{15}R_{16}$ ; un thiocarbamoyle libre ou substitué par un ou deux $(C_1-C_7)$alkyles ; un carboxy ; un $(C_1-C_7)$alcoxycarbonyle ; un groupe -COO-Ar ; un groupe $-COO-CH_2$-Ar ; un groupe $-CO-NH-CR_{17}R_{18}-COR_{19}$ ; un groupe $-SO_2NR_{20}R_{21}$ ; un groupe $-NHSO_2-(C_1-C_7)$alkyle ; un groupe $-NHSO_2$-Ar ; un groupe $-NHSO_2-CH_2$-Ar ; un diméthylaminosulfonamido ;

- $R_7$ et $R_8$ représentent chacun indépendamment un hydrogène ou un $(C_1-C_7)$alkyle ; $R_8$ peut de plus représenter un groupe protecteur ;

- $R_9$ et $R_{10}$ représentent chacun indépendamment un hydrogène ou un $(C_1-C_7)$alkyle ;

- ou bien $R_9$ et $R_{10}$ ensemble avec l'atome d'azote auquel ils sont liés constituent un hétérocycle choisi parmi la morpholine, la thiomorpholine, l'azétidine, la pyrrolidine, la pipéridine, la pipérazine substituée en position 4 par $R_{11}$ ou la perhydroazépine ;

- $R_{11}$ représente un hydrogène ; un $(C_1-C_7)$alkyle ; un phényle ; un benzyle ; un $(C_1-C_7)$alkylcarbonyle ; un benzoyle ; un $(C_1-C_7)$alcoxycarbonyle ; un phénoxycarbonyle ; un carbamoyle non substitué ou substitué par un ou deux $(C_1-C_7)$alkyles ;

- $R_{12}$ et $R_{13}$ représentent chacun indépendamment un hydrogène ; un $(C_1-C_7)$alkyle ; un groupe $-CH_2$-Ar ; $R_{13}$ peut de plus représenter un $(C_3-C_7)$cycloalkylméthyle ; un groupe Ar ; un groupe $-CH_2CH_2Ar$ ; un $(C_3-C_8)$alcényle ; un $(C_1-C_7)$alkylcarbonyle ; un $(C_1-C_7)$alkylthiocarbonyle ; un $(C_3-C_7)$cycloalkylcarbonyle ; un $(C_3-C_7)$ cycloalkylthiocarbonyle ; un groupe -CO-Ar ; un groupe $-CO-CH_2$-Ar ; un ω-$R_7R_8N(C_2-C_7)$alkylcarbonyle ; un ω-hydroxy$(C_1-C_7)$alkylcarbonyle ; un ω-benzyloxy$(C_1-C_7)$alkylcarbonyle ; un pyridylcarbonyle ; un méthylpyridylcarbonyle ; un thiénylcarbonyle ; un furylcarbonyle ; un pipérid-4-ylcarbonyle substitué en position 1 par $R_{11}$ ; un $(C_1-C_7)$alcoxycarbonyle ; un phénoxycarbonyle ; un phénoxythiocarbonyle ; un benzyloxycarbonyle ; un groupe $-CONR_{22}R_{23}$ ; un groupe $-CSNR_{22}R_{23}$ ; un groupe $-CO-CR_{17}R_{18}-NR_7R_8$ ; un groupe $-CR_{17}R_{18}COR_{19}$ ; un groupe $-(CH_2)_t-COR_{19}$ ; un groupe $-CO-(CH_2)_u-COR_{19}$ ;

- ou bien $R_{12}$ et $R_{13}$ ensemble avec l'atome d'azote auquel ils sont liés constituent l'hydantoïne, la N-méthylhydantoïne, ou un radical hétérocyclique choisi parmi le pyrrol-1-yle, le Δ3-pyrrolin-1-yle, la pyrrolidin-1-yle, l'isoindolin-2-yle dans lequel le noyau benzénique est non substitué ou substitué par un halogène, un $(C_1-C_7)$alkyle, un trifluorométhyle ou un $(C_1-C_7)$alcoxy ;

- $R_{14}$ représente l'hydrogène ; un $(C_1-C_7)$alkyle ; un phényle ; un benzyle ; un $(C_3-C_7)$cycloalkyle ; un $(C_3-C_7)$ alcényle ; un ω-halogéno$(C_2-C_7)$alkyle ; un polyhalogéno$(C_1-C_7)$alkyle ; un ω-hydroxy$(C_2-C_7)$alkyle ; un $(C_1-C_7)$ alkylcarbonyle ; un benzoyle ; un ω-carboxy$(C_1-C_7)$alkyle ; un ω-$(C_1-C_4)$alcoxycarbonyl$(C_1-C_7)$alkyle ; un ω-benzy-

loxycarbonyl($C_1$-$C_7$)alkyle ; un ω-$R_7R_8N$($C_2$-$C_7$)alkyle ; un ω-carbamoyl($C_1$-$C_7$)alkyle dans lequel le carbamoyle est libre ou substitué par un ou deux ($C_1$-$C_7$)alkyles ;

- $R_{15}$ et $R_{16}$ représentent chacun indépendamment un hydrogène ou un ($C_1$-$C_7$)alkyle ; $R_{16}$ peut de plus représenter un ($C_3$-$C_7$)cycloalkyle non substitué ou substitué par un ($C_1$-$C_4$)alkyle ; un groupe Ar ; un pyridyle ; un méthylpyridyle ; un pipérid-4-yle substitué en position 1 par $R_{11}$ ; un méthylpipérid-4-yle ; un pyrrolidin-1-yle ; un pipérid-1-yle ; un morpholin-4-yle ; un ($C_1$-$C_7$)alkyle substitué par un ou plusieurs halogènes ou par $R_{24}$ ;
- ou bien $R_{15}$ et $R_{16}$ ensemble avec l'atome d'azote auquel ils sont liés représentent un radical hétérocyclique $R_{25}$ ;
- $R_{17}$ et $R_{18}$ représentent chacun indépendamment l'hydrogène ; un ($C_1$-$C_7$)alkyle ; un benzyle ;
- ou bien $R_{17}$ et $R_{18}$ ensemble avec l'atome de carbone auquel ils sont liés constituent un ($C_3$-$C_7$)cycloalkyle ;
- $R_{19}$ représente un hydroxy ; un ($C_1$-$C_7$)alcoxy ; un amino libre ou substitué par un ou deux ($C_1$-$C_7$)alkyles ;
- $R_{20}$ et $R_{21}$ représentent chacun indépendamment l'hydrogène ; un ($C_1$-$C_7$)alkyle ; $R_{21}$ peut de plus représenter un ($C_3$-$C_7$)cycloalkyle ;
- ou bien $R_{20}$ et $R_{21}$ ensemble avec l'atome d'azote auquel ils sont liés constituent un radical hétérocyclique $R_{25}$ ;
- $R_{22}$ et $R_{23}$ représentent chacun indépendamment l'hydrogène ; un ($C_1$-$C_7$)alkyle ; $R_{23}$ peut de plus représenter un ($C_3$-$C_7$)cycloalkyle ; un adamantyle ; un groupe Ar ; un hydroxy ; un ($C_1$-$C_4$)alcoxy ; un ($C_1$-$C_7$)alkyle substitué par un groupe Ar, un pyridyle, un hydroxy, un ($C_1$-$C_7$)alcoxy, un groupe-$NR_7R_8$, un carboxy ou un ($C_1$-$C_7$) alcoxycarbonyle ;
- ou bien $R_{22}$ et $R_{23}$ ensemble avec l'atome d'azote auquel ils sont liés constituent un radical hétérocyclique $R_{25}$ ;
- $R_{24}$ représente un hydroxy ; un ($C_1$-$C_7$)alcoxy ; un cyano ; un carboxy ; un ($C_1$-$C_7$)alcoxycarbonyle ; un groupe -$NR_7R_8$ ; un carbamoyle libre ou substitué par un ou deux ($C_1$-$C_7$)alkyles ; un benzyloxycarbonyle ; un groupe Ar ; un ($C_3$-$C_7$)cycloalkyle ; un adamantyle ; un pyrrolidin-1-ylcarbonyle ; un pipérid-1-ylcarbonyle ; un perhydroazépin-1-ylcarbonyle ; un radical hétérocyclique choisi parmi un pyridyle, un méthylpyridyle, un furanyle, un tétrahydrofuranyle, un thiényle, un méthylthiényle, un pyrrolin-1-yle, un pipérid-1-yle, un perhydroazépin-1-yle ;
- $R_{25}$ représente un morpholin-4-yle ; un thiomorpholine-4-yle ; un azétidin-1-yle non substitué ou substitué en position 2 par un carboxy, un ($C_1$-$C_4$)alcoxycarbonyle ou en position 3 par un groupe -$NR_7R_8$, un ($C_1$-$C_7$)alkyle, un phényle, un benzyle ou un ($C_1$-$C_7$)alkylcarbonyle ; un perhydroazépin-1-yle ; un pipérazin-1-yle non substitué ou substitué en position 4 par un ($C_1$-$C_7$)alkyle, un phényle, un benzyle, un ($C_1$-$C_7$)alkylcarbonyle, un ($C_1$-$C_7$)alcoxycarbonyle ou un benzyloxycarbonyle ; un pipérid-1-yle non substitué ou substitué en position 4 par un ($C_1$-$C_7$)alkyle, un phényle, un benzyle, un ($C_1$-$C_7$)alkylcarbonyle, un groupe -$NR_7R_8$ ; un cis-2,6-diméthylpipérid-1-yle ; un pyrrolidin-1-yle non substitué ou substitué par un ($C_1$-$C_7$)alkyle, un phényle, un benzyle, un ($C_1$-$C_7$)alkylcarbonyle, un hydroxyméthyle, un carboxy, un ($C_1$-$C_7$)alcoxycarbonyle, un carbamoyle non substitué ou substitué par un ou deux ($C_1$-$C_7$)alkyles ;
- Ar représente un phényle non substitué ou substitué une ou plusieurs fois par un substituant choisi parmi : un atome d'halogène, un ($C_1$-$C_7$)alkyle, un trifluorométhyle, un hydroxy, un ($C_1$-$C_7$)alcoxy, un carboxy, un ($C_1$-$C_7$)alcoxycarbonyle, un ($C_1$-$C_7$)alkylcarbonyloxy, un nitro, un cyano, un amino, un ($C_1$-$C_7$)alkylamino, un di($C_1$-$C_7$)alkylamino, lesdits substituants étant identiques ou différents ;
- t représente un nombre entier qui peut varier de 2 à 5 ;
- u représente un nombre entier qui peut varier de 0 à 7 ;
- p représente un nombre entier qui peut varier de 1 à 8

ainsi que leurs sels éventuels,
à la condition que lorsque $R_6$ représente un méthoxy, $R_5$ est autre que l'hydrogène.

Lorsqu'un composé selon l'invention présente un ou des carbones asymétriques, l'invention comprend tous les

isomères optiques de ce composé.

Les sels des composés de formule (I) selon la présente invention comprennent ceux avec des acides minéraux ou organiques qui permettent une séparation ou une cristallisation convenable des composés de formule (I), tels que l'acide picrique, l'acide oxalique ou un acide optiquement actif, par exemple un acide mandélique ou un acide camphosulfonique, et ceux qui forment des sels physiologiquement acceptables tels que le chlorhydrate, le bromhydrate, le sulfate, l'hydrogénosulfate, le dihydrogénophosphate, le maléate, le fumarate, le 2-naphtalènesulfonate.

Les sels des composés de formule (I) comprennent également les sels avec des bases organiques ou minérales, par exemple les sels des métaux alcalins ou alcalinoterreux comme les sels de sodium, de potassium, de calcium, les sels de sodium et de potassium étant préférés, ou avec une amine, telle que le trométamol, ou bien les sels d'arginine, de lysine, ou de toute amine physiologiquement acceptable.

Selon la présente invention, par halogène on entend un atome choisi parmi le fluor, le chlore, le brome ou l'iode, de préférence le fluor ou le chlore.

Selon la présente invention par groupe protecteur de l'azote on entend un groupe tel que : un ($C_1$-$C_4$) alkyle, par exemple un méthyle ou un *tert*-butyle ; un benzyle ; un benzyle substitué tel que p-nitrobenzyle, p-chlorobenzyle, p-méthoxybenzyle ; un benzhydryle ; un trityle ; un benzoyle ; un ($C_1$-$C_4$)alkylcarbonyle, par exemple un acétyle ; un ($C_1$-$C_4$)alcoxycarbonyle, par exemple un méthoxycarbonyle, un éthoxycarbonyle ou un *tert*-butoxycarbonyle ; un benzyloxycarbonyle.

Selon la présente invention par alkyle en $C_1$-$C_7$ ou respectivement en $C_1$-$C_8$ ou en $C_1$-$C_4$ on entend un alkyle droit ou ramifié en $C_1$-$C_7$ ou respectivement en $C_1$-$C_8$ ou en $C_1$-$C_4$. Par alcoxy en $C_1$-$C_7$ ou respectivement en $C_1$-$C_4$ on entend un alcoxy droit ou ramifié en $C_1$-$C_7$ ou respectivement en $C_1$-$C_4$.

Par convention, dans la description qui va suivre et dans les revendications, l'hétérocycle 1,3-dihydro-2*H*-benzimidazol-2-one est numéroté comme suit pour les composés selon l'invention :

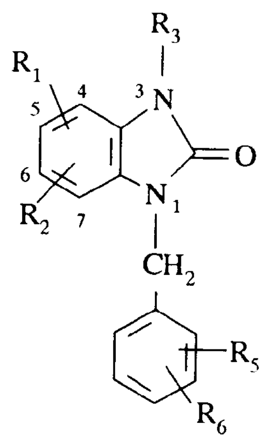

(I)

De façon avantageuse, la présente invention a pour objet des composés de formule :

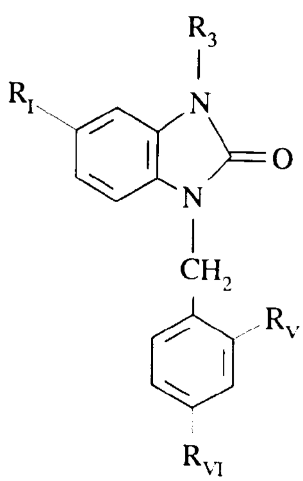

(Ia)

dans laquelle :

- $R_I$ représente un ($C_1$-$C_4$)alcoxy, un atome de chlore ou de fluor,
- $R_V$ représente l'hydrogène ou un méthoxy,

- $R_{VI}$ représente un $(C_1-C_7)$alkylcarboxamido, un groupe -NHCO-Ar, un groupe $-CONR_{15}R_{16}$, un groupe $-NR_{12}CONR_{22}R_{23}$ ;

et les substituants $R_3$, Ar, $R_{12}$, $R_{15}$, $R_{16}$, $R_{22}$, $R_{23}$ sont tels que définis ci-dessus pour les composés de formule (I) ; ainsi que leurs sels.

Les composés de formule (Ia) dans lesquels $R_3$ représente un cyclohexyle ou un groupe Ar sont des composés préférés.

Dans la description et dans les exemples, les abréviations suivantes sont utilisées.

DCM : dichlorométhane
Ether : éther diéthylique
Ether iso : éther diisopropylique
$CCl_4$ : tétrachlorure de carbone
MeOH : méthanol
EtOH : éthanol
AcOEt : acétate d'éthyle
DMF : diméthylformamide
THF : tétrahydrofurane
DIPEA : diisopropyléthylamine
AcOH : acide acétique
HCl : acide chlorhydrique
TFA : acide trifluoroacétique
$Na_2SO_4$ : sulfate de sodium
NaOH : soude
$NaHCO_3$ : hydrogénocarbonate de sodium
$K_2CO_3$ : carbonate de potassium
Boc : *tert*-butoxycarbonyle
Me, OMe : méthyle, méthoxy
Et, OEt : éthyle, éthoxy
Pr, iPr : n-propyle, isopropyle
Bu, iBu, tBu : butyle, isobutyle, *tert*-butyle
THP : tetrahydropyran-2-yle
BOP : hexafluorophosphate de benzotriazol-1-yloxy-tris(diméthylamino)phosponium
Réactif de Lawesson : 2,4-bis-(4-méthoxyphényl)-1,3-dithia-2,4-diphosphétane-2,4-disulfure
Eb : point d'ébullition
F : point de fusion
TA : température ambiante
RMN : résonnance magnétique nucléaire
s : singulet
se : singulet élargi
d : doublet
t : triplet
q : quadruplet
m : massif
mt : multiplet
t de d : triplet de doublet
d de d : doublet de doublet.

La présente invention a également pour objet un procédé de préparation des composés selon l'invention et de leurs sels éventuels, caractérisé en ce que :

1) on fait agir sur un composé de formule :

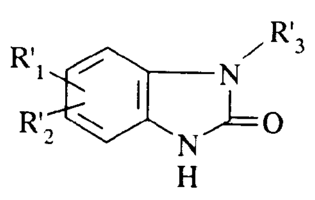

(II)

dans laquelle $R'_1$, $R'_2$ et $R'_3$ représentent, respectivement, soit $R_1$, $R_2$ et $R_3$ tels que définis pour (I), soit des groupes

précurseurs de $R_1$, $R_2$ et $R_3$, un halogénure de benzyle de formule :

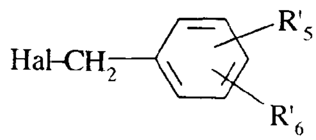

(III)

dans laquelle Hal représente un atome d'halogène, de préférence le chlore ou le brome et $R'_5$ et $R'_6$ représentent, respectivement, soit $R_5$ et $R_6$ tels que définis ci-dessus pour (I), soit des groupes précurseurs de $R_5$ et $R_6$ ; et,

2) soit, lorsque $R'_1 = R_1$, $R'_2 = R_2$, $R'_3 = R_3$, $R'_5 = R_5$ et $R'_6 = R_6$, on isole le composé de formule (I) ainsi obtenu ;

3) soit, lorsque l'un quelconque des groupes $R'_1$, $R'_2$, $R'_3$, $R'_5$ et/ou $R'_6$ représente respectivement un groupe précurseur de $R_1$, $R_2$, $R_3$, $R_5$ et/ou $R_6$, on soumet le composé obtenu, à l'étape 1, à un traitement ultérieur pour préparer le composé de formule (I) par transformation de l'un quelconque des groupes $R'_1$, $R'_2$, $R'_3$, $R'_5$ et/ou $R'_6$ en, respectivement, $R_1$, $R_2$, $R_3$, $R_5$ et/ou $R_6$ ;

4) éventuellement, on transforme le composé obtenu à l'étape 2) ou à l'étape 3), en l'un de ses sels.

On appelle composé (I'), un composé portant un substituant $R'_1$, $R'_2$, $R'_3$, $R'_5$ et/ou $R'_6$ précurseurs de $R_1$, $R_2$, $R_3$, $R_5$ et/ou $R_6$.

La transformation d'un substituant $R'_1$, $R'_2$, $R'_3$, $R'_5$ et/ou $R'_6$ en respectivement $R_1$, $R_2$, $R_3$, $R_5$ et/ou $R_6$ peut être effectuée soit à partir du composé de formule (I'), soit à partir d'un des composés intermédiaires utiles dans la préparation de (I).

La réaction de l'étape 1) est effectuée dans un solvant anhydre tel que le DMF ou le THF, en présence d'un hydrure métallique tel que l'hydrure de sodium par exemple, ou en présence d'un alcoolate comme le *tert*-butylate de potassium.

Les dérivés de benzimidazol-2-ones (II) sont connus ou peuvent être préparés selon différents modes opératoires par des méthodes connues.

On peut préparer des dérivés de benzimidazol-2-ones N-substitués (II), utiles comme produits de départ pour la préparation des composés selon l'invention, d'après des procédés décrits dans les brevets GB 2 127 408, GB 1 575 386 ; demandes de brevet JP 62-249 982, JP 53-009 770, JP 51-131 875 ; brevets BE 770 911, BE 859 415, BE 830 403 ; demandes de brevet EP 477 819, EP 454 330, EP 526 434.

De même les publications suivantes décrivent des dérivés de benzimidazol-2-ones N-substitués :

Monatsh. Chem., 1985, 116 (5), 639-644.

Eur. J. Med. Chem. - Chim. Ther., 1983, 18 (6), 495-500.

On peut également préparer des dérivés de benzimidazol-2-ones N-substitués par des méthodes telles que celles décrites dans les publications suivantes :

Pharmazie, 1979, 34 (9), 576.

Pol. J. Chem., 1979, 53 (9), 1883-1887.

J. Heterocycl. Chem., 1970, 7 (4), 807-813.

Eur. J. Med. Chem. - Chim. Ther., 1981, 16 (4), 321-326.

Selon un mode de réalisation particulier, les benzimidazol-2-ones peuvent être préparés selon le procédé décrit dans Eur. J. Med. Chem. - Chim. Ther., 1981, 16 (4), 321-326, de la manière suivante.

Par réaction en solution alcoolique, en présence ou non d'une base telle que la triéthylamine, à température ambiante ou à reflux, d'une amine primaire de formule :

$$H_2N\text{-}R'_3 \qquad (IV)$$

avec des ortho-dinitro ou ortho-chloro-nitrobenzènes (V) substitués de formules :

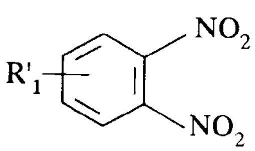

ou

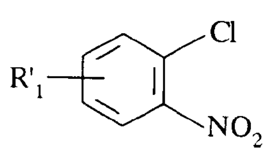

(V)

dans lesquelles $R'_1$ est différent d'un groupe nitro,

on obtient les 2-nitroanilines N-substituées de formule :

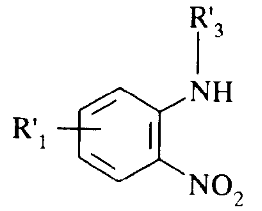

(VI)

Les composés (VI) peuvent également être obtenus par chauffage des composés (IV) et (V) dans le 2-éthoxyéthanol (J. Chem. Soc. 1960, 314-318) ou dans l'ethylène glycol en présence d'acétate de sodium ou dans le 1,2,3,4-tétraméthylbenzène ou dans la Décaline®.

Les composés (VI) dans lesquels $R'_1$ est un groupe $(C_1\text{-}C_7)$alcoxy, $(C_3\text{-}C_7)$cycloalkyloxy, $(C_3\text{-}C_7)$cycloalkylméthoxy, phénoxy, benzyloxy, ou ω-méthoxy$(C_2\text{-}C_7)$alcoxy sont obtenus par réaction d'un composé (VI) dans lequel le substituant $R'_1$ est un atome de chlore avec un alcoolate de sodium selon le procédé décrit dans J. Org. Chem. 1963, 28, 3117 ou avec un alcoolate de sodium en présence d'un catalyseur de transfert de phase tel que tris[2-(2-méthoxyéthoxy)éthyl] amine (TDA-1).

Les composés (VI) dans lesquels $R'_3$ est un groupe $(C_1\text{-}C_8)$alkyle substitué par un $(C_1\text{-}C_4)$ alcoxy peuvent être préparés par réaction d'un composé (VI) dans lequel le substituant $R'_3$ est un $(C_1\text{-}C_8)$alkyle substitué par un hydroxy avec un halogéno $(C_1\text{-}C_4)$alkyle, en présence d'un hydrure métallique tel que l'hydrure de sodium par exemple, dans un solvant anhydre tel que le DMF ou le THF.

Les composés de formule (VI) sont réduits en ortho-phénylènediamines N-substituées de formule (VII) :

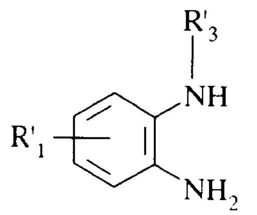

(VII)

La réduction peut être catalytique en utilisant par exemple le palladium sur charbon ou le nickel de Raney®, ou chimique en utilisant le fer, le zinc ou l'étain dans des conditions acides (J. Chem. Soc. 1960, 314).

Les composés de formule (VII), par réaction avec le chloroformiate d'éthyle ou le chloroformiate de méthyle dans un solvant tel que le chloroforme ou le dichlorométhane, en présence ou non d'une base telle que la triéthylamine, ou dans le mélange DMF/eau en présence de carbonate de potassium, conduisent aux composés de formule (VIII) et/ou (VIII') :

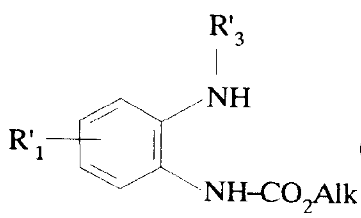

(VIII) et/ou

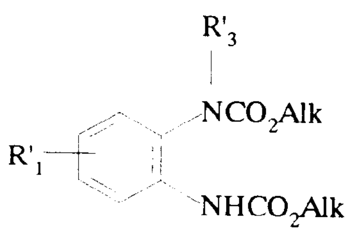

(VIII')

dans laquelle Alk représente éthyle ou méthyle.

Les composés de formule (VIII) et/ou (VIII') sont cyclisés en benzimidazol-2-one (IX) par chauffage avec l'éthylate de sodium.

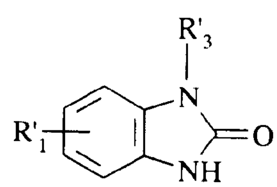

(IX)

Les composés (IX) peuvent également être obtenus par réaction d'un composé de formule (VII) avec de l'urée selon le procédé décrit dans J. Chem. Soc. 1960, 314 ou avec du 1,1'-carbonyldiimidazole selon un procédé décrit dans le brevet européen 92391.

Les composés de formule (II) portant certains substituants $R'_1$, $R'_2$ sur leur partie benzénique sont utilisés comme précurseurs pour la préparation de composés de formule (II) portant d'autres substituants $R'_1$, $R'_2$. Par exemple, les composés (II) dans lesquels $R'_1$ et/ou $R'_2$ = H peuvent être nitrés par des réactifs classiques. A partir d'un composé (II) dans lequel $R'_1$ est un groupe nitro et $R'_2$ est l'hydrogène, on prépare par hydrogénation catalytique le composé (II)

dans lequel $R'_1$ est un groupe amino.

Dans le cas particulier où $R'_3$ représente un azétidin-3-yle, un pipérid-4-yle, un pipérazin-1-yle ou un groupe -$(CH_2)_p$-azétidin-3-yle, -$(CH_2)_p$-pipérid-4-yle ou -$(CH_2)_p$-pipérazin-1-yle dans lequel l'atome d'azote est substitué par $R_{11}$ = un ($C_1$-$C_7$)alkylcarbonyle, un benzoyle, un ($C_1$-$C_7$)alcoxycarbonyle, un phénoxycarbonyle ou un carbamoyle non substitué ou substitué par un ou deux ($C_1$-$C_7$)alkyles, la substitution sur l'atome d'azote peut être réalisée soit sur le composé benzimidazol-2-one (II), soit sur le composé final (I) à partir d'un composé dans lequel l'atome d'azote n'est pas substitué ($R_{11}$ =H). Ainsi lorsque l'atome d'azote est substitué par $R_{11}$ = un ($C_1$-$C_7$)alkylcarbonyle ou un benzoyle, on fait agir sur un composé (II) ou un composé (I) dans lequel l'atome d'azote du radical hétérocyclique tel que défini ci-dessus est non substitué ($R_{11}$ = H), un chlorure d'acide ou un anhydride. Lorsque l'atome d'azote est substitué par $R_{11}$ = un ($C_1$-$C_7$) alcoxycarbonyle ou un phénoxycarbonyle, on fait agir sur un composé (II) ou un composé (I) dans lequel $R_{11}$ = H le chloroformiate approprié. Par action de l'ammoniac sur un composé de formule (II) ou un composé de formule (I) dans lequel $R_{11}$ = phénoxycarbonyle, on prépare un composé (II) ou un composé (I) dans lequel $R_{11}$ est un carbamoyle ; par action sur un tel composé d'une mono ou dialkylamine en ($C_1$-$C_7$), on prépare un composé de formule (II) ou un composé de formule (I) dans lequel $R_{11}$ est un N-($C_1$-$C_7$)alkylcarbamoyle ou N,N-di($C_1$-$C_7$)alkylcarbamoyle. On peut également préparer un composé (II) ou un composé (I) dans lequel $R_{11}$ est un N-alkylcarbamoyle par action d'un isocyanate d'alkyle sur un composé (II) ou un composé (I) dans lequel $R_{11}$ = H.

Les composés de formule (IV) sont connus ou peuvent être préparés par des méthodes connues. Par exemple les cyclohexylamines différemment substituées sont préparées selon J. Org. Chem., 1962, 27, 3568-3572.

Les halogénures de benzyle de formule (III) sont connus ou préparés par des méthodes connues.

A titre d'exemple, on peut citer des publications décrivant les dérivés d'halogénométhylbenzène suivants :

1,2,4- ou 4,2,1- ou 2,4,1-chlorométhyl-méthyl-méthoxybenzène : Bull. Soc. Chim., France, 1937, 4, 1092.

2-chlorométhyl-1,3-diméthoxybenzène : Chem. Listy, 1953, 47, 601-612.

1-bromométhyl-2-méthoxy-4-nitrobenzène : Sci. Sinica (Peking), 1962, 11, 483-498.

1-bromométhyl-2-méthyl-4-nitrobenzène : Pharmazie, 1969, 24 (1), 29-32.

1-bromométhyl-2-méthoxy-4-nitrobenzène : Bull. Soc. Chim., France, 1962, 2255.

1-bromométhyl-4-méthoxy-2-nitrobenzène : Zh. Obshch. Khim., 1963, 33 (8), 2792-2793.

4-bromométhyl-3-méthoxybenzoate de méthyle : demande de brevet européen EP 179 619.

2-bromométhyl-6-méthoxybenzoate d'éthyle : J. Org. Chem., 1983, 48, 3439-3444.

4-bromométhyl-2-méthoxybenzoate de méthyle : Bull. Soc. Chim., France, 1962, 2255.

1-bromométhyl-4-cyano-2-méthoxybenzène, chlorure de 4-bromométhylbenzènesulfonyle : J. Med. Chem., 1990, 33, 2437-2451.

D'une manière générale, les dérivés halogénométhylbenzène peuvent être préparés par action du N-bromosuccinimide sur les dérivés de méthylbenzène correspondants. La réaction est effectuée dans un solvant comme le tétrachlorure de carbone en présence de péroxyde de dibenzoyle. On peut également préparer un dérivé d'halogénométhylbenzène à partir d'un dérivé d'hydroxyméthylbenzène correspondant par action du tribromure de phosphore dans l'éther.

Selon un autre procédé, les dérivés d'halogénométhylbenzène de formule (III) peuvent être préparés à partir de l'alcool correspondant par action du chlorure de thionyle pour préparer un chlorure de méthylbenzène.

La transformation d'un composé (I') en un composé (I) est effectuée en utilisant des méthodes connues de l'homme de l'art.

Dans la description qui va suivre, on expose la préparation des composés de formule (I) portant des substituants $R_1$, $R_3$, $R_5$ et/ou $R_6$ ; les mêmes méthodes s'appliquent le cas échéant à la préparation des composés dans lesquels le substituant $R_5$ a la valeur indiquée pour $R_6$.

Les composés (I) dans lesquels $R_1$ et/ou $R_6$ est un hydroxy peuvent être obtenus par hydrogénation catalytique, par exemple en présence de palladium sur charbon, d'un composé de formule (I') dans lequel $R'_1$ et/ou $R'_6$ est un benzyloxy. Ces composés peuvent également être préparés à partir de composés analogues de formule (I') dans lesquels $R'_1$ et/ou $R'_6$ représente un groupe amino en utilisant la méthode décrite dans J. Org. Chem., 1977, 42, 2053.

Les composés de formule (I) dans lesquels $R_1$ et/ou $R_6$ représente un alcoxy en $C_1$-$C_7$ peuvent être préparés directement par le procédé selon l'invention à partir des composés de formule (II) et (III) correctement substitués.

Les composés (I') dans lesquels $R'_1$ et/ou $R'_6$ représente un hydroxy permettent également de préparer des composés (I) dans lesquels $R_1$ et/ou $R_6$ est un alcoxy en $C_1$-$C_7$ par action d'un halogénure d'alkyle en $C_1$-$C_7$ en présence d'une base telle qu'un hydrure métallique ou un carbonate alcalin ou alcalino-terreux comme $K_2CO_3$ ou $Cs_2CO_3$ dans un solvant tel que le THF ou le DMF. De même, on peut préparer les composés de formule (I) dans lesquels $R_6$ représente un ω-aminoalkyloxy par action d'une ω-chloro($C_2$-$C_7$)alkylamine sur les composés dans lesquels $R'_6$ = OH ; de même on peut préparer les composés dans lesquels $R_1$ et/ou $R_6$ représente un ω-hydroxy($C_2$-$C_7$)alkyloxy par action d'un chloro($C_2$-$C_7$)alcanol ; dans le cas particulier de la préparation d'un composé (I) dans lequel $R_1$ et/ou $R_6$ = $O(CH_2)_2OH$, on peut également faire agir le carbonate d'éthylène sur un composé (I') dans lequel $R'_1$ et/ou $R'_6$ = OH.

Les halogénures d'halogéno($C_2$-$C_7$)alkyloxybenzyle (III, $R'_6$ = ω-halogéno($C_2$-$C_7$)alkyloxy) peuvent être utilisés

pour la préparation de composés selon l'invention dans lesquels le substituant $R_6$ est un ω-amino($C_2$-$C_7$)alkyloxy non substitué ou substitué par un ou deux alkyles, selon le schéma suivant :

$$\text{-O-Alk'-Hal} + NHR_7R_8 \rightarrow \text{-OAlk'-}NR_7R_8$$

dans lequel Alk' représente un alkyle en $C_2$-$C_7$.

Les composés de formule (I) dans lesquels $R_6$ représente un ($C_1$-$C_7$)alkylcarbonyloxy ou un benzoyloxy sont obtenus par action d'un halogénure d'acide ou d'un anhydride sur un composé (I') dans lequel $R'_6$ est un hydroxy.

Les composés de formule (I) dans lesquels $R_6$ est un groupe $-OR_{14}$, $R_{14}$ représentant un ω-carbamoyl ($C_1$-$C_7$) alkyle libre ou substitué par un ou deux alkyles en $C_1$-$C_7$ peuvent être préparés à partir d'un composé (I') dans lequel $R'_6$ représente un groupe $-OR_{14}$, $R_{14}$ représentant un ω-carboxy($C_1$-$C_7$)alkyle estérifié par un alkyle en $C_1$-$C_4$. Cette préparation est effectuée de façon classique pour l'homme de l'art, par action d'une amine correctement choisie.

Un composé de formule (I') dans lequel $R'_6$ est un groupe nitro permet d'obtenir par hydrogénation catalytique, par exemple en présence d'oxyde de platine ou de nickel de Raney®, ou par réduction chimique, par exemple en présence d'étain ou de fer en milieu acide, un composé (I) dans lequel $R_6$ est un groupe amino ; on peut ensuite préparer d'autres composés dans lesquels le groupe amino est substitué en utilisant des réactions bien connues de l'homme de l'art.

Pour préparer des composés de formule (I) dans lesquels $R_1$ et/ou $R_6$ représente un ($C_1$-$C_7$) monoalkylamino, on fait réagir un composé de formule (I') dans lequel $R'_1$ et/ou $R'_6$ représente un groupe amino avec un aldéhyde ou une cétone, en milieu acide, en présence d'un agent réducteur tel que le cyanoborohydrure de sodium ; par une réaction identique, on prépare les composés (I) dans lesquels $R_1$ et/ou $R_6$ représente un dialkylamino.

On peut préparer les composés de formule (I) dans lesquels $R_6$ représente un groupe $-NR_{12}R_{13}$ dans lequel $R_{13}$ représente un benzyle, lui-même éventuellement substitué, par action d'un chlorure de benzyle éventuellement substitué sur un composé de formule (I') dans lequel $R'_6$ est un groupe-$NHR_{12}$.

Pour préparer les composés de formule (I) dans lesquels $R_6$ représente un groupe amino substitué par un ($C_3$-$C_7$) cycloalkylméthyle ou respectivement un phénétyle éventuellement substitué, on fait réagir un composé de formule (I') dans lequel $R'_6$ représente un groupe amino avec un ($C_3$-$C_7$)cycloalkylcarboxaldéhyde ou respectivement un phénylacétaldéhyde éventuellement substitué, en milieu acide, en présence d'un agent réducteur tel que le cyanoborohydrure de sodium.

Pour préparer les composés de formule (I) dans lesquels $R_6$ représente un groupe amino substitué par un ($C_3$-$C_8$) alcényle, on fait réagir un composé de formule (I') dans lequel $R'_6$ représente un groupe amino ou ($C_1$-$C_7$)alkylamino avec un chlorure d'alcényle en $C_3$-$C_8$.

Les composés de formule (I) dans lesquels $R_6$ représente un groupe Δ3-pyrrolin-1-yle se préparent par action, sous atmosphère inerte, du cis-1,4-dichlorobut-2-ène sur les composés de formule (I') dans lesquels $R'_6$ est un groupe amino en présence d'une base telle que la triéthylamine. Par hydrogénation on prépare ensuite les composés de formule (I) dans lesquels $R_6$ est un groupe pyrrolidin-1-yle. La réaction du cis-1,4-dichlorobut-2-ène avec les composés (I') dans lesquels $R'_6$ est un groupe amino, peut également s'effectuer à l'air en présence d'une base telle que le carbonate de sodium et conduit, dans ces conditions, à la formation d'un mélange d'un composé de formule (I) dans lequel $R_6$ représente un groupe Δ3-pyrrolin-1-yle et d'un composé de formule (I) dans lequel $R_6$ représente un groupe pyrrol-1-yle que l'on peut séparer par chromatographie.

On peut également préparer un composé de formule (I) dans laquelle $R_6$ représente un groupe pyrrol-1-yle par réaction d'un composé de formule (I) dans laquelle $R_6$ représente un groupe Δ3-pyrrolin-1-yle avec l'acide perbenzoïque, lui-même préparé par action du peroxyde d'hydrogène sur le chlorure de benzoyle.

Les composés de formule (I) dans lesquels $R_6$ représente un groupe isoindolin-2-yle se préparent par action de l'α,α'-dibromo-o-xylène sur les composés de formule (I') dans lesquels $R'_6$ est un groupe amino, en présence d'une base telle que la triéthylamine, et dans un solvant tel que le diméthylformamide à reflux.

Les composés de formule (I) dans lesquels $R_6$ représente un groupe 1-méthyl-2,4-dioxoimidazolin-3-yle se préparent en deux étapes : on fait réagir la sarcosine sur un composé de formule (I') dans lequel $R'_6$ représente un phénoxycarboxamido, en présence d'une base telle que la triéthylamine, pour obtenir un composé de formule (I') dans lequel $R'_6$ représente un N'-carboxyméthyl-N'-méthyluréido, puis par chauffage à 100°C, sous vide, le produit obtenu précédemment se cyclise.

Lorsque $R'_6$ représente un groupe amino, on peut également effectuer une nitrosation, par exemple en présence d'acide nitreux ou de nitrite de sodium, pour préparer un composé (I') dans lequel $R'_6$ représente un sel de diazonium ; par des réactions connues de l'homme de l'art, on accède alors aux composés (I) selon l'invention dans lesquels $R_6$ est un cyano, un halogéno ou un alkylthio en $C_1$-$C_7$.

Les composés de formule (I) dans lesquels $R_6$ représente un groupe $-CH_2NH_2$ se préparent à partir de composés analogues de formule (I') dans lesquels $R'_6$ représente un groupe cyano selon la méthode décrite dans J. Med. Chem., 1990 33, 2437-2451. A partir de ces composés, on prépare les composés de formule (I) dans lesquels $R_6$ représente un groupe $-CH_2NR_7R_8$, $R_8$ et/ou $R_7$ étant différent de l'hydrogène, selon des méthodes connues de l'homme de l'art.

Les composés (I) dans lesquels $R_6$ représente un groupe $-NR_{12}R_{13}$, $R_{13}$ étant un ($C_1$-$C_7$)alkylcarbonyle, un ($C_3$-$C_7$)

cycloalkylcarbonyle, un benzoyle éventuellement substitué, un phénacétyle dans lequel le noyau benzénique est éventuellement substitué, un pyridylcarbonyle, un méthylpyridylcarbonyle, un thiénylcarbonyle, un furylcarbonyle, ou un pipérid-4-ylcarbonyle, s'obtiennent par action de l'anhydride approprié ou du chlorure d'acide approprié sur un composé (I') dans lequel $R'_6$ est un groupe $R_{12}NH$-, en présence d'une amine comme la triéthylamine.

Les composés (I) dans lesquels $R_6$ représente un groupe $-NR_{12}R_{13}$, $R_{13}$ étant un $(C_1-C_7)$alcoxycarbonyle ou, respectivement, un phénoxycarbonyle ou un benzyloxycarbonyle, s'obtiennent par action d'un chloroformiate d'alkyle en $C_1-C_7$ ou, respectivement, de phényle ou de benzyle sur un composé (I') dans lequel $R'_6$ est un groupe $R_{12}NH$-. De même, par action d'un chlorure de phénoxythiocarbonyle sur un composé de formule (I') dans lequel $R'_6$ représente un groupe $R_{12}NH$-, on obtient un composé de formule (I) dans lequel $R_6$ est un groupe $-NR_{12}R_{13}$ dans lequel $R_{13}$ représente un phénoxythiocarbonyle.

Par action de l'ammoniac sur un composé de formule (I') dans lequel $R'_6$ est un groupe $-NR_{12}R_{13}$ dans lequel $R_{13}$ représente un phénoxycarbonyle ou un phénoxythiocarbonyle, on prépare un composé de formule (I) dans lequel $R_6$ est un groupe $-N(R_{12})CONH_2$ ou $-N(R_{12})CSNH_2$.

On peut également préparer des composés de formule (I) dans lesquels $R_6$ est un uréido ($-NR_{12}CONR_{22}R_{23}$) ou, respectivement, un thiouréido ($-NR_{12}CSNR_{22}R_{23}$) par action d'un composé $NHR_{22}R_{23}$, sur un composé (I') dans lequel $R'_6$ est un groupe $-NR_{12}R_{13}$ dans lequel $R_{13}$ représente un phénoxycarbonyle ou, respectivement, phénoxythiocarbonyle.

On peut aussi préparer des composés de formule (I) dans lesquels $R_6$ est un uréido ($-NR_{12}CONR_{22}R_{23}$) ou respectivement un thiouréido ($-NR_{12}CSNR_{22}R_{23}$) par action d'un chlorure de carbamoyle ($ClCONR_{22}R_{23}$) ou respectivement d'un chlorure de thiocarbamoyle ($ClCSNR_{22}R_{23}$) sur un composé de formule (I') dans lequel $R'_6$ est un groupe $R_{12}NH$-.

On peut également préparer un composé (I) dans lequel $R_6$ est un groupe $-NR_{12}R_{13}$ dans lequel $R_{13}$ représente un $(C_1-C_7)$alkylcarbamoyle par action d'un isocyanate d'alkyle en $C_1-C_7$ sur un composé (I') dans lequel $R'_6$ est un groupe $R_{12}NH$-.

On peut encore préparer un composé (I) dans lequel $R_6$ est un thiouréido par action du réactif de Lawesson sur un composé (I') dans lequel $R'_6$ est l'uréido correspondant.

Pour préparer un composé de formule (I) dans lequel $R_6$ représente un groupe $-NR_{12}CO-(C_2-C_7)alkyl-NR_7R_8$, on fait réagir un halogénure d'halogéno$(C_3-C_8)$acyle, tel que le chlorure de 3-chloropropionyle ou le chlorure de 4-chlorobutyryle par exemple, sur un composé de formule (I') dans lequel $R'_6$ est un groupe $-NHR_{12}$, en présence d'une base comme la triéthylamine ; puis par réaction du composé obtenu avec une amine $HNR_7R_8$ on obtient le composé de formule (I) désigné ci-dessus.

De même par action d'un halogénure d'ω-benzyloxy-$(C_1-C_7)$alkylcarbonyle, sur un composé de formule (I') dans lequel $R'_6$ représente un groupe $-NHR_{12}$, on prépare un composé de formule (I) dans lequel $R_6$ représente un groupe $-NR_{12}CO-(C_1-C_7)alkyl-O-CH_2-C_6H_5$. Par hydrogénation du composé précédent, en présence d'un catalyseur tel que par exemple le palladium sur charbon, on obtient un composé de formule (I) dans lequel $R_6$ est un groupe $-NR_{12}CO-(C_1-C_7)alkyl-OH$.

De la même façon, on fait agir le chlorure d'acide $R_7R_8NCR_{17}R_{18}COCl$ sur un composé de formule (I') dans lequel $R'_6$ est un groupe $-NHR_{12}$ pour préparer un composé de formule (I) dans lequel $R_6$ est un groupe $-NR_{12}COCR_{17}R_{18}NR_7R_8$.

On peut utiliser l'action d'un anhydride, tel que l'anhydride succinique ou l'anhydride glutarique, sur un composé (I') dans lequel $R'_6$ est un groupe $-NHR_{12}$ pour préparer un composé (I) dans lequel $R_6$ est un groupe $-NR_{12}CO(CH_2)_2CO_2H$ ou $-NR_{12}CO(CH_2)_3CO_2H$. Le cas échéant on transforme l'acide ainsi obtenu en ester ou en amide.

On peut également utiliser l'action du chlorure d'ethyloxalyle sur un composé (I') dans lequel $R'_6$ est un groupe $-NHR_{12}$ pour préparer un composé (I) dans lequel $R_6$ est un groupe $-NR_{12}COCO_2Et$.

Les composés (I) dans lesquels $R_6$ représente un groupe $-NR_{12}$ substitué par un groupe $-(CH_2)_t-COR_{19}$, s'obtiennent par action d'un composé de formule $Hal(CH_2)_tCOOR$ dans lequel Hal représente un halogène, par exemple le brome et R représente un alkyle en $C_1-C_7$ sur un composé (I') dans lequel $R'_6$ est un groupe $-NHR_{12}$, en présence de chlorure cuivreux ; le cas échéant, on transforme l'ester ainsi obtenu en acide ou en amide.

De la même façon, les composés de formule (I) dans lesquels $R_6$ est un groupe $-NR_{12}$ substitué par un groupe $-CR_{17}R_{18}COR_{19}$ sont préparés par action d'un composé de formule $Hal-CR_{17}R_{18}COR_{19}$ sur les composés (I') correspondants dans lesquels le substituant $R'_6$ est un groupe $-NHR_{12}$.

Les composés (I) dans lesquels $R_6$ représente un $(C_1-C_7)$ alkylsulfonamido ou respectivement un groupe $-NHSO_2-Ar$, un groupe $-NH-SO_2-CH_2-Ar$ ou un diméthylaminosulfonamido s'obtiennent par action d'un halogénure d'alkylsulfonyle dans lequel l'alkyle est en $C_1-C_7$ ou respectivement d'un composé $Ar-SO_2Cl$, d'un composé $Ar-CH_2-SO_2Cl$ ou d'un halogénure de diméthylsulfamoyle sur un composé (I') dans lequel $R'_6$ est un groupe amino.

Les composés (I) dans lesquels $R_6$ est un groupe guanidino non substitué ou substitué une ou deux fois par un alkyle en $C_1-C_7$, un phényle ou un benzyle, peuvent se préparer à partir de composés (I') dans lesquels $R'_6$ est un groupe phénoxycarboxamido par action du cyanamide ou d'un de ses dérivés correctement substitué sur l'azote.

Les composés (I) dans lesquels $R_6$ est un groupe guanidino substitué en position 2 par un cyano se préparent en deux étapes : on fait réagir le diméthyl N-cyanodithioiminocarbonate sur un composé (I') dans lequel $R'_6$ est un amino, dans un solvant tel que le n-butanol à reflux, pour obtenir un composé (I') dans lequel $R'_6$ est un groupe $-NHC(SCH_3)=N-CN$ ; puis par réaction du composé précédent avec une amine appropriée, on obtient le composé (I) attendu.

Les composés de formule (I) dans lesquels $R_6$ représente un ($C_1$-$C_7$)alcoxycarbonyle peuvent se préparer directement à partir de l'halogénure de formule (III) portant le même substituant par le procédé selon l'invention. Par des méthodes connues de l'homme de l'art, ils permettent d'obtenir les composés de formule (I) dans lesquels $R_6$ est un groupe carboxy.

Selon un autre mode opératoire, les composés de formule (I) dans lesquels $R_6$ est un benzyloxycarbonyle permettent d'obtenir par hydrogénation catalytique les composés (I) dans lesquels $R_6$ est un carboxyle. Par action d'un halogénure de thionyle, on obtient les composés de formule (I') dans lesquels $R'_6$ est un halogénocarbonyle. A partir de tels composés, on prépare des composés de formule (I) dans lesquels $R_6$ est un carbamoyle substitué par $R_{15}$ et $R_{16}$, par action d'un composé $HNR_{15}R_{16}$.

On peut également utiliser les composés de formule (I') dans lesquels $R'_6$ représente un phénoxycarbonyle pour obtenir les composés de formule (I) dans lesquels $R_6$ est un phénylcarbamoyle ou un ($C_1$-$C_7$)alkylcarbamoyle par action d'une aniline ou d'une ($C_1$-$C_7$)alkylamine. Une aniline substituée sur le phényle ou, respectivement, une alkylamine substituée sur l'alkyle par $R_{24}$ permettent d'obtenir des composés de formule (I) dans lesquels $R_6$ est un phénylcarbamoyle substitué sur le phényle ou, respectivement, un alkylcarbamoyle substitué sur l'alkyle par $R_{24}$.

On peut utiliser les composés de formule (I') dans lesquels $R'_6$ est un carboxy pour préparer les composés de formule (I) dans lesquels $R_6$ est un groupe $-CONR_{15}R_{16}$ par action d'un composé de formule $HNR_{15}R_{16}$, en présence de BOP et d'une base telle que la diisopropyléthylamine.

Les composés de formule (I) dans lesquels $R_6$ est un groupe $-COR_{25}$ peuvent également s'obtenir à partir de composés (I') dans lesquels $R'_6$ est un phénoxycarbonyle, par action d'un composé $R_{25}H$.

Les composés de formule (I) dans lesquels $R_6$ est un sulfamoyle substitué par $R_{20}$ et $R_{21}$, s'obtiennent par action d'un composé $HNR_{20}R_{21}$ sur un composé de formule (I') dans lequel $R'_6$ représente un groupe halogénosulfonyle.

On peut préparer un composé (I) dans lequel $R_6$ est un thiocarbamoyle par réaction du réactif de Lawesson sur un composé (I) dans lequel $R_6$ est le carbamoyle correspondant.

Les composés de formule (I) dans lesquels $R_6$ est un groupe $-CONHCR_{17}R_{18}COR_{19}$ sont préparés à partir de composés de formule (I') dans lesquels $R'_6$ représente soit un groupe -COCl, soit un groupe phénoxycarbonyle, par action de $H_2NCR_{17}R_{18}COR_{19}$. Ils peuvent également être préparés à partir de composés de formule (I') dans lesquels $R'_6$ est un carboxy par réaction avec un composé $H_2NCR_{17}R_{18}COR_{19}$ en présence de BOP et d'une amine telle que la diisopropyléthylamine.

L'affinité des composés selon l'invention pour les récepteurs de la vasopressine a été déterminée *in vitro* en utilisant la méthode décrite dans C.J. Lynch et al., J. Biol. Chem., 1985, 260 (5), 2844-2851. Cette méthode consiste à étudier le déplacement de la vasopressine tritiée fixée aux sites $V_1$ de membranes de foie de rats. Les concentrations inhibitrices de 50 % ($CI_{50}$) de la fixation de la vasopressine tritiée des composés selon l'invention sont faibles, allant jusqu'à $10^{-6}$M.

L'affinité des composés (I) selon l'invention pour les récepteurs $V_2$ a été mesurée sur une préparation membranaire de rein de boeuf selon une méthode adaptée de P. Crause et al., Molecular and Cellular Endocrinology, 1982, 28, 529-541 et de F.L. Stassen et al., J. Pharmacol. Exp. Ther., 1982, 223, 50-54. Les composés selon l'invention inhibent la fixation de l'arginine-vasopressine tritiée aux récepteurs de la préparation membranaire. Les $CI_{50}$ des composés selon l'invention sont faibles, allant jusqu'à $10^{-9}$M.

L'activité antagoniste des récepteurs $V_2$ des composés selon l'invention a été étudiée par le test de dosage de l'activité adénylate cyclase effectuée selon une méthode adaptée de M. Laburthe et al., Molecular Pharmacol., 1986, 29, 23-27. On utilise une préparation membranaire de rein de boeuf et chaque produit est incubé 10 minutes à 37°C, seul ou en présence d'AVP (arginine vasopressine) à la concentration de $3.10^{-8}$M. L'AMP cyclique (adénosine monophosphate cyclique) produite est mesurée par dosage radioimmunologique. On détermine la concentration inhibant de 50 % ($CI_{50}$) la stimulation de l'adénylate cyclase induite par $3.10^{-8}$M d'AVP. Les $CI_{50}$ déterminées sont faibles allant jusqu'à $10^{-8}$M.

L'activité agoniste ou antagoniste des récepteurs de la vasopressine des composés selon l'invention, administrés par voie orale, est évaluée chez le rat (souche OFA ou souche Sprague-Dawley), en surcharge hydrique, traité à la vasopressine. L'activité antagoniste des composés, selon l'invention a été également évaluée chez le rat normalement hydraté (souche OFA ou souche Sprague-Dawley) selon la technique décrite dans Br. J. Pharmacol., 1992, 105, 787-791. L'effet diurétique a été observé pour certains composés à la dose de 10 mg/kg.

De même, l'affinité des composés (I) selon l'invention pour les récepteurs de l'ocytocine a été déterminée *in vitro* par déplacement d'un analogue radioiodé de l'ocytocine fixé aux récepteurs d'une préparation membranaire de glandes mammaires de rates en gestation, selon une technique proche de celle décrite par J. Eland et al., dans Eur. J. Pharmacol., 1987, 147, 197-207. Les $CI_{50}$ des composés selon l'invention atteignent $10^{-6}$M.

Les composés selon l'invention sont actifs après administration par différentes voies, notamment par voie orale.

Aucun signe de toxicité n'est observé avec ces composés aux doses pharmaceutiquement actives.

Ainsi les composés selon l'invention peuvent être utilisés dans le traitement ou la prévention de différentes affections vasopressine-dépendantes ou ocytocine-dépendantes, les affections cardiovasculaires, comme l'hypertension, l'hypertension pulmonaire, l'insuffisance cardiaque, l'infarctus du myocarde, ou le vasospasme coronaire, en particulier chez le fumeur, les angines instables et PTCA (percutaneous transluminal coronary angioplasty), l'ischémie cardiaque, les dérèglements de l'hémostase notamment l'hémophilie, le syndrome de Von Willebrand ; les affections du système nerveux central, la migraine, le vasospasme cérébral, l'hémorragie cérébrale, les oedèmes cérébraux, la dépression, l'anxiété, les états psychotiques, les troubles de la mémoire par exemple ; les affections du système rénal comme les oedèmes, le vasospasme rénal, la nécrose du cortex rénal, l'hyponatrémie, l'hypokaliémie, le syndrome de Schwartz Bartter ; les affections du système gastrique, comme le vasospasme gastrique, l'hépatocirrhose, les ulcères, la pathologie des vomissements, par exemple la nausée y compris la nausée due à une chimiothérapie, le mal des transports, ou encore le syndrome de la sécrétion inappropriée de l'hormone antidiurétique (SIADH), le diabète insipide et l'énurésie. Les composés selon l'invention peuvent également être utilisés dans le traitement des troubles du comportement sexuel. Chez la femme, les composés selon l'invention peuvent être utilisés pour traiter la dysménorrhée ou le travail prématuré. On peut également utiliser les composés selon l'invention dans le traitement des cancers pulmonaires à petites cellules, des encéphalopathies hyponatrémiques, de la maladie de Raynaud, le syndrome pulmonaire, le glaucome, la cataracte et dans les traitements post-opératoires, notamment après une chirurgie abdominale.

La présente invention a également pour objet des compositions pharmaceutiques contenant une dose efficace d'un composé selon l'invention ou d'un sel pharmaceutiquement acceptable de celui-ci, et des excipients convenables.

Lesdits excipients sont choisis selon la forme pharmaceutique et le mode d'administration souhaité.

Dans les compositions pharmaceutiques de la présente invention pour l'administration par voie orale, sublinguale, inhalée, sous-cutanée, intramusculaire, intraveineuse, topique, intratrachéale, intranasale, transdermique ou rectale, les principes actifs de formule (I) ci-dessus, ou leurs sels éventuels, peuvent être administrés sous formes unitaires d'administration, en mélange avec des supports pharmaceutiques classiques, aux animaux et aux êtres humains pour la prophylaxie ou le traitement des troubles ou des maladies ci-dessus. Les formes unitaires d'administration appropriées comprennent les formes par voie orale telles que les comprimés, les gélules, les poudres, les granules et les solutions ou suspensions orales, les formes d'administration sublinguale, buccale, intratrachéale, intranasale, les aérosols, les implants, les formes d'administration sous-cutanée, intramusculaire ou intraveineuse et les formes d'administration rectale. Pour l'application topique, on peut utiliser les composés selon l'invention dans des crèmes, pommades ou lotions.

Afin d'obtenir l'effet prophylactique ou thérapeutique désiré, la dose de principe actif peut varier entre 0,01 et 50 mg par kg de poids du corps et par jour.

Chaque dose unitaire peut contenir de 0,5 à 1000 mg, de préférence de 1 à 500 mg, d'ingrédients actifs en combinaison avec un support pharmaceutique. Cette dose unitaire peut être administrée 1 à 5 fois par jour de façon à administrer un dosage journalier de 0,5 à 5000 mg, de préférence de 1 à 2500 mg.

Lorsqu'on prépare une composition solide sous forme de comprimés, on mélange l'ingrédient actif principal avec un véhicule pharmaceutique tel que la silice, la gélatine, l'amidon, le lactose, le stéarate de magnésium, le talc, la gomme arabique ou analogues. On peut enrober les comprimés de saccharose, d'un dérivé cellulosique, ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant tel qu'un glycol ou un ester de glycérol et en incorporant le mélange obtenu dans des gélules molles ou dures.

Une préparation sous forme de sirop ou d'élixir ou pour l'administration sous forme de gouttes peut contenir l'ingrédient actif conjointement avec un édulcorant, acalorique de préférence, du méthylparaben et du propylparaben comme antiseptique, ainsi qu'un agent donnant du goût et un colorant approprié.

Les poudres ou les granules dispersibles dans l'eau peuvent contenir l'ingrédient actif en mélange avec des agents de dispersion ou des agents mouillants, ou des agents de mise en suspension, comme la polyvinylpyrrolidone, de même qu'avec des édulcorants ou des correcteurs de goût.

Pour une administration rectale, on recourt à des suppositoires qui sont préparés avec des liants fondant à la température rectale, par exemple du beurre de cacao ou des polyéthylèneglycols.

Pour une administration parentérale, on utilise des suspensions aqueuses, des solutions salines isotoniques ou des solutions stériles et injectables qui contiennent des agents de dispersion et/ou des mouillants pharmacologiquement compatibles, par exemple le propylèneglycol, le polyéthylèneglycol ou le butylèneglycol.

Pour une administration par inhalation on utilise un aérosol contenant en outre, par exemple du trioléate de sorbitane ou de l'acide oléique ainsi que du trichlorofluorométhane, du dichlorofluorométhane, du dichlorotétrafluoroéthane ou tout autre gaz propulseur biologiquement compatible ; on peut également utiliser un système contenant le principe actif, seul ou associé à un excipient, sous forme de poudre.

Le principe actif peut être formulé également sous forme de microcapsules, éventuellement avec un ou plusieurs supports ou additifs.

Les compositions de la présente invention peuvent contenir, à côté des produits de formule (I) ci-dessus ou de leurs sels pharmaceutiquement acceptables, d'autres principes actifs qui peuvent être utiles dans le traitement des troubles ou maladies indiquées ci-dessus.

Ainsi, la présente invention a également pour objet des compositions pharmaceutiques contenant plusieurs principes actifs en association dont l'un est un composé selon l'invention.

Ainsi, selon la présente invention, on peut préparer des compositions pharmaceutiques contenant un composé selon l'invention associé à un composé agissant sur le système rénine-angiotensine tel qu'un inhibiteur de l'enzyme de conversion, un antagoniste de l'angiotensine II, un inhibiteur de la rénine. On peut également associer un composé selon l'invention, par exemple, avec un vasodilatateur périphérique, un inhibiteur calcique, un béta-bloquant, un alpha-1-bloquant ou un diurétique. De telles compositions seront utiles en particulier dans le traitement de l'hypertension ou de la défaillance cardiaque.

On peut également associer deux composés selon l'invention : un antagoniste spécifique du récepteur $V_1$ à un antagoniste spécifique du récepteur $V_2$ ou bien un antagoniste spécifique du récepteur $V_1$ à un antagoniste spécifique de l'ocytocine.

Par antagoniste spécifique du récepteur $V_1$, ou du récepteur $V_2$ ou respectivement de l'ocytocine, on entend un composé qui présente une affinité pour le récepteur $V_1$, le récepteur $V_2$ ou respectivement l'ocytocine, notoirement supérieure (au moins 10 fois) à son affinité pour les deux autres récepteurs.

Ces associations permettront de renforcer les activités thérapeutiques des composés selon l'invention.

## PREPARATIONS

Préparations des 1,3-dihydro-2*H*-benzimidazol-2-one.

### Préparation 1

5-chloro-3-cyclohexyl-1,3-dihydro-2*H*-benzimidazol-2-one.

A) 4-chloro-2-cyclohexylamino-1-nitrobenzène

On maintient au reflux pendant 12 heures le mélange constitué de 19,4 g de 2,4-dichloro-1-nitrobenzène, 40 g de cyclohexylamine et 100 ml de 2-éthoxyéthanol.

On évapore sous vide le solvant et reprend le résidu par de l'éther éthylique, et lave avec $H_2O$, séche sur $Na_2SO_4$ et évapore sous vide le solvant. On obtient 11,7 g du produit attendu après cristallisation dans l'éther iso, F = 125°C.

B) 4-chloro-2-cyclohexylamino-1-aminobenzène

On porte au reflux 12 g du composé obtenu à l'étape A), 8 g de fer en poudre, 15 ml d'eau et 15 ml d'éthanol. On introduit ensuite goutte à goutte en 30 minutes, 30 ml d'acide chlorhydrique concentré dans 20 ml d'eau et 20 ml d'éthanol. On maintient ensuite le milieu réactionnel au reflux pendant 1 heure 30 minutes.

Après refoidissement, on verse le milieu réactionnel sur de la glace et on y ajoute une solution saturée de $NaHCO_3$. On extrait avec AcOEt, lave à l'eau puis avec une solution saturée de $NaHCO_3$, ensuite à l'eau, sèche sur $Na_2SO_4$ et évapore sous vide le solvant. On chromatographie le résidu sur silice en éluant avec de l'éther isopropylique. On obtient 9,4 g du produit attendu, qui est utilisé tel quel à l'étape suivante.

C) 5-chloro-3-cyclohexyl-1,3-dihydro-2*H*-benzimidazol-2-one.

On chauffe à 170-180°C pendant 90 minutes un mélange de 4,5 g du composé obtenu à l'étape B) avec 2,5 g d'urée et 10 ml de 1, 2, 3, 4-tétraméthylbenzène.

Après refroidissement on reprend le milieu réactionnel par de l'acétate d'éthyle. On lave à l'eau, sèche sur sulfate de sodium et évapore sous vide le solvant. Après cristallisation dans de l'heptane et recristallisation dans AcOEt on obtient 3,5 g du produit attendu, F = 213°C.

On peut également préparer ce composé selon Eur. J. Med. Chem. - Chimica Therapeutica, 1981, 16 (4), 321-326, F = 206-208°C.

### Préparation 2.

3-Cyclohexyl-1,3-dihydro-5-methoxy-2*H*-benzimidazol-2-one.

A) 2-Cyclohexylamino-4-méthoxy-1-nitrobenzène.

On prépare une solution de méthylate de sodium en ajoutant 0,5 g de sodium dans 60 ml de MeOH. Puis on

ajoute successivement 5,1 g du composé obtenu à la préparation 1 étape A, 7,5 ml de tris [2-(2-méthoxyéthoxy) éthyl]amine et chauffe à reflux pendant 24 heures. On évapore sous vide le solvant, reprend le résidu à l'eau, extrait au DCM, lave à l'eau, sèche sur $Na_2SO_4$ et évaporé sous vide. On obtient 3,86 g du produit attendu sous forme d'huile qui cristallise, F = 78-80°C.

B) 1-Amino-2-cyclohexylamino-4-méthoxybenzène.

On chauffe à reflux un mélange de 11,86 g du composé obtenu à l'étape précédente, 7,9 g de fer en poudre, 15 ml d'eau et 15 ml d'EtOH, puis on ajoute, goutte à goutte, une solution de 29,4 ml d'acide chlorhydrique concentré dans 20 ml d'eau et 20 ml d'EtOH. On laisse le mélange réactionnel à reflux pendant 2 heures. Après refroidissement, on verse le mélange réactionnel sur de la glace, ajoute une solution saturée de $NaHCO_3$, extrait au DCM et filtre un insoluble gris sur Célite. Après décantation du filtrat, on lave la phase organique par une solution saturée de $NaHCO_3$, à l'eau, sèche sur $Na_2SO_4$ et évapore sous vide. On chromatographie le résidu sur silice en éluant par le mélange DCM/MeOH (98/2 ; v/v). On obtient 7,5 g du produit attendu sous forme d'une huile noire que l'on utilise tel quel à l'étape suivante.

C) 3-Cyclohexyl-1,3-dihydro-5-méthoxy-2*H*-benzimidazol-2-one.

On chauffe à 170-180°C pendant 1 heure 30 minutes un mélange de 7,5 g du composé obtenu à l'étape précédente, 4,1 g d'urée dans 20 ml de 1,2,3,4-tetraméthylbenzène. Après refroidissement, on extrait à l'AcOEt, lave à l'eau, sèche sur $Na_2SO_4$ et évapore sous vide. On reprend le résidu à l'hexane et essore le précipité marron formé. On chromatographie le précipité sur silice en éluant par le mélange DCM/AcOEt (50/50 ; v/v). On obtient 2,76 g du produit attendu après cristallisation dans l'AcOEt, F = 163-165°C.

Préparation 3

3-Cyclohexyl-5-éthoxy-1,3-dihydro-2*H*-benzimidazol-2-one.

A) 4-Ethoxy-1-nitro-2-cyclohexylaminobenzène.

On prépare une solution d'éthylate de sodium en ajoutant 0,5 g de sodium dans 60 ml d'éthanol. Puis on ajoute 5,1 g de 4-chloro-2-cyclohexylamino-1-nitrobenzène décrit à la préparation 1 étape A. Ensuite on ajoute 7,5 ml de tris 2-(2-méthoxyéthoxy)éthylamine et porte au reflux pendant 3 heures. On évapore sous vide le solvant et reprend le résidu par de l'eau, extrait au DCM, lave à l'eau, sèche sur $Na_2SO_4$ et évapore sous vide le solvant. Après chromatographie sur silice en éluant au DCM on obtient 4,26 g du produit attendu sous la forme d'une huile jaune qui a cristallisé dans l'éther iso, F = 80-82°C.

B) 4-Ethoxy-1-amino-2-cyclohexylaminobenzène.

On porte au reflux un mélange de 4,26 g du composé obtenu à l'étape A), de 2,7 g de fer en poudre dans 5,1 ml d'eau et 5,1 ml d'éthanol. Puis on ajoute goutte à goutte une solution de 10 ml d'HCl concentré dans 7 ml d'eau et 7 ml d'éthanol et on maintient encore le reflux pendant 2 heures. Après refroidissement, on verse le mélange réactionnel sur de la glace et on traite par une solution saturée de $NaHCO_3$, extrait au DCM, filtre sur Celite un insoluble gris. Après décantation de la phase organique, on lave à l'eau, sèche sur $Na_2SO_4$ et évapore sous vide le solvant. On obtient 3,05g d'une huile noire qui a été utilisée telle que dans l'étape suivante.

C) 3-Cyclohexyl-5-éthoxy-1,3-dihydro-2*H*-benzimidazol-2-one.

On chauffe à 170-180°C pendant 1 heure 30 minutes un mélange de 3,05 g de l'huile obtenue à l'étape B, de 1,6 g d'urée dans 8 ml de 1, 2, 3, 4-tétraméthylbenzène. Après refoidissement, on reprend dans AcOEt, lave à l'eau, sèche sur sulfate de sodium et évapore sous vide. On reprend le résidu dans de l'hexane et essore le précipité marron formé. On chromatographie le précipité sur silice en éluant avec un mélange DCM/AcOEt (50/50 ; v/v). On obtient 1,33 g du produit attendu qui a été précipité par de l'éther isopropylique. F = 203°C.

Préparation 4

5-Chloro-3-cyclohexylméthyl-1,3-dihydro-2*H*-benzimidazol-2-one.

A) 4-Chloro-1-nitro-2-[(cyclohexylméthyl)amino]benzène

A une solution de 8,1 g de 1,2-dinitro-4-chlorobenzène dans 20 ml d'éthanol 95 on ajoute goutte à goutte une solution de 13,6 g de cyclohexylméthylamine dans 10 ml d'éthanol 95. La température s'élève à 50°C. On maintient le milieu réactionnel sous agitation pendant 2 heures, puis on évapore sous vide le solvant. On reprend le résidu au DCM, lave à l'eau, puis avec de l'acide chlorhydrique 2N, et ensuite à l'eau, sèche sur $Na_2SO_4$ puis évapore

sous vide le solvant. On chromatographie le résidu sur silice en éluant avec de l'éther isopropylique. Après recristallisation dans l'heptane on obtient 4,21 g du produit attendu, F = 73°C.

B) 4-Chloro-1-amino-2-[(cyclohexylméthyl)amino]benzène.

On porte au reflux une solution de 21,5 g du produit obtenu à l'étape A) et de 13,4 g de fer en poudre dans un mélange de 25 ml d'eau et de 25 ml d'éthanol. Puis on ajoute lentement une solution de 50 ml d'acide chlorhydrique concentré dans un mélange de 34 ml d'eau et 34 ml d'éthanol. On maintient le reflux pendant 2 heures. Puis on coule le milieu réactionnel sur de la glace et on traite avec une solution saturée de $NaHCO_3$, puis on extrait au DCM, lave à l'eau, sèche sur $Na_2SO_4$ et évapore sous vide le solvant. On chromatographie le résidu sur silice en éluant avec DCM. Après cristallisation dans l'heptane on obtient 7,2 g du produit attendu, F = 62°C.

C) 5-Chloro-3-cyclohexylméthyl-1,3-dihydro-2*H*-benzimidazol-2-one.

On chauffe à reflux pendant 5 minutes un mélange de 7,2 g du composé obtenu à l'étape B), 6,7 g de 1,1'-carbonyldiimidazole dans 100 ml d'acétonitrile. On évapore sous vide le solvant, reprend le résidu à l'eau, extrait au DCM, lave par une solution saturée de $NaHCO_3$, sèche sur $Na_2SO_4$ et évapore sous vide. On chromatographie le résidu sur silice en éluant par le mélange DCM/MeOH (95/5 ; v/v). On obtient 5,6 g du produit attendu, F = 173°C.

Préparation 5

5-Ethoxy-1,3-dihydro-3-(4(a,e)-méthylcyclohexyl)-2*H*-benzimidazol-2-one.

A) 4-Chloro-2-(4(a,e)-méthylcyclohexyl)amino-1-nitrobenzène.

A une solution de 20 g de 4-chloro-1,2-dinitrobenzène dans 80 ml d'EtOH 95 on ajoute goutte à goutte 30 g de 4-méthylcyclohexylamine (mélange d'isomères) et laisse 24 heures sous agitation à TA. On évapore sous vide le mélange réactionnel, extrait le résidu à l'heptane et évapore sous vide le solvant. On obtient 25 g du produit attendu sous forme d'huile rouge et que l'on utilise tel quel à l'étape suivante.

B) 4-Ethoxy-2-(4(a,e)-méthylcyclohexyl)amino-1-nitrobenzène.

On prépare ce composé selon le mode opératoire décrit à la préparation 3 étape A à partir de 25 g du composé obtenu à l'étape précédente. On obtient 14 g du produit attendu, F = 85°C.

C) 1-Amino-4-éthoxy-2-(4(a,e)-méthylcyclohexyl)aminobenzène et 1-amino-4-éthoxy-2-(4(a)-méthylcyclohexyl)aminobenzène.

On hydrogène à TA, sous une pression de 2 bars, un mélange de 14 g du composé obtenu à l'étape précédente, 0,8 g de palladium sur charbon à 5 % dans 150 ml d'EtOH 95. On filtre le catalyseur sur Célite et évapore sous vide le filtrat. On reprend le résidu dans l'heptane à chaud et après refroidissement on essore le solide formé. On obtient 6 g du produit attendu sous forme du mélange d'isomères axial-équatorial, F = 92°C. On concentre sous vide les jus d'essorage précédents et on obtient 3 g de l'isomère axial du produit attendu.

D) 4-Ethoxy-1-éthoxycarboxamido-2-(4(a,e)-méthylcyclohexyl)aminobenzène.

On porte à reflux pendant 1 heure un mélange de 5,8 g du composé obtenu à l'étape précédente, 10 g de chloroformiate d'éthyle dans 100 ml de chloroforme. On évapore sous vide le solvant et chromatographie le résidu sur silice en éluant au DCM. On obtient 5 g du produit attendu qui est utilisé tel quel à l'étape suivante.

E) 5-Ethoxy-1,3-dihydro-3-(4(a,e)-méthylcyclohexyl)-2*H*-benzimidazol-2-one.

A une solution d'éthylate de sodium, préparée en ajoutant 0,75 g de sodium dans 30 ml d'EtOH absolu, on ajoute 5 g du composé obtenu à l'étape précédente et chauffe à reflux pendant 6 heures. On évapore sous vide le mélange réactionnel, extrait le résidu au DCM, lave à l'eau, sèche sur $Na_2SO_4$ et évapore sous vide. On obtient 3 g du produit attendu après cristallisation dans l'éther iso, F = 190°C.

Préparation 6

5-Ethoxy-1,3-dihydro-3-(4(a)-méthylcyclohexyl)-2*H*-benzimidazol-2-one, isomère axial.

A) 4-Ethoxy-1-éthoxycarboxamido-2-(4(a)-methylcyclohexyl)aminobenzène.

On prépare ce composé selon le mode opératoire décrit dans la Préparation 5 étape D à partir de 3 g de l'isomère axial du composé obtenu à la Préparation 5 étape C. On chromatographie sur silice en éluant par le mélange DCM/AcOEt (90/10 ; v/v). On obtient 2,8 g du produit attendu après cristallisation dans l'éther iso, F =

183°C.

B) 5-Ethoxy-1,3-dihydro-3-(4(a)-méthylcyclohexyl)-2H-benzimidazol-2-one, isomère axial.

On prépare ce composé selon le mode opératoire décrit à la Préparation 5 étape E à partir de 2,8 g du composé obtenu à l'étape précédente. On obtient 1,3 g du produit attendu après cristallisation dans l'éther iso, F = 170°C.

Préparation 7

5-Ethoxy-1,3-dihydro-3-(4(a,e)-méthoxycyclohexyl)-2*H*-benzimidazol-2-one.

A) 4(a,e)-méthoxycyclohexylamine.

On hydrogène pendant 3 heures, à la température de 75-80°C, sous 45 bars de pression, un mélange de 100 g de 4-méthoxyaniline, 48 g de palladium sur charbon à 5 % dans 400 ml d'AcOH. On filtre le catalyseur, ajoute 20 ml d'eau au filtrat et évapore sous vide le filtrat. On reprend le résidu avec 100 ml d'eau, refroidit à 0°C, alcalinise par ajout de NaOH concentrée, extrait à l'éther, sèche sur $Na_2SO_4$ et évapore le solvant à pression atmosphérique. On distille l'huile obtenue à pression atmosphérique. On obtient 31 g du produit attendu sous forme d'huile, Eb = 183-188°C.

B) 4-Chloro-2-(4(a,e)-méthoxycyclohexyl)amino-1-nitrobenzène.

On laisse 15 heures sous agitation un mélange de 12 g de 4-chloro-1,2-dinitrobenzène, 7 g du composé obtenu à l'étape précédente dans 30 ml d'EtOH. On évapore sous vide le solvant, extrait le résidu à l'éther, lave à l'eau, par une solution 1N de NaOH, par une solution 1N d'HCl, à l'eau, sèche sur $Na_2SO_4$ et évapore sous vide le solvant. On chromatographie le résidu sur silice en éluant au DCM. On obtient 6,6 g du produit attendu, sous forme d'huile, qui est utilisé tel quel à l'étape suivante.

C) 4-Ethoxy-2-(4(a,e)-méthoxycyclohexyl)amino-1-nitrobenzène.

On prépare ce composé selon le mode opératoire décrit à la Préparation 3 étape A à partir de 15,4 g du composé obtenu à l'étape précédente. On obtient 12 g du produit attendu après cristallisation dans l'éther iso, F = 93°C.

D) 1-Amino-4-éthoxy-2-(4(a,e)-méthoxycyclohexyl)aminobenzène.

On hydrogène à TA, à pression atmosphérique, pendant 4 heures, un mélange de 10 g du composé obtenu à l'étape précédente, 3 g de palladium sur charbon à 5 % dans 100 ml d'EtOH. On filtre le catalyseur et évapore sous vide le filtrat. On obtient 8,5 g du produit attendu, sous forme d'huile rouge, qui est utilisé tel quel à l'étape suivante.

E) 4-Ethoxy-1-éthoxycarboxamido-2-(4(a,e)-méthoxycyclohexyl) aminobenzène.

On refroidit à 10°C une solution de 8,4 g du composé obtenu à l'étape précédente, 13 g de triéthylamine dans 100 ml de DCM et ajoute, goutte à goutte, une solution de 5 ml de chloroformiate d'éthyle dans 15 ml de THF. On laisse 3 heures sous agitation en laissant remonter la température à TA et évapore sous vide les solvants. On extrait le résidu à l'éther iso, lave à l'eau, par une solution à 10 % de $Na_2CO_3$, sèche sur $Na_2SO_4$ et évapore sous vide le solvant. On obtient 12 g du produit attendu qui est utilisé tel quel à l'étape suivante.

F) 5-Ethoxy-1,3-dihydro-3-(4(a,e)-méthoxycyclohexyl)-2*H*-benzimidazol-2-one.

On chauffe à reflux pendant 4 heures un mélange de 12 g du composé obtenu à l'étape précédente, 4,1 g d'éthylate de sodium dans 150 ml de THF. On évapore sous vide le mélange réactionnel, dissout le résidu dans 50 ml d'eau, acidifie à pH = 1 par ajout d'HCl 2N, essore le précipité formé et lave ce dernier à l'eau. On chromatographie le précipité sur silice en éluant au DCM puis par le mélange DCM/AcOEt (70/30 ; v/v). On obtient 7,8 g du produit attendu, F = 201°C.

Préparation 8.

5-Ethoxy-1,3-dihydro-3-[4(a,e)-(2-méthoxyéthoxy)cyclohexyl]-2*H*-benzimidazol-2-one.

A) 4-(2-méthoxyéthoxy)-1-nitrobenzène.

On chauffe à reflux pendant 20 heures un mélange de 40 g de 4-nitrophénol, 41 g de 1-bromo-2-méthoxyéthane, 45 g de $K_2CO_3$, 80 ml de tris[2-(2-méthoxyéthoxy)éthyl]amine dans 80 ml d'acétone. On filtre un insoluble et concentre sous vide le filtrat. On reprend le résidu dans l'eau, essore le précipité formé et lave à l'eau. On dissout le précipité dans l'AcOEt, lave par une solution 1N de NaOH, à l'eau, par une solution 1N d'HCl, à l'eau, sèche sur $Na_2SO_4$ et évapore sous vide le solvant. On obtient 59 g du produit attendu qui est utilisé tel quel à l'étape suivante.

B) 4-(2-méthoxyéthoxy)aniline.

On hydrogène à 40°C, à pression atmosphérique, pendant 5 heures, un mélange de 59 g du composé obtenu à l'étape précédente, 6 g de palladium sur charbon à 5 % dans 400 ml d'EtOH. On filtre le catalyseur et évapore sous vide le filtrat. On obtient 43 g du produit attendu qui est utilisé tel quel à l'étape suivante.

C) 4(a,e)-(2-méthoxyéthoxy)cyclohexylamine.

On prépare ce composé selon le mode opératoire décrit à la Préparation 7 étape A à partir de 43 g du composé obtenu à l'étape précédente. On distille l'huile obtenue sous pression réduite. On obtient 19 g du produit attendu sous forme d'huile, Eb = 123-127°C sous 15 mm de Hg.

D) 4-Chloro-2-[[4(a,e)-(2-méthoxyéthoxy)cyclohexyl]amino]-1-nitrobenzène.

On laisse 15 heures sous agitation à TA un mélange de 19 g du composé obtenu à l'étape précédente, 22,2 g de 4-chloro-1,2-dinitrobenzène, 20 ml de triéthylamine dans 30 ml d'EtOH. On évapore sous vide le mélange réactionnel, reprend le résidu à l'eau, extrait à l'éther, lave par une solution 1N d'HCl, à l'eau, par une solution 1N de NaOH, à l'eau, sèche sur $Na_2SO_4$ et évapore sous vide. On chromatographie le résidu sur silice en éluant à l'éther iso puis au DCM. On obtient 20 g du produit attendu, sous forme d'huile orange, que l'on utilise tel quel à l'étape suivante.

E) 4-Ethoxy-2-[[4(a,e)-(2-méthoxyéthoxy)cyclohexyl]amino]-1-nitrobenzène.

On prépare une solution d'éthylate de sodium en ajoutant 1,8 g de sodium dans 50 ml d'EtOH, puis on ajoute 19,9 g du composé obtenu à l'étape précédente, 30 ml de tris[2-(2-méthoxyéthoxy)éthyl]amine et 80 ml d'EtOH et chauffe à reflux pendant 5 heures. On évapore sous vide le mélange réactionnel, reprend le résidu par une solution 2N d'HCl, extrait à l'éther, lave à l'eau, sèche sur $Na_2SO_4$ et évapore sous vide. On obtient 17,5 g du produit attendu que l'on utilise tel quel à l'étape suivante.

F) 5-Ethoxy-1,3-dihydro-3-[4(a,e)-(2-méthoxyéthoxy)cyclohexyl]-2*H*-benzimidazol-2-one.

On prépare ce composé selon les modes opératoires décrits à la Préparation 7 étapes D, E puis F à partir de 17,4 g du composé obtenu à l'étape précédente. On obtient 11,5 g du produit attendu, F = 118-120°C.

Préparation 9.

5-Ethoxy-1,3-dihydro-3-(2-méthoxy-1,1-diméthyléthyl)-2*H*-benzimidazol-2-one.

A) 4-Chloro-2-[(2-hydroxy-1,1-diméthyléthyl)amino]-1-nitrobenzène.

On chauffe à reflux pendant 36 heures un mélange de 20 g de 4-chloro-1,2-dinitrobenzène, 36 g de 2-amino-2-méthylpropane-1-ol dans 100 ml d'EtOH. On évapore sous vide le mélange réactionnel, extrait le résidu à l'AcOEt, lave par une solution 1N d'HCl, à l'eau, sèche sur $Na_2SO_4$ et évapore sous vide. On chromatographie le résidu sur silice en éluant au DCM. On obtient 16 g du produit attendu que l'on utilise tel quel à l'étape suivante.

B) 4-Chloro-2-[(2-méthoxy-1,1-diméthyléthyl)amino]-1-nitrobenzène.

A une solution de 15 g du composé obtenu à l'étape précédente dans 200 ml de THF, on ajoute, par portions, 1,6 g d'hydrure de sodium et laisse 30 minutes sous agitation à TA. Puis on ajoute 6 ml d'iodure de méthyle et laisse 2 heures sous agitation à TA. On évapore sous vide le solvant, reprend le résidu dans 300 ml d'eau, extrait à l'AcOEt, sèche sur $Na_2SO_4$ et évapore sous vide. On chromatographie le résidu sur silice en éluant au DCM. On obtient 12,5 g du produit attendu que l'on utilise tel quel à l'étape suivante.

C) 4-Ethoxy-2-[(2-méthoxy-1,1-diméthyléthyl)amino]-1-nitrobenzène.

On prépare une solution d'éthylate de sodium en ajoutant 2 g de sodium dans 100 ml d'EtOH, puis ajoute 12,5 g du composé obtenu à l'étape précédente et chauffe à reflux pendant 5 heures. On concentre sous vide le mélange réactionnel, reprend le résidu dans 300 ml d'eau, extrait à l'AcOEt, sèche sur $Na_2SO_4$ et évapore sous vide le solvant. On obtient 12 g du produit attendu que l'on utilise tel quel à l'étape suivante.

D) 1-Amino-4-éthoxy-2- [(2-méthoxy-1,1-diméthyléthyl)amino]benzène.

On hydrogène pendant 24 heures, à 40°C et à pression atmosphérique, un mélange de 12 g du composé obtenu à l'étape précédente, 1,2 g de palladium sur charbon à 5 % dans 250 ml d'AcOEt. On filtre le catalyseur sur Célite et évapore sous vide le filtrat. On obtient 12 g du produit attendu que l'on utilise tel quel à l'étape suivante.

E) 4-Ethoxy-1-éthoxycarboxamido-2-[(2-méthoxy-1,1-diméthyléthyl)amino] benzène.

On chauffe 2 heures à reflux un mélange de 12 g du composé obtenu à l'étape précédente, 14 g de chloroformiate d'éthyle dans 200 ml de chloroforme. Après refroidissement on lave par une solution 1N de NaOH, sèche sur $Na_2SO_4$ et évapore sous vide. On chromatographie le résidu sur silice en éluant par le mélange DCM/MeOH (99/1 ; v/v). On obtient 8,4 g du produit attendu, F = 138°C.

F) 5-Ethoxy-1,3-dihydro-3-(2-méthoxy-1,1-diméthyléthyl)-2*H*-benzimidazol-2-one.

On prépare ce composé selon le mode opératoire décrit à la Préparation 5 étape E à partir de 8,4 g du composé obtenu à l'étape précédente. On obtient 4,9 g du produit attendu après cristallisation dans l'EtOH, F = 149°C.

Préparation 10.

5-Ethoxy-1,3-dihydro-3-(1,1,3,3-tétraméthylbutyl)-2*H*-benzimidazol-2-one.

A) 4-Chloro-2-[(1,1,3,3-tetraméthylbutyl)amino]-1-nitrobenzène.

On chauffe à reflux pendant 16 heures un mélange de 20 g de 4-chloro-1,2-dinitrobenzène, 30 g de tert-octylamine dans 300 ml d'EtOH 95. On évapore sous vide le solvant. On obtient 13 g du produit attendu après cristallisation dans le mélange éther iso/heptane (40/60 ; v/v), F = 108°C.

B) 4-Ethoxy-2-[(1,1,3,3-tetraméthylbutyl)amino]-1-nitrobenzène.

On prépare ce composé selon le mode opératoire décrit dans la Préparation 9 étape C à partir de 18 g du composé obtenu à l'étape précédente. On chromatographie sur silice en éluant à l'heptane. On obtient 4 g du produit attendu que l'on utilise tel quel à l'étape suivante.

C) 1-Amino-4-éthoxy-2-[(1,1,3,3-tetraméthylbutyl)amino]benzène.

On hydrogène à TA et à pression atmosphérique un mélange de 4 g du composé obtenu à l'étape précédente, 0,2 g de palladium sur charbon à 5 % dans 150 ml d'AcOEt. On filtre le catalyseur sur Célite et évapore sous vide le filtrat. On obtient 3,6 g du produit attendu que l'on utilise tel quel à l'étape suivante.

D) 4-Ethoxy-1-éthoxycarboxamido-2-[(1,1,3,3-tétraméthylbutyl) amino]benzène.

On laisse 1 heure sous agitation un mélange de 3,6 g du composé obtenu à l'étape précédente, 2 ml de chloroformiate d'éthyle, 2 ml de triéthylamine dans 100 ml de chloroforme. On lave par une solution 1N de NaOH, à l'eau par une solution 1N d'HCl, à l'eau, sèche sur $Na_2SO_4$ et évapore sous vide le solvant. On obtient 4 g du produit attendu que l'on utilise tel quel à l'étape suivante.

E) 5-Ethoxy-1,3-dihydro-3-(1,1,3,3-tétraméthylbutyl)-2*H*-benzimidazol-2-one.

On prépare une solution d'éthylate de sodium à partir de 0,6 g de sodium et 100 ml d'EtOH, ajoute 4 g du composé obtenu à l'étape précédente et chauffe 3 heures à reflux. On évapore sous vide le solvant, reprend dans 100 ml d'eau, filtre le précipité formé, lave à l'eau puis à l'éther iso. On obtient 2,6 g du produit attendu après séchage, F = 157°C.

Préparation 11.

5-Chloro-1,3-dihydro-3-phényl-2*H*-benzimidazol-2-one.

On prépare ce composé selon le mode opératoire décrit dans Eur. J. Med. Chem. - Chimica Therapeutica, 1981, 16(4), 321-326.

Préparation 12.

3-(2-Chlorophényl)-5-éthoxy-1,3-dihydro-2*H*-benzimidazol-2-one.

A) 4-Chloro-2-[(2-chlorophényl)amino]-1-nitrobenzène.

On chauffe à reflux pendant 96 heures un mélange de 101 g de 4-chloro-1,2-dinitrobenzène, 191 g de 2-chloroaniline dans 750 ml d'EtOH 95. On évapore sous vide le solvant, extrait le résidu au DCM, lave par une solution 3N d'HCl, à l'eau, sèche sur $Na_2SO_4$ et évapore sous vide le solvant. On chromatographie le résidu sur silice en éluant par le mélange DCM/hexane (50/50 ; v/v). On obtient 7 g du produit attendu après cristallisation dans l'EtOH, F = 97° C.

B) 2-[(2-Chlorophényl)amino]-4-éthoxy-1-nitrobenzène.

On prépare ce composé selon le mode opératoire décrit dans la Préparation 3 étape A à partir de 7 g du composé obtenu à l'étape précédente. On obtient 3,3 g du produit attendu après cristallisation dans l'éther iso.

C) 1-Amino-2-[(2-chlorophényl)amino]-4-éthoxybenzène.

On chauffe à reflux un mélange de 3,3 g du composé obtenu à l'étape précédente, 2 g de fer en poudre, dans 3 ml d'eau et 3 ml d'EtOH, puis on ajoute goutte à goutte une solution de 0,17 ml d'HCl concentré dans 0,7 ml d'eau et 0,7 ml d'EtOH. On laisse deux heures à reflux, puis après refroidissement, on alcalinise par ajout de NaOH concentré, filtre le mélange réactionnel sur Célite, et lave abondamment à l'AcOEt. Après décantation du filtrat, on sèche la phase organique sur $Na_2SO_4$ et évapore sous vide le solvant. On chromatographie le résidu sur silice en éluant par le mélange DCM/AcOEt (90/10 ; v/v). On obtient 1,75 g du produit attendu que l'on utilise tel quel à l'étape suivante.

D) 2-[(2-Chlorophényl)amino]-4-éthoxy-1-méthoxycarboxamidobenzène.

On chauffe à reflux pendant 3 heures un mélange de 1,75 g du composé obtenu à l'étape précédente, 3 g de chloroformiate de méthyle dans 30 ml de chloroforme. On évapore sous vide le solvant, extrait le résidu au DCM, lave à l'eau, sèche sur $Na_2SO_4$ et évapore sous vide le solvant. On obtient 1,2 g du produit attendu que l'on utilise tel quel à l'étape suivante.

E) 3-(2-Chlorophényl)-5-éthoxy-1,3-dihydro-2*H*-benzimidazol-2-one.

On prépare ce composé selon le mode opératoire décrit à la Préparation 5 étape E à partir de 1,2 g du composé obtenu à l'étape précédente. Après évaporation sous vide du mélange réactionnel, on reprend le résidu à l'AcOEt, lave à l'eau et le produit précipite. On filtre le précipité et on obtient 1 g du produit attendu après séchage, F = 213°C.

Préparation 13.

5-Ethoxy-3-(tétrahydropyran-4-yl)-1,3-dihydro-2*H*-benzimidazol-2-one.

A) Tétrahydro-4*H*-pyran-4-one oxime.

A une solution de 35 g de tétrahydro-4*H*-pyran-4-one dans 225 ml de pyridine on ajoute une solution de 29 g de chlorhydrate d'hydroxylamine dans 90 ml d'EtOH et laisse 48 heures sous agitation à TA. On concentre le mélange réactionnel jusqu'à 50 ml, ajoute 500 ml d'eau glacée, extrait 6 fois à l'AcOEt, sèche sur $Na_2SO_4$ et évapore sous vide le solvant. On obtient 26 g du produit attendu, F = 94°C.

B) 4-Aminotétrahydropyrane.

On hydrogène pendant 3 heures à 60°C et sous 20 bars de pression un mélange de 32 g du composé obtenu à l'étape précédente dans 300 ml d'EtOH, en présence de nickel de Raney. On filtre le catalyseur, évapore sous vide le solvant et distille l'huile obtenue à pression atmosphérique. On obtient 18 g du produit attendu, Eb = 150-175°C.

C) 4-Chloro-1-nitro-2-[(tétrahydropyran-4-yl)amino]benzène.

On chauffe à 60°C pendant 48 heures un mélange de 33 g de 4-chloro-1,2-dinitrobenzène, 18 g du composé obtenu à l'étape précédente, 22 g de triéthylamine dans 250 ml d'EtOH 96. Après refroidissement, on filtre le précipité formé, lave à l'EtOH puis à l'éther iso. On obtient 24,4 g du produit attendu, F = 155°C.

D) 4-Ethoxy-1-nitro-2-[(tétrahydropyran-4-yl)amino]benzène.

On prépare ce composé selon le mode opératoire décrit à la Préparation 9 étape C à partir de 24,4 g du composé obtenu à l'étape précédente. On obtient 21,4 g du produit attendu après cristallisation dans l'éther iso, F = 117°C.

E) 1-Amino-4-éthoxy-2-[(tetrahydropyran-4-yl)amino]benzène.

On hydrogène à 40°C et à la pression atmosphérique un mélange de 21,4 g du composé obtenu à l'étape précédente, 2 g de palladium sur charbon à 5 % dans 500 ml d'AcOEt. On filtre le catalyseur sur Célite et évapore sous vide le filtrat. On obtient 18 g du produit attendu, F = 101°C.

F) 4-Ethoxy-2-[(tetrahydropyran-4-yl)amino]-1-methoxycarboxamidobenzène.

A une solution de 19 g du composé obtenu à l'étape précédente, 15 ml de triéthylamine dans 500 ml de chloroforme, on ajoute goutte à goutte 30 ml de chloroformiate de méthyle et laisse 3 heures sous agitation à TA. On lave par une solution 1N d'HCl, par une solution 1N de NaOH, sèche sur $Na_2SO_4$ et évapore sous vide le solvant. On obtient 13,8 g du produit attendu après cristallisation dans le mélange éther iso/EtOH(80/20 ; v/v), F = 185°C.

G) 5-Ethoxy-3-(tétrahydropyran-4-yl)-1,3-dihydro-2*H*-benzimidazol-3-one.

On chauffe à reflux pendant 3 heures un mélange de 13,8 g du composé obtenu à l'étape précédente avec une solution d'éthylate de sodium préparée à partir de 3,5 g de sodium dans 300 ml d'EtOH. On évapore sous vide le solvant, reprend le résidu à l'eau, filtre le précipité formé, lave ce dernier à l'AcOEt. On obtient 8,4 g du produit attendu, F = 222°C.

Préparation 14.

3-Cyclohexyl-5-cyclopentyloxy-1,3-dihydro-2*H*-benzimidazol-2-one.

A) 2-Cyclohexylamino-4-cyclopentyloxy-1-nitrobenzène.

On chauffe à 50°C une solution de cyclopentylate de sodium, préparée à partir de 0,9 g de sodium dans 150 ml de cyclopentanol, ajoute 10 g du composé obtenu à la Préparation 1 étape A et 12 ml de tris [2-(2-méthoxyéthoxy) éthyl]amine puis chauffe à 100°C pendant 30 heures. On distille sous vide le cyclopentanol, reprend le résidu à l'eau, extrait à l'AcOEt, lave à l'eau, sèche sur $Na_2SO_4$ et évapore sous vide le solvant. On chromatographie le résidu sur silice en éluant à l'heptane. On obtient 4,7 g du produit attendu, F = 98°C.

B) 1-Amino-2-cyclohexylamino-4-cyclopentyloxybenzène.

On hydrogène pendant 3 heures, à TA et sous 2 bars de pression, un mélange de 4,7 g du composé obtenu à l'étape précédente, 0,3 g de palladium sur charbon à 5 % dans 120 ml d'EtOH 95. On filtre le catalyseur sur Célite et évapore sous vide le filtrat. On obtient 4 g du produit attendu, F = 80°C.

C) 2-Cyclohexylamino-4-cyclopentyloxy-1-éthoxycarboxamidobenzène.

On chauffe à reflux pendant 2 heures un mélange de 4 g du composé obtenu à l'étape précédente, 6 g de chloroformiate d'éthyle dans 50 ml de chloroforme. On lave à l'eau le mélange réactionnel, sèche sur $Na_2SO_4$ et évapore sous vide le solvant. On chromatographie le résidu sur silice en éluant par le mélange DCM/AcOEt (75/25 ; v/v). On obtient 2,6 g du produit attendu, après cristallisation dans l'éther iso, F = 202°C.

D) 3-Cyclohexyl-5-cyclopentyloxy-1,3-dihydro-2*H*-benzimidazol-2-one.

A une solution d'éthylate de sodium préparée à partir de 0,2 g de sodium dans 50 ml d'EtOH, on ajoute 2,5 g du composé obtenu à l'étape précédente et chauffe à reflux pendant 18 heures. On évapore sous vide le solvant, extrait le résidu à l'AcOEt, lave à l'eau, sèche sur $Na_2SO_4$ et évapore sous vide le solvant. On obtient 1,7 g du produit attendu après cristallisation dans l'éther iso, F = 242°C.

Préparation 15.

3-Cyclohexyl-1,3-dihydro-5-(2-méthoxyéthoxy)-2*H*-benzimidazol-2-one.

A) 2-Cyclohexylamino-4-(2-méthoxyéthoxy)-1-nitrobenzène.

A une solution de (2-méthoxy)éthylate de sodium préparée à partir de 0,9 g de sodium dans 100 ml de (2-méthoxy)éthanol, on ajoute 10 g du composé obtenu à la Préparation 1 étape A et 12 ml de tris [2-(2-méthoxyéthoxy) éthyl]amine puis chauffe à reflux pendant 5 heures. On évapore sous vide le mélange réactionnel, extrait le résidu à l'AcOEt, lave à l'eau, sèche sur $Na_2SO_4$ et évapore sous vide le solvant. On chromatographie le résidu sur silice en éluant par le mélange DCM/heptane (50/50 ; v/v). On obtient 7 g du produit attendu qui est utilisé tel quel à l'étape suivante.

B) 1-Amino-2-cyclohexylamino-4-(2-méthoxyéthoxy)benzène.

On hydrogène à TA et sous 2 bars de pression, un mélange de 7 g du composé obtenu à l'étape précédente, 0,5 g de palladium sur charbon à 5 % dans 200 ml d'EtOH 95. On filtre le catalyseur sur Célite et évapore sous vide le filtrat. On obtient 5 g du produit attendu qui est utilisé tel quel à l'étape suivante.

C) 2-Cyclohexylamino-1-éthoxycarboxamido-4-(2-méthoxyéthoxy)benzène.

On chauffe à reflux pendant 3 heures un mélange de 5 g du composé obtenu à l'étape précédente, 6 g de chloroformiate d'éthyle dans 50 ml de chloroforme. On évapore sous vide le solvant et chromatographie le résidu sur silice en éluant au DCM. On obtient 6 g du produit attendu, F = 145°C.

D) 3-Cyclohexyl-1,3-dihydro-5-(2-méthoxyéthoxy)-2*H*-benzimidazol-2-one.

A une solution d'éthylate de sodium, préparée à partir de 0,45 g de sodium dans 100 ml d'EtOH, on ajoute 6 g

du composé obtenu à l'étape précédente et chauffe à reflux pendant 25 heures. On évapore sous vide le solvant, extrait le résidu à l'AcOEt, lave à l'eau, sèche sur $Na_2SO_4$ et évapore sous vide le solvant. On obtient 2,2 g du produit attendu après cristallisation dans l'éther iso, F = 182°C.

Préparation 16.

5-Chloro-3-(3-chlorophényl)-1,3-dihydro-2*H*-benzimidazol-2-one.

A) 4-Chloro-2-[(3-chlorophényl)amino]-1-nitrobenzène.

On chauffe à reflux pendant 72 heures un mélange de 50 g de 2,4-dichloro-1-nitrobenzène, 40 ml de 3-chloroaniline, 43 g d'acétate de sodium anhydre dans 220 ml d'éthylène glycol. Après refroidissement, on filtre le précipité formé et le lave à l'eau. On obtient 39 g du produit attendu après cristallisation dans l'éther iso, F = 112°C.

B) 1-Amino-4-chloro-2-[(3-chlorophényl)amino]benzène.

A un mélange de 38 g du composé obtenu à l'étape précédente, 140 ml d'HCl concentré et 390 ml d'EtOH, on ajoute par portions 64 g d'étain en poudre, en maintenant la température inférieure à 50°C. On laisse 1 heure sous agitation, on filtre le mélange réactionnel sur Célite et évapore sous vide le filtrat. On extrait le résidu au DCM, lave à l'eau, sèche sur $Na_2SO_4$ et évapore sous vide le solvant. On obtient 38 g du produit attendu, F = 82°C.

C) 4-Chloro-2-[(3-chlorophényl)amino]-1-éthoxycarboxamidobenzène.

A un mélange de 18 g du composé obtenu à l'étape précédente, 10 g de carbonate de potassium dans 160 ml de DMF et 55 ml d'eau, on ajoute lentement 6,8 ml de chloroformiate d'éthyle, en maintenant la température à 20°C. On laisse 1 heure sous agitation, ajoute 300 ml d'eau, extrait au DCM, lave à l'eau, sèche sur $Na_2SO_4$ et évapore sous vide le solvant. On chromatographie le résidu sur silice en éluant par le mélange cyclohexane/DCM (80/20 ; v/v). On obtient 19 g du produit attendu, F = 96°C.

D) 5-Chloro-3-(3-chlorophényl)-1,3-dihydro-2*H*-benzimidazol-2-one.

A une solution d'éthylate de sodium préparée à partir de 2,6 g de sodium dans 100 ml d'EtOH, on ajoute 18 g du composé obtenu à l'étape précédente et chauffe à 60°C pendant 1 heure. On évapore sous vide le solvant, reprend le résidu par 200 ml d'eau, acidifie à pH = 1 par ajout d'HCl concentré, filtre le précipité formé. On obtient 12,6 g du produit attendu, F = 245°C.

Préparation 17.

3-(1-Benzylpipérid-4-yl)-5-chloro-1,3-dihydro-2*H*-benzimidazol-2-one.

A) 2-[(1-Benzylpipérid-4-yl)amino]-4-chloro-1-nitrobenzène.

On chauffe à 100°C une solution de 38,4 g de 2,4-dichloro-1-nitrobenzène dans 160 ml de 2-éthoxyéthanol. Puis on ajoute lentement une solution de 152,23 g de N-benzyl-4-aminopipéridine dans 40 ml de 2-éthoxyéthanol. On porte au reflux pendant 5 heures. On évapore sous vide le solvant et reprend le résidu par $H_2O$, on extrait par AcOEt, lave à l'eau, sèche sur $Na_2SO_4$ et évapore sous vide le solvant. On chromatographie le résidu sur silice en éluant avec de l'éther isopropylique. On obtient 24,4 g du produit attendu après cristallisation dans l'éther isopropylique, F = 84°C.

B) 1-Amino-2-[(1-benzylpipérid-4-yl)amino]-4-chlorobenzène.

On porte au reflux un mélange de 20,75 g du composé obtenu à l'étape A et de 10 g de fer en poudre dans 19 ml d'eau et 19 ml d'éthanol. On ajoute goutte à goutte à ce mélange une solution de 37,5 ml d'acide chlorhydrique concentré dans 25 ml d'eau et 25 ml d'éthanol et on maintient le reflux pendant 1 heure 30 minutes. Après refroidissement on coule le mélange réactionnel sur de la glace, puis on traite avec une solution saturée de $NaHCO_3$ et extrait avec AcOEt. On lave à l'eau, sèche sur $Na_2SO_4$ et évapore sous vide le solvant. On chromatographie le résidu sur silice en éluant avec un mélange DCM/MeOH (92/8 ; v/v). Après cristallisation dans l'éther isopropylique, on obtient 12,71 g du produit attendu, F = 108°C.

C) 3-(1-Benzylpipérid-4-yl)-5-chloro-1,3-dihydro-2*H*-benzimidazol-2-one.

On maintient au reflux pendant 3 heures un mélange de 12,71 g du composé obtenu à l'étape B, de 8,9 g de 1,1'-carbonyldiimidazole dans 130 ml d'acétonitrile. On évapore le solvant sous vide et reprend le résidu par de l'eau, extrait au DCM, lave par une solution saturée de $NaHCO_3$, lave à l'eau, sèche sur $Na_2SO_4$ et évapore sous vide le solvant. On chromatographie le résidu sur silice en éluant avec un mélange DCM/MeOH (92/8 ; v/v). Après

recristallisation dans l'éthanol absolu, on obtient 8,9 g du produit attendu, F = 204-206°C.

Préparation 18.

5-Chloro-3-cycloheptyl-1,3-dihydro-2*H*-benzimidazol-2-one.

A) 4-Chloro-2-cycloheptylamino-1-nitrobenzène.

On laisse 15 heures sous agitation à TA un mélange de 18,2 g de 4-chloro-1,2-dinitrobenzène, 31 g de cycloheptylamine dans 55 ml d'EtOH 95. On concentre sous vide le mélange réactionnel, extrait le résidu à l'AcOEt, lave par une solution d'HCl 1N, à l'eau, sèche sur $Na_2SO_4$ et évapore sous vide le solvant. On chromatographie le résidu sur silice en éluant à l'éther de pétrole. On obtient 13 g du produit attendu après cristallisation dans l'isopropanol.

B) 1-Amino-4-chloro-2-cycloheptylaminobenzène.

On chauffe à reflux un mélange de 12,9 g du composé obtenu à l'étape précédente, 8 g de fer en poudre dans 15 ml d'eau et 15 ml d'EtOH. Puis on ajoute goutte à goutte une solution de 30 ml d'HCl concentré dans 20 ml d'EtOH et 20 ml d'eau et on maintient à reflux pendant 1 heures et 30 minutes. Après refroidissement, on filtre le mélange réactionnel sur Célite, lave au MeOH et concentre sous vide le filtrat. On reprend le résidu par de la glace, alcalinise par ajout d'une solution saturée de $NaHCO_3$, extrait au DCM, lave à l'eau, sèche sur $Na_2SO_4$ et évapore sous vide le solvant. On chromatographie le résidu sur silice en éluant à l'éther de pétrole puis à l'éther iso. On obtient 10 g du produit attendu sous forme d'huile que l'on utilise tel quel à l'étape suivante.

C) 5-Chloro-3-cycloheptyl-1,3-dihydro-2*H*-benzimidazol-2-one.

On chauffe à reflux pendant 10 minutes un mélange de 9,9 g du composé obtenu à l'étape précédente, 9,3 g de 1,1'-carbonyldiimidazole dans 150 ml d'acétonitrile. On évapore sous vide le solvant, reprend le résidu par une solution saturée de $NaHCO_3$, extrait au DCM, lave à l'eau, sèche sur $Na_2SO_4$ et évapore sous vide le solvant. On reprend le résidu dans 100 ml d'éther iso et essore le précipité formé. On chromatographie le précipité sur silice en éluant au DCM puis avec le mélange DCM/AcOEt (80/20 ; v/v). On obtient 8,1 g du produit attendu, F = 201°C.

Préparation 19.

5-Ethoxy-1,3-dihydro-3-[4(a,e)-(tétrahydropyran-2(R,S)-yloxy)cyclohexyl]-2*H*-benzimidazol-2-one.

A) 4-Chloro-2-[(4(a,e)-hydroxycyclohexyl)amino]-1-nitrobenzène.

On laisse 15 heures sous agitation à TA un mélange de 19,8 g de 4-chloro-1,2-dinitrobenzène, 45 g de 4-aminocyclohexanol (mélange d'isomères) dans 75 ml d'EtOH. On évapore sous vide le mélange réactionnel, extrait le résidu à l'éther, lave par une solution 1N d'HCl, à l'eau, sèche sur $Na_2SO_4$ et évapore sous vide le solvant. On chromatographie le résidu sur silice en éluant à l'éther iso. On obtient 14,8 g du produit attendu sous forme d'huile que l'on utilise tel quel à l'étape suivante.

B) 4-Chloro-2-[[4(a,e)-(tétrahydropyran-2(R,S)-yloxy)cyclohexyl]amino]-1-nitrobenzène.

On laisse 20 heures sous agitation à TA un mélange de 15,5 g du composé obtenu à l'étape précédente, 10,5 g de 3,4-dihydro-2*H*-pyrane, 0,1 g d'acide paratoluènesulfonique dans 250 ml d'éther. On évapore sous vide le solvant et obtient 23 g du produit attendu sous forme d'huile que l'on utilise tel quel à l'étape suivante.

C) 4-Ethoxy-2-[[4(a,e)-(tétrahydropyran-2(R,S)-yloxy)cyclohexyl]amino]-1-nitrobenzène.

A une solution d'éthylate de sodium préparée à partir de 1,8 g de sodium dans 30 ml d'EtOH on ajoute 21 g du composé obtenu à l'étape précédente, 10 ml de tris [2-(2-méthoxyéthoxy)éthyl]amine et chauffe à reflux pendant 5 heures. On évapore sous vide le mélange réactionnel, reprend le résidu à l'eau, refroidit à 0°C, acidifie à pH = 1 par ajout d'HCl 1N, extrait rapidement à l'éther, lave à l'eau, sèche sur $Na_2SO_4$ et évapore sous vide le solvant. On obtient 23 g du produit attendu sous forme d'huile que l'on utilise tel quel à l'étape suivante.

D) 1-Amino-4-éthoxy-2[[4(a,e)-(tétrahydropyran-2(R,S)-yloxy)cyclohexyl] amino]benzène.

On hydrogène à 35-40°C, à pression atmosphérique pendant 6 heures un mélange de 23 g du produit obtenu à l'étape précédente, 3 g de palladium sur charbon à 5 % et 90 ml d'EtOH. On filtre le catalyseur et évapore sous vide le filtrat. On obtient 20 g du produit attendu sous forme d'huile que l'on utilise tel quel à l'étape suivante.

E) 4-Ethoxy-1-éthoxycarboxamido-2-[[4(a,e)-(tétrahydropyran-2(R,S)-yloxy) cyclohexyl]amino]benzène.

On refroidit à 5°C un mélange de 19,9 g du composé obtenu à l'étape précédente, 27 g de triéthylamine dans

150 ml de DCM, ajoute goutte à goutte une solution de 6,5 ml de chloroformiate d'éthyle dans 25 ml de THF et laisse 3 heures sous agitation en laissant remonter la température à TA. On évapore sous vide le mélange réactionnel, extrait le résidu à l'éther, lave à l'eau, sèche sur $Na_2SO_4$ et évapore sous vide le solvant. On chromatographie le résidu sur alumine en éluant à l'éther iso, puis rechromatographie le produit obtenu sur silice en éluant à l'éther iso puis au DCM. On obtient 4 g du produit attendu sous forme d'huile que l'on utilise tel quel à l'étape suivante.

F) 5-Ethoxy-1,3-dihydro-3-[4(a,e)-(tétrahydropyran-2(R,S)-yloxy)cyclohexyl]-2*H*-benzimidazol-2-one.

A une solution d'éthylate de sodium préparée à partir de 0,3 g de sodium dans 50 ml d'EtOH, on ajoute 4 g du composé obtenu à l'étape précédente et chauffe 3 heures à reflux. On évapore sous vide le mélange réactionnel, extrait le résidu à l'AcOEt, lave à l'eau, sèche sur $Na_2SO_4$ et évapore sous vide le solvant. On obtient 1,5 g du produit attendu après cristallisation dans l'éther iso, F = 135-145°C.

Préparation 20.

5-Ethoxy-1,3-dihydro-3-[2-(N,N-diisopropylamino)éthyl]-2*H*-benzimidazol-2-one.

A) 4-Chloro-2-[N-[2-(N',N'-diisopropylamino)éthyl]amino]-1-nitrobenzène.

A une solution de 19,5 g de 4-chloro-1,2-dinitrobenzène dans 150 ml d'EtOH on ajoute 39 g de N,N-diisopropyléthylènediamine. La température s'élève à 50°C. On maintient le milieu réactionnel sous agitation pendant 3 heures puis évapore sous vide le solvant. On reprend le résidu dans 200 ml d'isopropanol, refroidit à 0°C, laisse au repos à cette température puis filtre le précipité formé. On obtient 12 g du produit attendu après recristallisation dans l'isopropanol.

B) 4-Ethoxy-2-[N-[2-(N',N'-diisopropylamino)éthyl]amino]-1-nitrobenzène.

On prépare ce composé selon le mode opératoire décrit à la Préparation 3 étape A à partir de 11,3 g du composé obtenu à l'étape précédente. On chromatographie sur silice en éluant par le mélange DCM/MeOH (97/3 ; v/v). On obtient 6,7 g du produit attendu après cristallisation dans l'heptane, F = 89°C.

C) 1-Amino-4-éthoxy-2-[N-[2-(N',N'-diisopropylamino)éthyl]amino]benzène.

On hydrogène pendant 8 heures à TA et à pression atmosphérique un mélange de 6,7 g du composé obtenu à l'étape précédente, 0,55 g de palladium sur charbon à 5 % dans 550 ml d'EtOH. On filtre le catalyseur sur Célite et évapore sous vide le filtrat. On obtient 5,8 g du produit attendu que l'on utilise tel quel à l'étape suivante.

D) 4-Ethoxy-1-éthoxycarboxamido-2-[N-éthoxycarbonyl-N-[2-(N',N'-diisopropylamino)éthyl]amino]benzène.

A une solution de 5,8 g du composé obtenu à l'étape précédente dans 25 ml de chloroforme, on ajoute goutte à goutte, 5,5 ml de chloroformiate d'éthyle et chauffe à reflux pendant 20 heures. On évapore sous vide le solvant, reprend le résidu par une solution saturée de $NaHCO_3$, extrait au DCM, sèche sur $Na_2SO_4$ et évapore sous vide le solvant. On chromatographie le résidu sur silice en éluant par le mélange DCM/MeOH (95/5 ; v/v). On obtient 5,9 g du produit attendu que l'on utilise tel quel à l'étape suivante.

E) 5-Ethoxy-1,3-dihydro-3-[2-(N,N-diisopropylamino)éthyl]-2*H*-benzimidazol-2-one.

A une solution d'éthylate de sodium préparée à partir de 0,8 g de sodium dans 35 ml d'EtOH, on ajoute 5,9 g du composé obtenu à l'étape précédente et chauffe à reflux pendant 3 heures. On évapore sous vide le solvant, reprend le résidu à l'eau, extrait au DCM, sèche sur $Na_2SO_4$ et évapore sous vide le solvant. On reprend le résidu dans l'éther iso, essore le précipité formé. On obtient 2,4 g du produit attendu, F = 120°C.

Préparation 21.

5-Ethoxy-1,3-dihydro-3-[2-(morpholin-4-yl)éthyl]-2*H*-benzimidazol-2-one.

A) 4-Chloro-2-[N-[2-(morpholin-4-yl)éthyl]amino]-1-nitrobenzène.

On laisse 20 heures sous agitation à TA un mélange de 19,5 g de 4-chloro-1,2-dinitrobenzène, 35 g de 4-(2-aminoéthyl)morpholine dans 180 ml d'EtOH. On essore le précipité formé, le lave à l'éther iso. On obtient 17,1 g du produit attendu après deux cristallisations successives dans l'isopropanol.

B) 4-Ethoxy-2-[N-[2-(morpholin-4-yl)éthyl]amino]-1-nitrobenzène.

On prépare ce composé selon le mode opératoire décrit à la Préparation 3 étape A à partir de 8,6 g du composé obtenu à l'étape précédente. On chromatographie sur silice en éluant par le mélange DCM/MeOH (99/1 ; v/v). On

obtient 4,3 g du produit attendu, F = 107°C.

C) 1-Amino-4-éthoxy-2-[N-[2-(morpholin-4-yl)éthyl]amino]-1-nitrobenzène.

On hydrogène pendant 1 heure à TA et à pression atmosphérique un mélange de 4,3 g du composé obtenu à l'étape précédente, 0,4 g de palladium sur charbon à 5 % dans 400 ml d'EtOH. On filtre le catalyseur sur Célite et évapore sous vide le filtrat. On chromatographie le résidu sur silice en éluant par le mélange DCM/MeOH (95/5 ; v/v). On obtient 3,7 g du produit attendu sous forme d'huile que l'on utilise tel quel à l'étape suivante.

D) 4-Ethoxy-1-éthoxycarboxamido-2-[N-[2-(morpholin-3-yl)éthyl]amino] benzène.

A une solution de 3,7 g du composé obtenu à l'étape précédente dans 20 ml de chloroforme, on ajoute à TA, goutte à goutte, 3 ml de chloroformiate d'éthyle et chauffe à reflux pendant 4 heures. On évapore sous vide le solvant, reprend le résidu par une solution saturée de $NaHCO_3$, extrait au DCM, sèche sur $Na_2SO_4$ et évapore sous vide le solvant. On obtient 4,5 g du produit attendu que l'on utilise tel quel à l'étape suivante.

E) 5-Ethoxy-1,3-dihydro-3-[2-(morpholin-4-yl)éthyll-2*H*-benzimidazol-2-one.

A une solution d'éthylate de sodium préparée à partir de 0,6 g de sodium dans 25 ml d'EtOH, on ajoute 4,5 g du composé obtenu à l'étape précédente et chauffe à reflux pendant 3 heures. On évapore sous vide le solvant, reprend le résidu à l'eau, extrait au DCM, sèche sur $Na_2SO_4$ et évapore sous vide le solvant. On chromatographie le résidu sur silice en éluant par le mélange DCM/MeOH (95/5 ; v/v). On reprend le produit obtenu dans l'éther iso, essore le précipité formé. On obtient 0,85 g du produit attendu, F = 160°C.

Préparation 22.

5-Ethoxy-1,3-dihydro-3-(4(a,e)-diméthylaminocyclohexyl)-2*H*-benzimidazol-2-one.

A) 4-Diméthylaminocyclohexylamine.

On hydrogène à la température de 75-90°C, sous 50 bars de pression, un mélange de 68g de 4-diméthylaminoaniline, 34 g de palladium sur charbon à 5 % dans 250 ml d'AcOH. On filtre le catalyseur, lave à l'eau et évapore sous vide le filtrat. On reprend la résidu à l'eau, alcalinise par ajout de NaOH concentrée, extrait à l'AcOEt, sèche sur $Na_2SO_4$ et évapore sous vide le solvant. On distille l'huile obtenue sous pression réduite. On obtient 16,2 g du produit attendu sous forme d'huile, Eb = 102-110°C sous 20 mm de Hg.

B) 4-Chloro-2-[(4(a,e)-diméthylaminocyclohexyl)amino]-1-nitrobenzène.

On laisse 20 heures sous agitation à TA un mélange de 22 g de 4-chloro-1,2-dinitrobenzène, 16 g du composé obtenu à l'étape précédente, 20 ml de triéthylamine dans 30 ml d'EtOH. On évapore sous vide le mélange réactionnel, reprend le résidu à l'eau, extrait à l'AcOEt, lave à l'eau, sèche sur $Na_2SO_4$ et évapore sous vide le solvant. On chromatographie le résidu sur alumine en éluant au DCM. On obtient 14,9 g du produit attendu sous forme d'huile qui cristallise, F = 85°C.

C) 4-Ethoxy-2-[(4(a,e)-diméthylaminocyclohexyl)amino]-1-nitrobenzène.

A une solution d'éthylate de sodium préparée à partir de 1,5 g de sodium dans 80 ml d'EtOH, on ajoute 14,9 g du composé obtenu à l'étape précédente, 15 ml de tris[2-(2-méthoxyéthoxy)éthyl]amine et chauffe à reflux pendant 5 heures. On évapore sous vide le mélange réactionnel, reprend le résidu à l'eau, extrait à l'éther iso, lave à l'eau, sèche sur $Na_2SO_4$ et évapore sous vide le solvant. On obtient 9 g du produit attendu après cristallisation dans l'éther iso, F = 75°C.

D) 1-Amino-4-éthoxy-2-[(4(a,e)-diméthylaminocyclohexyl) amino]benzène.

On hydrogène pendant 3 heures à TA et à pression atmosphérique un mélange de 9 g du composé obtenu à l'étape précédente, 2 g de palladium sur charbon à 5 % et 40 ml d'EtOH. On filtre le catalyseur, lave au MeOH et évapore sous vide le filtrat. On obtient 7,3 g du produit attendu sous forme d'huile que l'on utilise tel quel à l'étape suivante.

E) 4-Ethoxy-1-éthoxycarboxamido-2-[(4(a,e)-diméthylaminocyclohexyl)amino] benzène.

On refroidit à 10°C un mélange de 7,2 g du composé obtenu à l'étape précédente, 12 g de triéthylamine dans 70 ml de DCM et ajoute goutte à goutte une solution de 2,9 ml de chloroformiate d'éthyle dans 10 ml de DCM. On laisse 3 heures sous agitation en laissant remonter la température à TA et évapore sous vide. On reprend le résidu à l'eau, extrait au DCM, sèche sur $Na_2SO_4$ et évapore sous vide le solvant. On obtient 3,7 g du produit attendu après cristallisation dans l'éther iso, F = 193-195°C.

F) 5-Ethoxy-1,3-dihydro-3-(4(a,e)-diméthylaminocyclohexyl)-2*H*-benzimidazol-2-one.

A une solution d'éthylate de sodium préparée à partir de 0,3 g de sodium dans 30 ml d'EtOH, on ajoute 3,6 g du composé obtenu à l'étape précédente et chauffe à reflux pendant 5 heures. On reprend le résidu à l'eau, essore le précipité formé et lave ce dernier à l'eau, à l'isopropanol et au pentane. On obtient 2,15 g du produit attendu, F = 215-217°C.

Préparation 23.

5-Ethoxy-1,3-dihydro-3-(4-méthylpipérazin-1-yl)-2*H*-benzimidazol-2-one.

A) 4-Chloro-2-[(4-méthylpipérazin-1-yl)amino]-1-nitrobenzène.

On laisse 24 heures sous agitation à TA un mélange de 35 g de 4-chloro-1,2-dinitrobenzène, 20 g de 1-amino-4-méthylpipérazine dans 200 ml d'EtOH 96. On évapore sous vide le mélange réactionnel et chromatographie le résidu sur silice en éluant par le mélange DCM/AcOEt (80/20 ; v/v). On obtient 17,5 g du produit attendu après cristallisation dans le mélange éther iso/heptane (50/50 ; v/v), F = 108°C.

B) 4-Ethoxy-2-[(4-méthylpipérazin-1-yl)amino]-1-nitrobenzène.

A une solution d'éthylate de sodium préparée à partir de 1,5 g de sodium dans 85 ml d'EtOH, on ajoute 17,5 g du composé obtenu à l'étape précédente, 13 ml de tris[2-(2-méthoxyéthoxy)éthyl]amine et chauffe à reflux pendant 4 heures. On évapore sous vide le mélange réactionnel, reprend le résidu à l'eau, extrait au DCM, sèche sur $Na_2SO_4$ et évapore sous vide le solvant. On obtient 13,5 g du produit attendu après cristallisation dans l'éther iso, F = 145°C.

C) 1-Amino-4-éthoxy-2-[(4-méthylpipérazin-1-yl)amino]benzène.

On hydrogène pendant 3 heures à TA et à pression atmosphérique un mélange de 13,5 g du composé obtenu à l'étape précédente, 0,75 g de palladium sur charbon à 5 % et 350 ml d'EtOH 96. On filtre le catalyseur et évapore sous vide le filtrat. On obtient 10,5 g du produit attendu que l'on utilise tel quel à l'étape suivante.

D) 4-Ethoxy-1-éthoxycarboxamido-2-[(4-méthylpipérazin-1-yl)amino]benzène.

On refroidit au bain de glace un mélange de 5 g du composé obtenu à l'étape précédente, 2,2 ml de triéthylamine dans 60 ml de DCM et ajoute goutte à goutte une solution de 2,2 ml de chloroformiate d'éthyle dans 20 ml de DCM. On laisse 16 heures sous agitation en laissant remonter la température à TA et évapore sous vide le solvant. On chromatographie le résidu sur silice en éluant par le mélange DCM/MeOH (90/10 ; v/v). On obtient 2,5 g du produit attendu que l'on utilise tel quel à l'étape suivante.

E) 5-Ethoxy-1,3-dihydro-3-(4-méthylpipérazin-1-yl)-2*H*-benzimidazol-2-one.

A une solution d'éthylate de sodium préparée à partir de 0,36 g de sodium dans 15 ml d'EtOH on ajoute une solution de 2,5 g du composé obtenu précédemment dans 20 ml d'EtOH et chauffe à reflux pendant 8 heures. On évapore sous vide, reprend le résidu à l'eau, extrait à l'AcOEt, lave à l'eau, sèche sur $Na_2SO_4$ et évapore sous vide le solvant. On chromatographie le résidu sur silice en éluant par le mélange DCM/AcOEt (90/10 ; v/v). On obtient 0,75 g du produit attendu après cristallisation dans l'éther iso, F = 203°C.

Préparation 24.

5-Ethoxy-1,3-dihydro-3-(morpholin-4-yl)-2*H*-benzimidazol-2-one.

A) 4-Chloro-2-[(morpholin-4-yl)amino]-1-nitrobenzène.

On laisse 48 heures sous agitation à TA un mélange de 45 g de 4-chloro-1,2-dinitrobenzène, 25 g de 4-aminomorpholine, 30 g de triéthylamine dans 250 ml d'EtOH 96. On filtre le précipité formé et lave ce dernier à l'éther iso. On obtient 30,2 g du produit attendu, F = 155°C.

B) 4-Ethoxy-2-[(morpholin-4-yl)amino]-1-nitrobenzène.

A une solution d'éthylate de sodium préparée à partir de 3,5 g de sodium dans 250 ml d'EtOH, on ajoute 30,2 g du composé obtenu à l'étape précédente et chauffe à reflux pendant 5 heures. On évapore sous vide le solvant, extrait le résidu à l'AcOEt, lave à l'eau, sèche sur $Na_2SO_4$ et évapore sous vide le solvant. On obtient 19 g du produit attendu après cristallisation dans l'EtOH, F = 152°C.

C) 1-Amino-4-éthoxy-2-[(morpholin-4-yl)amino]benzène.

On hydrogène à TA et pression atmosphérique un mélange de 19 g du composé obtenu à l'étape précédente,

2 g du palladium sur charbon à 5 % dans 1000 ml d'AcOEt. On filtre le catalyseur et évapore sous vide le filtrat. On obtient 14 g du produit attendu, sous forme d'huile, utilisé tel quel à l'étape suivante.

D) 4-Ethoxy-1-éthoxycarboxamido-2-[(morpholin-4-yl)amino]benzène.

On refroidit au bain de glace un mélange de 14 g du composé obtenu à l'étape précédente, 5 ml de triéthylamine dans 300 ml de chloroforme et ajoute 10 ml de chloroformiate d'éthyle. On laisse 30 minutes sous agitation à TA, lave le mélange réactionnel par une solution de NaOH 1N, sèche la phase organique sur $Na_2SO_4$ et évapore sous vide le solvant. On reprend le résidu dans l'éther iso, filtre un insoluble et chromatographie le filtrat sur silice en éluant par le mélange DCM/MeOH (99/1 ; v/v). On obtient 5 g du produit attendu que l'on utilise tel quel à l'étape suivante.

E) 5-Ethoxy-1,3-dihydro-3-(morpholin-4-yl)-2*H*-benzimidazol-2-one.

A une solution d'éthylate de sodium préparée à partir de 1 g de sodium dans 50 ml d'EtOH, on ajoute 5 g du composé obtenu à l'étape précédente et chauffe à reflux pendant 5 heures. On évapore sous vide, extrait le résidu à l'AcOEt, lave à l'eau, sèche sur $Na_2SO_4$ et évapore sous vide le solvant. On obtient 1,87 g du produit attendu après cristallisation dans le DCM et recristallisation dans l'EtOH, F = 228°C.

Préparation 25.

5-Ethoxy-1,3-dihydro-3-(4-méthoxyphényl)-2*H*-benzimidazol-2-one.

A) 4-Chloro-2-[(4-méthoxyphényl)amino]-1-nitrobenzène.

On chauffe 15 heures à reflux un mélange de 10 g de 4-chloro-1,2-dinitrobenzène, 6,5 g de 4-méthoxyaniline et 16 g de 1,2,3,4-tétraméthylbenzène. Après refroidissement, on ajoute de l'eau, extrait à l'AcOEt, lave par une solution d'HCl 1N, par une solution de NaOH 1N, à l'eau, sèche sur $Na_2SO_4$ et évapore sous vide le solvant. On reprend le résidu à l'éther iso, sépare un insoluble gommeux et chromatographie le filtrat sur alumine en éluant à l'éther iso. On obtient 4,6 g du produit attendu après cristallisation dans l'isopropanol, F = 98°C.

B) 4-Ethoxy-2-[(4-méthoxyphényl)amino]-1-nitrobenzène.

A une solution d'éthylate de sodium préparée à partir de 0,45 g de sodium dans 20 ml d'EtOH, on ajoute 4,5 g du composé obtenu à l'étape précédente, 5 ml de tris[2-(2-méthoxyéthoxy)éthyl]amine et chauffe à reflux pendant 3 heures. On évapore sous vide, reprend le résidu à l'eau, extrait à l'éther, lave par une solution d'HCl 1N, à l'eau, sèche sur $Na_2SO_4$ et évapore sous vide. On obtient 2,2 g du produit attendu après cristallisation dans l'éther iso, F = 109°C.

C) 1-Amino-4-éthoxy-2- [(4-méthoxyphényl)amino]benzène.

On hydrogène pendant 8 heures à TA et pression atmosphérique un mélange de 2,2 g du composé obtenu à l'étape précédente, 0,5 g de palladium sur charbon à 5 % et 20 ml d'EtOH. On filtre le catalyseur, lave à l'EtOH et évapore sous vide le filtrat. On obtient 1,9 g du produit attendu que l'on utilise tel quel à l'étape suivante.

D) 4-Ethoxy-1-éthoxycarboxamido-2-[(4-méthoxyphényl)amino]benzène.

On refroidit à 5°C un mélange de 1,9 g du composé obtenu à l'étape précédente, 3 g de triéthylamine dans 20 ml de DCM et ajoute 1,1 g de chloroformiate d'éthyle. On laisse 3 heures sous agitation en laissant remonter la température à TA et évapore sous vide. On reprend le résidu à l'eau, sèche sur $Na_2SO_4$ et évapore sous vide le solvant. On obtient 2,4 g du produit attendu que l'on utilise tel quel à l'étape suivante.

E) 5-Ethoxy-1,3-dihydro-3-(4-méthoxyphényl)-2*H*-benzimidazol-2-one.

A une solution d'éthylate de sodium préparée à partir de 0,17 g de sodium dans 10 ml d'EtOH on ajoute une solution de 2,4 g du composé obtenu à l'étape précédente dans 15 ml d'EtOH et chauffe à reflux pendant 4 heures. On évapore sous vide, reprend le résidu par une solution d'HCl 1N, essore le précipité formé, le lave à l'eau puis au DCM. On obtient 1,7 g du produit attendu, F = 204°C.

Préparation 26.

5-Ethoxy-1,3-dihydro-3-(4-isopropylphényl)-2*H*-benzimidazol-2-one.

A) 4-Chloro-2-[(4-isopropylphényl)amino]-1-nitrobenzène.

On chauffe à reflux pendant 15 heures un mélange de 15 g de 4-chloro-1,2-nitrobenzène, 10 g de 4-isopropylaniline et 25 ml de Décaline®. On concentre le mélange réactionnel sous 0,01 mm de Hg, reprend le résidu à l'eau, extrait à l'éther, lave par une solution d'HCl 1N, à l'eau, sèche sur $Na_2SO_4$ et évapore sous vide le solvant. On chromatographie le résidu sur silice en éluant à l'éther iso. On obtient 13 g du produit attendu que l'on utilise tel quel à l'étape suivante.

B) 4-Ethoxy-2-[(4-isopropylphényl)amino]-1-nitrobenzène.

On prépare ce composé selon le mode opératoire décrit à la Préparation 25 étape B à partir de 13 g du composé obtenu à l'étape précédente. On chromatographie sur silice en éluant au pentane. On obtient 4,4 g du produit attendu, F = 100,5°C.

C) 1-Amino-4-éthoxy-2-[(4-isopropylphényl)amino]benzène.

On prépare ce composé selon le mode opératoire décrit à la Préparation 25 étape C à partir de 4,3 g du composé obtenu à l'étape précédente. On obtient 4 g du produit attendu que l'on utilise tel quel à l'étape suivante.

D) 4-Ethoxy-1-éthoxycarboxamido-2-[(4-isopropylphényl)amino]benzène.

On prépare ce composé selon le mode opératoire décrit à la Préparation 25 étape D à partir de 3,9 g du composé obtenu à l'étape précédente. On chromatographie sur silice en éluant à l'éther iso. On obtient 5 g du produit attendu que l'on utilise tel quel à l'étape suivante.

E) 5-Ethoxy-1,3-dihydro-3-(4-isopropylphényl)-2*H*-benzimidazol-2-one.

On prépare ce composé selon le mode opératoire décrit à la Préparation 25 étape E à partir de 4,9 g du composé obtenu à l'étape précédente. On obtient 2,8 g du produit attendu après cristallisation dans l'EtOH, F = 202°C.

Préparation 27.

5-Ethoxy-1,3-dihydro-3-(indan-2-yl)-2*H*-benzimidazol-2-one.

A) 4-Chloro-2-[(indan-2-yl)amino]-1-nitrobenzène.

A une solution d'éthylate de sodium préparée à partir de 0,61 g de sodium dans 200 ml d'EtOH, on ajoute 26 g de chlorhydrate de 2-aminoindane puis 20 g de 4-chloro-1,2-dinitrobenzène et laisse 48 heures sous agitation à TA. On évapore sous vide le solvant, extrait le résidu à l'AcOEt, lave à l'eau, sèche sur $Na_2SO_4$ et évapore sous vide le solvant. On obtient 10,3 g du produit attendu après cristallisation dans l'EtOH, F = 108°C.

B) 4-Ethoxy-2-[(indan-2-yl)amino]-1-nitrobenzène.

A une solution d'éthylate de sodium préparée à partir de 2 g de sodium dans 100 ml d'EtOH, on ajoute 10,3 g du composé obtenu à l'étape précédente et chauffe à reflux pendant 4 heures. On évapore sous vide le solvant, extrait à l'AcOEt, lave à l'eau, sèche sur $Na_2SO_4$ et évapore sous vide le solvant. On chromatographie le résidu sur silice en éluant au DCM. On obtient 8,5 g du produit attendu, F = 151°C.

C) 1-Amino-4-éthoxy-2-[(indan-2-yl)amino]benzène.

On hydrogène à TA et à pression atmosphérique un mélange de 8,5 g du composé obtenu à l'étape précédente, 1 g de palladium sur charbon à 5 % dans 500 ml d'AcOEt. On filtre le catalyseur et évapore sous vide le filtrat. On obtient 7,2 g du produit attendu que l'on utilise tel quel à l'étape suivante.

D) 4-Ethoxy-1-éthoxycarboxamido-2-[(indan-2-yl)amino]benzène.

On chauffe à reflux pendant 2 heures un mélange de 7,2 g du composé obtenu à l'étape précédente, 8,4 g de chloroformiate d'éthyle dans 100 ml de chloroforme. On extrait au chloroforme, lave par une solution de NaOH 1N, sèche sur $Na_2SO_4$ et évapore sous vide le solvant. On chromatographie le résidu sur silice en éluant au DCM. On obtient 3,5 g du produit attendu, F = 122°C.

E) 5-Ethoxy-1,3-dihydro-3-(indan-2-yl)-2*H*-benzimidazol-2-one.

On prépare une solution d'éthylate de sodium à partir de 0,7 g de sodium dans 50 ml d'EtOH, ajoute 3,5 g du composé obtenu à l'étape précédente et chauffe 3 heures à reflux. On évapore sous vide le solvant, reprend le résidu à l'eau, extrait au DCM, sèche sur $Na_2SO_4$ et évapore sous vide le solvant. On obtient 2,6 g du produit attendu après cristallisation dans l'EtOH, F = 225°C.

Préparation 28.

3-(Adamant-1-yl)-5-éthoxy-1,3-dihydro-2*H*-benzimidazol-2-one.

A) 2-[(Adamant-1-yl)amino]-4-chloro-1-nitrobenzène.

A une solution de 20 g de 4-chloro-1,2-dinitrobenzène dans 80 ml d'EtOH 96 on ajoute 15,5 ml de triéthylamine puis une suspension de 15 g de 1-aminoadamantane dans 50 ml d'EtOH 96. On chauffe à reflux pendant 7 heures et après refroidissement on essore le précipité formé. On obtient 7 g du produit attendu, F = 146°C.

B) 2-[(Adamant-1-yl)amino]-4-éthoxy-1-nitrobenzène.

A une solution d'éthylate de sodium préparée à partir de 0,6 g de sodium dans 70 ml d'EtOH, on ajoute 7 g du composé obtenu à l'étape précédente, 8 ml de tris[2-(2-méthoxyéthoxy)éthyl]amine et chauffe à reflux pendant 3 heures. On évapore sous vide le solvant, reprend le résidu à l'eau, extrait au DCM, sèche sur $Na_2SO_4$ et évapore sous vide le solvant. On obtient 6 g du produit attendu, F = 147°C.

C) 2-[(Adamant-1-yl)amino]-1-amino-4-éthoxybenzène.

On hydrogène à TA et sous 2 bars de pression un mélange de 6 g du composé obtenu à l'étape précédente, 0,85 g de palladium sur charbon à 5 % et 70 ml d'EtOH 96. On filtre le catalyseur, le lave à l'AcOEt et évapore sous vide le filtrat. On obtient 4,7 g du produit attendu que l'on utilise tel quel à l'étape suivante.

D) 2-[(Adamant-1-yl)amino]-4-éthoxy-1-éthoxycarboxamidobenzène.

On chauffe à reflux pendant 1 heure 30 minutes un mélange de 4,7 g du composé obtenu à l'étape précédente, 6 ml de chloroformiate d'éthyle dans 70 ml de chloroforme. On évapore sous vide le solvant, reprend le résidu dans le mélange éther iso/AcOEt (50/50 ; v/v) et essore le précipité formé. On chromatographie le précipité sur silice en éluant au DCM puis par le mélange DCM/AcOEt (80/20 ; v/v). On obtient 5 g du produit attendu que l'on utilise tel quel à l'étape suivante.

E) 3-(Adamant-1-yl)-5-éthoxy-1,3-dihydro-2*H*-benzimidazol-2-one.

On prépare ce composé selon le mode opératoire décrit à la Préparation 27 étape E à partir de 5 g du composé obtenu précédemment. Après évaporation sous vide du solvant, on reprend le résidu dans l'AcOEt et essore le précipité formé. On obtient 2,5 g du produit attendu, F = 264°C.

Préparation 29.

3-Cycloheptyl-5-éthoxy-1,3-dihydro-2*H*-benzimidazol-2-one

A) 2-Cycloheptylamino-4-éthoxy-1-nitrobenzène.

A une solution d'éthylate de sodium préparée à partir de 2,2 g de sodium dans 250 ml d'EtOH, on ajoute 25 g du composé obtenu à la Préparation 18 étape A, 30 ml de tris[2-(2-méthoxyéthoxy)éthyl]amine et chauffe à reflux pendant 24 heures. On concentre sous vide et chromatographie le résidu sur silice en éluant par le mélange DCM/heptane (50/50 ; v/v). On obtient 14 g du produit attendu, F = 83°C.

B) 1-Amino-2-cycloheptylamino-4-éthoxybenzène.

On hydrogène pendant 24 heures à TA et sous 2 bars de pression, un mélange de 13 g du composé obtenu à l'étape précédente, 0,3 g de palladium sur charbon à 5 % et 250 ml d'EtOH 95. On filtre le catalyseur et évapore sous vide le filtrat. On chromatographie le résidu sur silice en éluant par le mélange DCM/heptane (50/50 ; v/v) puis au DCM et enfin par le mélange DCM/AcOEt (90/10 ; v/v). On obtient 6 g du produit attendu que l'on utilise tel quel à l'étape suivante.

C) 4-Ethoxy-1-éthoxycarboxamido-2-cycloheptylaminobenzène.

On chauffe à reflux pendant 5 heures un mélange de 6 g du composé obtenu à l'étape précédente, 7,5 g de chloroformiate d'éthyle dans 50 ml de chloroforme. On concentre sous vide et chromatographie le résidu sur silice en éluant au DCM. On obtient 4,7 g du produit attendu après cristallisation dans l'éther iso, F = 172°C.

D) 3-Cycloheptyl-5-éthoxy-1,3-dihydro-2*H*-benzimidazol-2-one.

A une solution d'éthylate de sodium préparée à partir de 0,35 g de sodium dans 100 ml d'EtOH on ajoute 4,5 g du composé obtenu à l'étape précédente et chauffe à reflux pendant 24 heures. On concentre sous vide, extrait le résidu à l'AcOEt, lave à l'eau, sèche sur $Na_2SO_4$ et évapore sous vide le solvant. On obtient 2,9 g du produit

attendu après cristallisation dans l'éther iso, F = 204°C.

Préparation 30

5-Chloro-3-(2-chlorophényl)-1,3-dihydro-2*H*-benzimidazol-2-one.

A) 1-Amino-4-chloro-2-[(2-chlorophényl)amino]benzène.

On hydrogène pendant 4 heures à TA et sous 2 bars de pression un mélange de 3 g du composé obtenu à la Préparation 12 étape A, 0,5 g de nickel de Raney et 100 ml d'EtOH 95. On filtre le catalyseur et évapore sous vide le filtrat. On obtient 2 g du produit attendu que l'on utilise tel quel à l'étape suivante.

B) 5-Chloro-3-(2-chlorophényl)-1,3-dihydro-2*H*-benzimidazol-2-one.

On chauffe à reflux pendant 30 minutes un mélange de 2 g du composé obtenu à l'étape précédente, 2 g de 1,1'-carbonyldiimidazole dans 50 ml d'acétonitrile. On concentre sous vide le mélange réactionnel, extrait le résidu à l'AcOEt, lave par une solution d'HCl 1N, à l'eau, par une solution saturée de $NaHCO_3$, à l'eau, sèche sur $Na_2SO_4$ et évapore sous vide le solvant. On obtient 1 g du produit attendu après cristallisation dans l'éther iso, F = 239°C.

Préparation 31

5-Ethoxy-1,3-dihydro-3-(pyrid-2-yl)-2*H*-benzimidazol-2-one.

A) 4-Chloro-1-nitro-2-[(pyrid-2-yl)amino]benzène.

On prépare ce composé selon le mode opératoire décrit dans Eur. J. Med. Chem. - Chim. Ther., 1983, 18 (6), 495-500.

B) 4-Ethoxy-1-nitro-2-[(pyrid-2-yl)amino]benzène.

A une solution d'éthylate de sodium préparée à partir de 0,8 g de sodium dans 50 ml d'EtOH, on ajoute 6,2 g du composé obtenu à l'étape précédente et chauffe à reflux pendant 3 heures. On concentre sous vide le mélange réactionnel, extrait le résidu à l'AcOEt, lave à l'eau, sèche sur $Na_2SO_4$ et évapore sous vide le solvant. On chromatographie le résidu sur silice en éluant par le mélange DCM/pentane (50/50 ; v/v). On obtient 3,5 g du produit attendu que l'on utilise tel quel à l'étape suivante.

C) 1-Amino-4-éthoxy-2-[(pyrid-2-yl)amino]benzène.

On hydrogène à TA et à pression atmosphérique un mélange de 3,5 g du composé obtenu à l'étape précédente, 0,2 g de palladium sur charbon à 5 % dans 250 ml d'AcOEt. On filtre le catalyseur et évapore sous vide le filtrat. On obtient 2,6 g du produit attendu, F = 95°C.

D) 4-Ethoxy-1-éthoxycarboxamido-2-[(pyrid-2-yl)amino]benzène et 4-éthoxy-1-éthoxycarboxamido-2-[N-éthoxycarbonyl-N-(pyrid-2-yl)amino]benzène.

A une solution de 3,6 g du composé obtenu à l'étape précédente, 2 ml de triéthylamine dans 100 ml de DCM, on ajoute 2 ml de chloroformiate d'éthyle et laisse 2 heures sous agitation à TA. On concentre sous vide le mélange réactionnel, extrait le résidu à l'AcOEt, lave à l'eau, sèche sur $Na_2SO_4$ et évapore sous vide le solvant. On obtient 3,2 g du mélange des deux composés cités en titre de l'étape D) et que l'on utilise tel quel à l'étape suivante.

E) 5-Ethoxy-1,3-dihydro-3-(pyrid-2-yl)-2*H*-benzimidazol-2-one.

A une solution d'éthylate de sodium préparée à partir de 0,6 g de sodium dans 100 ml d'EtOH, on ajoute 3,2 g du mélange des composés obtenus à l'étape précédente, et chauffe à reflux pendant 3 heures. On concentre sous vide le mélange réactionnel, extrait le résidu à l'AcOEt, lave à l'eau, sèche sur $Na_2SO_4$ et évapore sous vide le solvant. On obtient 2,7 g du produit attendu après cristallisation dans l'éther iso, F = 205°C.

Préparation 32

3-Cyclopentyl-5-éthoxy-1,3-dihydro-2*H*-benzimidazol-2-one.

A) 4-Chloro-2-cyclopentylamino-1-nitrobenzène.

On laisse une nuit sous agitation à TA, un mélange de 20 g de 4-chloro-1,2-dinitrobenzène, 20 g de cyclopentylamine dans 50 ml d'EtOH 95. On essore le précipité formé, le lave à l'EtOH 95. On obtient 14 g du produit attendu, F = 75°C.

B) 2-Cyclopentylamino-4-éthoxy-1-nitrobenzène.

A une solution d'éthylate de sodium préparée à partir de 2 g de sodium dans 150 ml d'EtOH, on ajoute 20 ml de tris[2-(2-méthoxyéthoxy)éthyl]amine et 14 g du composé obtenu à l'étape précédente puis chauffe à reflux pendant 24 heures. On concentre sous vide le mélange réactionnel, extrait le résidu à l'AcOEt, lave à l'eau, sèche sur $Na_2SO_4$ et évapore sous vide le solvant. On chromatographie le résidu sur silice en éluant par le mélange DCM/heptane (50/50 ; v/v). On obtient 10 g du produit attendu que l'on utilise tel quel à l'étape suivante.

C) 1-Amino-2-cyclopentylamino-4-éthoxybenzène.

On hydrogène pendant 3 heures à TA et sous 2 bars de pression, un mélange de 10 g du composé obtenu à l'étape précédente, 0,6 g de palladium sur charbon à 5 % dans 200 ml d'EtOH 95. On filtre le catalyseur et évapore sous vide le filtrat. On obtient 8,2 g du produit attendu que l'on utilise tel quel à l'étape suivante.

D) 2-Cyclopentylamino-4-éthoxy-1-éthoxycarboxamidobenzène.

On chauffe à reflux pendant 5 heures un mélange de 8 g du composé obtenu à l'étape précédente, 8 ml de chloroformiate d'éthyle dans 50 ml de chloroforme. On concentre sous vide le mélange réactionnel, reprend le résidu dans le mélange, à chaud, éther iso/AcOEt (75/25 ; v/v), essore le précipité et le lave à l'éther iso. On obtient 9,5 g du produit attendu, F = 173°C.

E) 3-Cyclopentyl-5-éthoxy-1,3-dihydro-2*H*-benzimidazol-2-one.

A une solution d'éthylate de sodium préparée à partir de 0,9 g de sodium dans 225 ml d'EtOH, on ajoute 9,5 g du composé obtenu à l'étape précédente, et chauffe à reflux pendant 18 heures. On concentre sous vide le mélange réactionnel, extrait le résidu à l'AcOEt, lave à l'eau, sèche sur $Na_2SO_4$ et évapore sous vide le solvant. On obtient 4 g du produit attendu après cristallisation dans le mélange AcOEt/éther iso (50/50 ; v/v), F = 178°C.

Préparations des halogénures de benzyles, (III).

Préparation 33.

1-Bromométhyl-3,4-diméthoxybenzène.

On chauffe à reflux, pendant 3 heures, sous éclairage d'une lampe UV un mélange de 15,2 g de 3,4-diméthoxytoluène, 17,8 g de N-bromosuccinimide, 0,3 g de peroxyde de dibenzoyle dans 100 ml de $CCl_4$. On essore le précipité et évapore sous vide le filtrat. On reprend le résidu à l'éther, lave à l'eau, sèche sur $Na_2SO_4$ et évapore sous vide. On obtient une huile qui contient un mélange de 50 % du produit attendu et 50 % du produit de départ et que l'on utilise tel quel par la suite.

Préparation 34.

1-Bromométhyl-3-méthoxy-4-nitrobenzène.

On chauffe à reflux pendant 5 heures un mélange de 20 g de 3-méthoxy-4-nitrotoluène, 21,6 g de N-bromosuccinimide, 0,3 g de peroxyde de dibenzoyle dans 120 ml de $CCl_4$. On évapore sous vide le solvant, reprend le résidu à l'eau, extrait au DCM, lave à l'eau, sèche sur $Na_2SO_4$ et évapore sous vide. On obtient 11,35 g du produit attendu après cristallisation dans l'EtOH, F = 98°C.

Préparation 35.

1-Bromométhyl-2-méthoxy-4-nitrobenzène.

A) 2-Hydroxy-4-nitrotoluène.

On laisse 1 heure sous agitation un mélange de 45,6 g de 2-méthyl-5-nitroaniline dans 680 ml d'une solution d'acide sulfurique à 10 %, puis refroidit à 0°C et ajoute, goutte à goutte, une solution de 21,7 g de nitrite de sodium dans 70 ml d'eau. On laisse 5 minutes sous agitation à 0°C puis conserve cette solution à 0°C.

On chauffe à reflux une solution de 460 ml d'acide sulfurique concentré dans 910 ml d'eau, ajoute, par portion, la solution de diazonium préparée ci-dessus et poursuit le chauffage (105°C) jusqu'à la fin du dégagement d'azote. Après refroidissement, on extrait le mélange réactionnel trois fois à l'éther, lave par une solution à 10 % de $NaHCO_3$, extrait le phénol par une solution à 10 % de NaOH, acidifie la phase aqueuse basique par ajout d'HCl concentré, extrait à l'éther, sèche la phase organique sur $Na_2SO_4$ et évapore sous vide le solvant. On chromatographie le résidu sur silice en éluant par le mélange DCM/MeOH (99/1 ; v/v). On obtient 28,6 g du produit attendu après cristallisation dans l'EtOH, F = 118°C.

B) 2-Méthoxy-4-nitrotoluène.

On chauffe à reflux pendant 3 heures un mélange de 15,3 g du composé obtenu précédemment, 20,7 g de carbonate de potassium, 14,2 ml de diméthylsulfate dans 200 ml d'acétone. On évapore sous vide le solvant, reprend le résidu à l'eau, extrait à l'éther, lave à l'eau, sèche sur $Na_2SO_4$ et évapore sous vide. On obtient 10,1 g du produit attendu après cristallisation dans l'EtOH, F = 78°C.

C) 1-Bromométhyl-2-méthoxy-4-nitrobenzène.

On porte à reflux un mélange de 10 g du composé obtenu précédemment dans 90 ml de $CCl_4$ et ajoute en plusieurs fois 0,3 g de péroxyde de dibenzoyle et 10,7 g de N-bromosuccinimide. On laisse 2 heures à reflux sous éclairage d'une lampe UV et évapore sous vide le solvant. On reprend le résidu à l'eau, extrait à l'éther, lave à l'eau, sèche sur $Na_2SO_4$ et évapore sous vide. On obtient 9,3 g du produit attendu après cristallisation dans l'EtOH, F = 94-96°C.

Préparation 36.

4-Bromométhyl-3-méthoxybenzoate de méthyle.

On prépare ce composé selon EP 0179619.

A) 3-Méthoxy-4-méthylbenzoate de méthyle.

A un mélange de 6 g d'acide 3-méthoxy-4-méthylbenzoïque dans 120 ml de MeOH, on ajoute 6 ml de chlorure d'acétyle et laisse 36 heures sous agitation à TA. On évapore sous vide le solvant, dissout le résidu dans 100 ml de MeOH et évapore à nouveau le solvant sous vide. On obtient 6,21 g du produit attendu sous forme d'huile jaune qui cristallise, F = 50-52°C.

B) 4-Bromométhyl-3-méthoxybenzoate de méthyle.

On chauffe à reflux une solution de 6,2 g du composé obtenu à l'étape précédente dans 50 ml de $CCl_4$ et ajoute en plusieurs fois 0,17 g de péroxyde de dibenzoyle et 6,1 g de N-bromosuccinimide. On laisse 2 heures à reflux sous éclairage d'une lampe UV, évapore sous vide le solvant, reprend le résidu à l'eau, extrait à l'AcOEt, lave à l'eau, sèche sur $Na_2SO_4$ et évapore sous vide le solvant. On obtient 4,34 g du produit attendu après cristallisation dans l'EtOH, F = 92°C.

Préparation 37.

N-(1,1-Diméthylpropyl)-4-bromométhylbenzènesulfonamide.

A une solution de 10 g de chlorure de 4-bromométhylbenzènesulfonyle, 10 ml de triéthylamine dans 50 ml de toluène on ajoute à TA, goutte à goutte, une solution de 3,2 g de *tert*-amylamine dans 10 ml de toluène et laisse 3 heures sous agitation à TA. Puis on ajoute de l'eau au mélange réactionnel, après décantation lave la phase organique à l'eau, sèche sur $Na_2SO_4$ et évapore sous vide le solvant. On chromatographie le résidu sur silice en éluant par le mélange DCM/heptane (50/50 ; v/v). On obtient 1 g du produit attendu après cristallisation dans l'éther iso, F = 100°C.

Préparation 38.

Bromure de 4-(6-bromohexyloxy)benzyle.

A) 4-(6-Bromohexyloxy)toluène.

On chauffe à reflux pendant 15 heures un mélange de 13,5 g de *p*-crésol, 26,4 g de 1,6-dibromohexane, 27,6 g de $K_2CO_3$ dans 100 ml d'acétone. On filtre le mélange réactionnel et concentre sous vide le filtrat. On extrait le résidu à l'éther, lave la phase organique par une solution de NaOH 1N, à l'eau, sèche sur $Na_2SO_4$ et évapore sous vide le solvant. On distille l'huile obtenue sous vide. On obtient 15,3 g d'huile incolore, Eb = 125-140°C sous 13,33 Pa.

B) Bromure de 4-(6-bromohexyloxy)benzyle.

On chauffe à reflux pendant 5 heures un mélange de 14,2 g du composé obtenu à l'étape précédente, 9,5 g de N-bromosuccinimide, 0 1 g de péroxyde de dibenzoyle dans 30 ml de $CCl_4$. On filtre le mélange réactionnel et concentre sous vide le filtrat. On chromatographie le résidu huileux sur silice en éluant à l'éther de pétrole. On obtient 5,5 g du produit attendu que l'on utilise tel quel.

EXEMPLE 1

5-Chloro-3-cyclohexyl-1,3-dihydro-1-(3,4-diméthoxybenzyl)-2*H*-benzimidazol-2-one.

A une solution de 0,5 g du composé obtenu à la préparation 1 dans 3 ml de THF, on ajoute par portions 0,066 g d'hydrure de sodium à 80 % dans l'huile et laisse 30 minutes sous agitation. On refroidit au bain de glace, ajoute 0,9 g de 1-bromométhyl-3,4-diméthoxybenzène (évalué à 50 %, en mélange avec du 3,4-diméthoxytoluène) et laisse 72 heures sous agitation en laissant remonter la température à TA. On évapore sous vide le solvant, extrait le résidu à l'éther, lave à l'eau, sèche sur $Na_2SO_4$ et évapore sous vide. On chromatographie le résidu sur silice en éluant par le mélange DCM/AcOEt (90/10 ; v/v). On obtient 0,27 g du produit attendu après concrétisation dans l'hexane, F = 59°C.

EXEMPLE 2

5-Chloro-3-cyclohexyl-1,3-dihydro-1-(2,4-diméthoxybenzyl)-2*H*-benzimidazol-2-one.

On refroidit à -10°C, sous atmosphère d'azote, une solution de 0,537 g de 1-hydroxyméthyl-2,4-diméthoxybenzène dans 5 ml d'éther et ajoute, goutte à goutte, une solution de 0,1 ml de tribromure de phosphore dans 2 ml d'éther. Le 1-bromométhyl-2,4-diméthoxybenzène ainsi obtenu est conservé à -30°C en solution.

On refroidit à -50°C, sous atmosphère d'azote, une solution de 0,531 g du composé obtenu à la Préparation 1 dans 25 ml de THF et ajoute 0,250 g de tert-butylate de potassium puis laisse revenir à 0°C sous agitation. On refroidit à nouveau à -50°C, ajoute 0,357 g de tert-butylate de potassium, puis refroidit à -70°C et ajoute la solution de dérivé bromé préparée ci-dessus. On laisse sous agitation 24 heures en laissant remonter la température à TA. On ajoute 5 ml d'eau, évapore les solvants sous vide, extrait à l'AcOEt, lave à l'eau, sèche sur $Na_2SO_4$ et évapore sous vide. On chromatographie le résidu sur silice en éluant par le mélange DCM/AcOEt (95/5 ; v/v). On obtient 0,44 g du produit attendu qui cristallise dans l'hexane, F = 112°C.

EXEMPLE 3

5-Chloro-3-cyclohexyl-1,3-dihydro-1-(4-nitrobenzyl)-2*H*-benzimidazol-2-one.

A une solution de 1,0 g du composé obtenu à la Préparation 1 dans 10 ml de DMF, on ajoute par portions 0,130 g d'hydrure de sodium à 80 % dans l'huile et laisse 30 minutes sous agitation à TA. On refroidit au bain de glace, ajoute 0,950 g de 1-bromométhyl-4-nitrobenzène et laisse 24 heures sous agitation en laissant remonter la température à TA. On évapore le solvant sous vide, reprend le résidu à l'eau, extrait au DCM, lave à l'eau, sèche sur $Na_2SO_4$ et évapore sous vide. On chromatographie le résidu sur silice en éluant par le mélange DCM/AcOEt (95/5 ; v/v). On obtient 0,90 g du produit attendu après cristallisation dans l'EtOH, F = 139°C.

EXEMPLE 4

5-Chloro-3-cyclohexyl-1,3-dihydro-1-(3-méthoxy-4-nitrobenzyl)-2*H*-benzimidazol-2-one.

A une solution de 0,25 g du composé obtenu à la Préparation 1 dans 5 ml de DMF, on ajoute par portions 0,033 g d'hydrure de sodium à 80 % dans l'huile et laisse 30 minutes sous agitation à TA. On refroidit au bain de glace, ajoute 0,25 g de 1-bromométhyl-3-méthoxy-4-nitrobenzène et laisse 72 heures sous agitation en laissant remonter la température à TA. On évapore sous vide le solvant, reprend le résidu à l'eau, extrait au DCM, lave à l'eau, sèche sur $Na_2SO_4$ et évapore sous vide. On chromatographie le résidu sur silice en éluant par le mélange DCM/AcOEt (95/5 ; v/v). On obtient 0,125 g du produit attendu après cristallisation dans l'EtOH, F = 176°C.

EXEMPLE 5

3-Cyclohexyl-5-éthoxy-1,3-dihydro-1-(2-méthoxy-4-nitrobenzyl)-2*H*-benzimidazol-2-one.

A une solution refroidie à 10°C, de 4 g du composé obtenu à la Préparation 3 dans 40 ml de DMF, on ajoute par portions 0,7 g d'hydrure de sodium à 60 % dans l'huile et laisse 1 heure 30 minutes sous agitation. On refroidit au bain de glace, ajoute, par portions en 20 minutes, 4 g de 1-bromométhyl-2-méthoxy-4-nitrobenzène et laisse 48 heures sous agitation en laissant remonter la température à TA. On verse le mélange réactionnel sur un mélange d'eau et de glace, extrait à l'AcOEt, lave à l'eau, sèche sur $Na_2SO_4$ et évapore sous vide. On obtient 4 g du produit attendu après cristallisation dans l'éther iso, F = 120°C.

EXEMPLE 6

1-(4-Amino-2-méthoxybenzyl)-3-cyclohexyl-5-éthoxy-1,3-dihydro-2*H*-benzimidazol-2-one.

On hydrogène à TA, pendant 20 heures, sous une pression de 60 bars un mélange de 3,5 g du composé obtenu

à l'EXEMPLE 5 dans 350 ml d'EtOH absolu, en présence de nickel de Raney. On filtre le mélange réactionnel sur Célite et évapore sous vide le filtrat. On chromatographie le résidu sur silice en éluant par le mélange DCM/AcOEt (90/10 ; v/v). On obtient 2,7 g du produit attendu après cristallisation dans le mélange éther iso/AcOEt, F = 191°C.

EXEMPLE 7

3-Cyclohexyl-5-éthoxy-1,3-dihydro-1-[2-méthoxy-4-(phénoxycarboxamido)benzyl]-2*H*-benzimidazol-2-one.

On refroidit au bain de glace un mélange de 1 g du composé obtenu à l'EXEMPLE 6 dans 40 ml de THF, puis ajoute une solution de 0,15 g de NaOH dans 2 ml d'eau et ajoute goutte à goutte 1,5 ml de chloroformiate de phényle. Après 30 minutes d'agitation, on évapore sous vide le solvant, reprend le résidu à l'eau, extrait au DCM, lave à l'eau, sèche sur $Na_2SO_4$ et évapore sous vide. On obtient 1,1 g du produit attendu après cristallisation dans l'éther iso, F = 189°C.

EXEMPLE 8

3-Cyclohexyl-5-éthoxy-1-[4-(N',N'-diéthyluréido)-2-méthoxybenzyl]-1,3-dihydro-2*H*-benzimidazol-2-one.

A une solution de 0,6 g du composé obtenu à l'EXEMPLE 7 dans 25 ml de DCM on ajoute 1 ml de diéthylamine et laisse 20 heures sous agitation à TA. On évapore sous vide le mélange réactionnel et chromatographie le résidu sur silice en éluant par le mélange DCM/AcOEt (85/15 ; v/v) puis (80/20 ; v/v). On obtient 0,415 g du produit attendu après cristallisation dans l'AcOEt, F = 158°C.

EXEMPLE 9

3-Cyclohexyl-5-éthoxy-1-[4-(N',N'-diméthyluréido)-2-méthoxybenzyl]-1,3-dihydro-2*H*-benzimidazol-2-one.

On prépare ce composé selon le mode opératoire décrit à l'EXEMPLE 8 à partir de 0,6 g du composé obtenu à l'EXEMPLE 7 et 30 ml d'une solution à 33 % de diméthylamine dans l'EtOH. On chromatographie sur silice en éluant par le mélange DCM/AcOEt (90/10 ; v/v). On obtient 0,43 g du produit attendu après cristallisation dans le mélange AcOEt/Ether iso, F = 250°C.

EXEMPLE 10

3-Cyclohexyl-5-éthoxy-1,3-dihydro-1-[4-[N'-méthyl-N'-(2-hydroxyéthyl)uréido]-2-méthoxybenzyl]-2*H*-benzimidazol-2-one.

On prépare ce composé selon le mode opératoire décrit à l'EXEMPLE 8 à partir de 0,6 g du composé obtenu à l'EXEMPLE 7 et 2 ml de 2-(méthylamino)éthanol. On chromatographie sur silice en éluant par le mélange DCM/AcOEt (90/10 ; v/v) puis par le mélange DCM/MeOH (90/10 ; v/v). On obtient 0,47 g du produit attendu après cristallisation dans l'éther iso, F = 157°C.

EXEMPLE 11

1-[4-(2-cyano-3-méthylguanidino)-2-méthoxybenzyl]-3-cyclohexyl-5-éthoxy-1,3-dihydro-2*H*-benzimidazol-2-one.

A) 1-[4-(3-cyano-2-méthyl-1-isothiouréido)-2-méthoxybenzyl]-3-cyclohexyl-5-éthoxy-1,3-dihydro-2H-benzimidazol-2-one, ($R'_6$ = -NH-C(SMe)=N-CN).

On chauffe à reflux pendant 24 heures un mélange de 1,0 g du composé obtenu à l'EXEMPLE 6, 0,4 g de diméthyl N-cyanodithioiminocarbonate et 25 ml de n-butanol, puis on concentre sous vide le mélange réactionnel. On chromatographie le résidu sur silice en éluant par le mélange DCM/AcOEt (95/5 ; v/v) puis (85/15 ; v/v). On obtient 0,15 g du produit attendu, F = 195°C.

B) 1-[4-(2-cyano-3-méthylguanidino)-2-méthoxybenzyl]-3-cyclohexyl-5-éthoxy-1,3-dihydro-2*H*-benzimidazol-2-one.

On laisse sous agitation à TA pendant 16 heures une solution de 0,15 g du composé obtenu à l'étape précédente dans 10 ml d'une solution à 33 % de méthylamine dans l'EtOH. On évapore sous vide le mélange réactionnel, et chromatographie le résidu sur silice en éluant par le mélange DCM/AcOEt (80/20 ; v/v). On obtient 0,1 g du produit attendu, F = 210°C.

EXEMPLE 12

5-Chloro-3-cyclohexyl-1-[4-(N',N'-diéthyluréido)-2-méthoxybenzyl]-1,3-dihydro-2*H*-benzimidazol-2-one.

A) 5-Chloro-3-cyclohexyl-1,3-dihydro-1-(2-méthoxy-4-nitrobenzyl)-2H-benzimidazol-2-one.

On prépare ce composé selon le mode opératoire décrit à l'EXEMPLE 5 à partir de 3 g du composé obtenu à la Préparation 1 et 3 g de 1-bromométhyl-2-méthoxy-4-nitrobenzène. On obtient 2 g du produit attendu qui est utilisé tel quel à l'étape suivante.

B) 1-(4-Amino-2-méthoxybenzyl)-5-chloro-3-cyclohexyl-1,3-dihydro-2*H*-benzimidazol-2-one.

On prépare ce composé selon le mode opératoire décrit à l'EXEMPLE 6 à partir de 2 g du composé obtenu à l'étape précédente. On obtient 1,5 g du produit attendu qui est utilisé tel quel à l'étape suivante.

C) 5-Chloro-3-cyclohexyl-1,3-dihydro-1-[2-méthoxy-4-(phénoxycarboxamido)benzyl]-2*H*-benzimidazol-2-one.

On prépare ce composé selon le mode opératoire décrit à l'EXEMPLE 7 à partir de 1,5 g du composé obtenu à l'étape précédente et 2 ml de chloroformiate de phényle. On obtient 1,3 g du produit attendu après cristallisation dans l'éther iso, F = 190°C.

D) 5-Chloro-3-cyclohexyl-1-[4-(N',N'-diéthyluréido)-2-méthoxybenzyl]-1,3-dihydro -2*H*-benzimidazol-2-one.

On prépare ce composé selon le mode opératoire décrit à l'EXEMPLE 8 à partir de 0,7 g du composé obtenu à l'étape précédente et 2 ml de diéthylamine. On chromatographie sur silice en éluant par le mélange DCM/AcOEt (90/10 ; v/v). On obtient 0,47 g du produit attendu après cristallisation dans l'éther iso, F = 205°C.

EXEMPLE 13

3-(2-Chlorophényl)-5-éthoxy-1-[4-(N',N'-diéthyluréido)-2-méthoxybenzyl]-1,3-dihydro-2*H*-benzimidazol-2-one.

A) 3-(2-Chlorophényl)-5-éthoxy-1,3-dihydro-1-(2-méthoxy-4-nitrobenzyl)-2H-benzimidazol-2-one.

On prépare ce composé selon le mode opératoire décrit à l'EXEMPLE 5 à partir de 0,74 g du composé obtenu à la Préparation 12 et 0,7 g de 1-bromométhyl-2-méthoxy-4-nitrobenzène. On obtient 0,5 g du produit attendu, F = 150°C.

B) 1-(4-Amino-2-méthoxybenzyl)-3-(2-chlorophényl)-5-éthoxy-1,3-dihydro-2*H*-benzimidazol-2-one.

On hydrogène à TA, sous 2 bars de pression, un mélange de 0,5 g du composé obtenu à l'étape précédente dans 50 ml d'EtOH, en présence de nickel de Raney. On filtre le catalyseur sur Célite et évapore sous vide le filtrat. On obtient 0,3 g du produit attendu que l'on utilise tel quel à l'étape suivante.

C) 3-(2-Chlorophényl)-5-éthoxy-1,3-dihydro-1-[2-méthoxy-4-(phénoxycarboxamido)benzyl]-2*H*-benzimidazol-2-one.

On prépare ce composé selon le mode opératoire décrit à l'EXEMPLE 7 à partir de 0,3 g du composé obtenu à l'étape précédente et 1 ml de chloroformiate de phényle. On obtient 0,25 g du produit attendu que l'on utilise tel quel à l'étape suivante.

D) 3-(2-Chlorophényl)-5-éthoxy-1-[4-(N',N'-diéthyluréido)-2-méthoxybenzyl]-1,3-dihydro-2*H*-benzimidazol-2-one.

On prépare ce composé selon le mode opératoire décrit à l'EXEMPLE 8 à partir de 0,25 g du composé obtenu à l'étape précédente et 1 ml de diéthylamine. On obtient 0,05 g du produit attendu après cristallisation dans l'éther iso, F = 181°C.

EXEMPLE 14

5-Ethoxy-1-[4-(N',N'-diéthyluréido)-2-méthoxybenzyl]-1,3-dihydro-3-(tétrahydropyran-4-yl)-2*H*-benzimidazol-2-one.

A) 5-Ethoxy-1,3-dihydro-3-(tétrahydropyran-4-yl)-1-(2-méthoxy-4-nitrobenzyl)-2*H*-benzimidazol-2-one.

On prépare ce composé selon le mode opératoire décrit à l'EXEMPLE 5 à partir de 1,2 g du composé obtenu à la Préparation 13 et 1,5 g de 1-bromométhyl-2-méthoxy-4-nitrobenzène. On obtient 1,2 g du produit attendu après cristallisation dans le mélange éther iso/EtOH (80/20 ; v/v), F = 175°C.

B) 1-(4-Amino-2-méthoxybenzyl)-5-éthoxy-1,3-dihydro-3-(tétrahydropyran-4-yl)-2H-benzimidazol-2-one.

On hydrogène à TA et à pression atmosphérique un mélange de 1,2 g du composé obtenu à l'étape précédente, 0,15 g de palladium sur charbon à 5 % dans 150 ml d'AcOEt. On filtre le catalyseur et évapore sous vide le filtrat. On obtient 1 g du produit attendu que l'on utilise tel quel à l'étape suivante.

C) 5-Ethoxy-1,3-dihydro-3-(tétrahydropyran-4-yl)-1-[2-méthoxy-4-(phénoxycarboxamido)benzyl]-2*H*-benzimidazol-2-one.

On prépare ce composé selon le mode opératoire décrit à l'EXEMPLE 7 à partir de 1,1 g du composé obtenu à l'étape précédente et 2 ml de chloroformiate de phényle. On obtient 1,05 g du produit attendu après cristallisation dans l'éther iso, F = 168°C.

D) 5-Ethoxy-1-[4-(N',N'-diéthyluréido)-2-méthoxybenzyl]-1,3-dihydro-3-(tétrahydropyran-4-yl)-2*H*-benzimidazol-2-one.

On prépare ce composé selon le mode opératoire décrit à l'EXEMPLE 8 à partir de 1,05 g du composé obtenu à l'étape précédente et 1 ml de diéthylamine. On chromatographie sur silice en éluant par le mélange DCM/MeOH (99/1 ; v/v). On obtient 0,49 g du produit attendu, F = 201°C.

EXEMPLE 15

1-[4-(N-tert-Butylcarbamoyl)-2-méthoxybenzyl]-3-cyclohexyl-5-éthoxy-1,3-dihydro-2*H*-benzimidazol-2-one.

A) 4-[[3-Cyclohexyl-5-éthoxy-2,3-dihydro-2-oxo-1*H*-benzimidazol-1-yl]méthyl]-3-méthoxybenzoate de méthyle.

A une solution de 0,53 g du composé obtenu à la Préparation 3 dans 20 ml de THF et 10 ml de DMF, on ajoute à TA, par portions, 0,09 g d'hydrure de sodium à 60 % dans l'huile et laisse 30 minutes sous agitation. Puis on ajoute 0,6 g de 4-bromométhyl-3-méthoxybenzoate de méthyle et laisse 1 heure sous agitation à TA. On évapore sous vide les solvants, extrait le résidu à l'AcOEt, lave à l'eau, sèche sur $Na_2SO_4$ et évapore sous vide le solvant. On chromatographie le résidu sur silice en éluant au DCM. On obtient 0,49 g du produit attendu que l'on utilise tel quel à l'étape suivante.

B) Acide 4-[[3-cyclohexyl-5-éthoxy-2,3-dihydro-2-oxo-1*H*-benzimidazol-1-yl] méthyl]-3-méthoxybenzoïque.

A une solution de 0,49 g du composé obtenu à l'étape précédente dans 20 ml de THF, on ajoute une solution de 0,3 g de NaOH dans 30 ml d'eau et laisse 3 heures sous agitation à TA. On extrait le mélange réactionnel à l'AcOEt, acidifie la phase aqueuse à pH = 1 par ajout d'HCl 1N, extrait à l'AcOEt, sèche sur $Na_2SO_4$ et évapore sous vide le solvant. On obtient 0,45 g du produit attendu qui est utilisé tel quel à l'étape suivante.

C) 1-[4-(N-tert-Butylcarbamoyl)-2-méthoxybenzyl]-3-cyclohexyl-5-éthoxy-1,3-dihydro-2*H*-benzimidazol-2-one.

A une solution de 0,45 g du composé obtenu à l'étape précédente, 0,52 g de BOP, 1 ml de DIPEA dans 30 ml de DCM, on ajoute 0,5 g de tert-butylamine et laisse 2 heures sous agitation à TA. On lave le mélange réactionnel par une solution 1N d'HCl, à l'eau, par une solution 1N de NaOH, sèche sur $Na_2SO_4$ et évapore sous vide le solvant. On chromatographie le résidu sur silice en éluant par le mélange DCM/AcOEt (85/15 ; v/v). On obtient 0,13 g du produit attendu, F = 186°C.

EXEMPLE 16

3-Cyclohexyl-5-éthoxy-1,3-dihydro-1-[2-méthoxy-4-[N-(1,1-diméthylpropyl) carbamoyl]benzyl]-2*H*-benzimidazol-2-one.

A une solution de 0,9 g du composé obtenu à l'EXEMPLE 15 étape B, 0,9 g de BOP, 1 ml de DIPEA, dans 20 ml de DCM, on ajoute 1 g de tert-amylamine et laisse 3 heures sous agitation à TA. On lave le mélange réactionnel par une solution 1N d'HCl, par une solution 1N de NaOH, sèche sur $Na_2SO_4$ et évapore sous vide le solvant. On chromatographie le résidu sur silice en éluant au DCM. On obtient 0,34 g du produit attendu, F = 164°C.

EXEMPLE 17

1-[4-(N-tert-Butylcarbamoyl)-2-méthoxybenzyl]-5-éthoxy-1,3-dihydro-3-(tétrahydropyran-4-yl)-2*H*-benzimidazol-2-one.

A) 4-[[5-Ethoxy-2,3-dihydro-3-(tétrahydropyran-4-yl)-2-oxo-1*H*-benzimidazol-1-yl]méthyl]-3-méthoxybenzoate de méthyle.

On prépare ce composé selon le mode opératoire décrit à l'EXEMPLE 15 étape A à partir de 0,6 g du composé obtenu à la Préparation 13 et 0,7 g de 4-bromométhyl-3-méthoxybenzoate de méthyle. On obtient 0,8 g du produit attendu.

B) Acide 4-[[5-éthoxy-2,3-dihydro-3-(tétrahydropyran-4-yl)-2-oxo-1*H*-benzimidazol-1-yl]méthyl]-3-méthoxybenzoïque.

A une solution de 0,8 g du composé obtenu à l'étape précédente dans 20 ml de THF, on ajoute une solution de 0,4 g de NaOH dans 50 ml d'eau et laisse 5 heures sous agitation à TA. On ajoute 150 ml d'eau, extrait à l'AcOEt, acidifie la phase aqueuse à pH = 1 par ajout d'HCl 1N, extrait à l'AcOEt, sèche sur $Na_2SO_4$ et évapore sous vide le solvant. On obtient 0,7 g du produit attendu après cristallisation dans le mélange éther iso/EtOH (80/20 ; v/v), F = 209°C.

C) 1-[4-(N-tert-Butylcarbamoyl)-2-méthoxybenzyl]-5-éthoxy-1,3-dihydro-3-(tétrahydropyran-4-yl)-2*H*-benzimidazol-2-one.

A une solution de 0,7 g du composé obtenu à l'étape précédente, 0,9 g de BOP, 1 ml de DIPEA dans 20 ml de DCM, on ajoute 1,2 ml de tert-butylamine et laisse 3 heures sous agitation à TA. On évapore sous vide le solvant, extrait le résidu à l'AcOEt, lave par une solution d'HCl 1N, par une solution de NaOH 1N, sèche sur $Na_2SO_4$ et évapore sous vide. On chromatographie le résidu sur silice en éluant par le mélange DCM/MeOH (99/1 ; v/v). On obtient 0,26 g du produit attendu après cristallisation dans l'AcOEt, F = 211°C.

En procédant selon les modes opératoires décrits dans les EXEMPLES ci-dessus, à partir des benzimidazol-2-ones décrites dans les Préparations ci-dessus, on prépare les composés selon l'invention rassemblés dans le TABLEAU I ci-après.

TABLEAU I

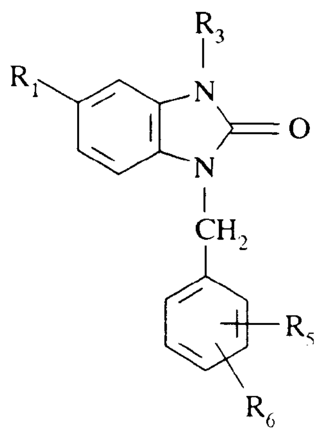

(I)

| EXEMPLES | $R_1$ | $R_3$ | $R_5$ | $R_6$ | F°C ou RMN solvant de cristallisation |
|---|---|---|---|---|---|
| *18 (1) | -OMe | 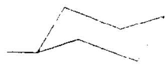 | 3-OMe | 4-OMe | RMN |
| *19 (2) | -Cl | 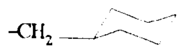 | 2-OMe | 4-OMe | 80 cyclohexane/ éther iso |
| *20 (3) | -OEt | 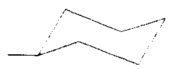 | 2-OMe | 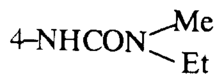 | 193 éther iso |
| *21 (3) | -OEt | 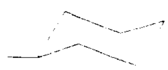 | 2-OMe | 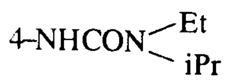 | 160 éther iso |
| *22 (4) | -OMe | 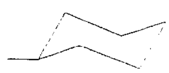 | 2-OMe | 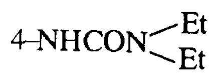 | 149 éther iso |
| *23 (5) | -OEt | 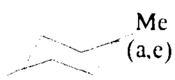 | 2-OMe | 4-$NO_2$ | 148 éther iso |
| *24 (6) | -OEt | 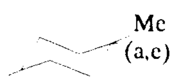 | 2-OMe | 4-$NH_2$ | 155 éther iso |
| *25 (7) | -OEt | 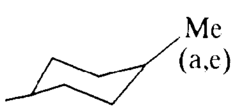 | 2-OMe | 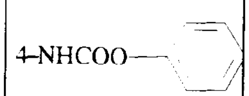 | 187 éther iso |

TABLEAU I (suite)

| EXEMPLES | $R_1$ | $R_3$ | $R_5$ | $R_6$ | F°C ou RMN solvant de cristallisation |
|---|---|---|---|---|---|
| *26 (8) | -OEt | 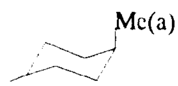 | 2-OMe | 4-$NH_2$ | 112 |
| *27 (7) | -OEt | 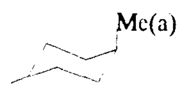 | 2-OMe | 4-NHCOO— 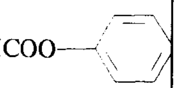 | 166 |
| *28 (5) | -OEt | 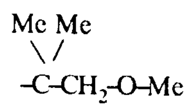 | 2-OMe | 4-$NO_2$ | 132 éther iso/ pentane |
| *29 (9) | -OEt | 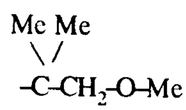 | 2-OMe | 4-$NH_2$ | 115 |
| *30 (7) | -OEt | 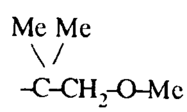 | 2-OMe | 4-NHCOO— 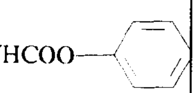 | 173 |
| *31 (10) | -OEt | 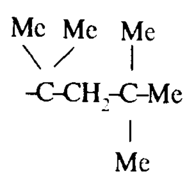 | 2-OMe | 4-NHCOO— 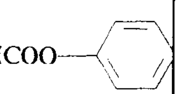 | 173 éther iso |
| *32 (8) | -O 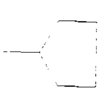 | 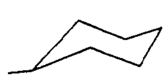 | 2-OMe | 4-$NH_2$ | 95 |

TABLEAU I (suite)

| EXEMPLES | $R_1$ | $R_3$ | $R_5$ | $R_6$ | F°C ou RMN solvant de cristallisation |
|---|---|---|---|---|---|
| *33 (7) | 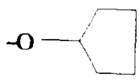 | 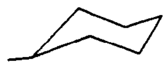 | 2-OMe | 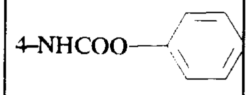 | 166 éther iso |
| *34 (8) | -O-$CH_2CH_2$-O-Me | 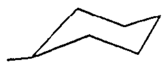 | 2-OMe | 4-$NH_2$ | 163 |
| *35 (3) | -OEt | 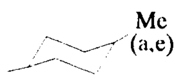 | 2-OMe | 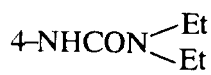 | 204 éther iso |
| *36 (3) | -OEt | 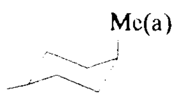 | 2-OMe | 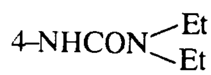 | 154 éther iso |
| *37 (4) | -OEt | 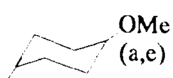 | 2-OMe | 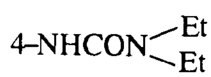 | 151-153 éther iso |
| *38 (4) | -OEt | 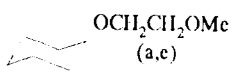 | 2-OMe | 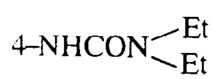 | 153 éther iso |
| *39 (3) | -OEt | 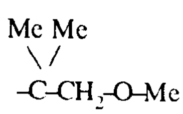 | 2-OMe | 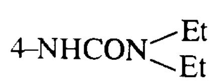 | 183 |

TABLEAU I (suite)

| EXEMPLES | $R_1$ | $R_3$ | $R_5$ | $R_6$ | F°C ou RMN solvant de cristallisation |
|---|---|---|---|---|---|
| *40 (3) | -OEt | 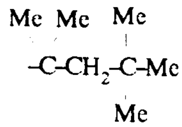 | 2-OMe | 4-NHCON Et Et | 185 |
| *41 (3) | 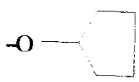 | 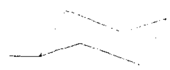 | 2-OMe | 4-NHCON Et Et | 166 éther iso |
| *42 (11) | -O-$CH_2CH_2$-O-Me | 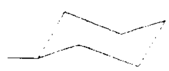 | 2-OMe | 4-NHCON Et Et | 138 éther iso |
| *43 (4) | - OEt | 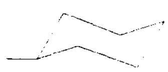 | 2-OMe | 5-NHCON Et Et | 187 AcOEt |
| *44 (4) | -OEt | 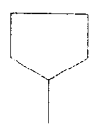 | 2-OMe | 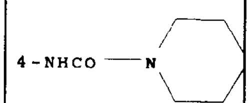 | 195 AcOEt |
| 45 (3) | -OEt | 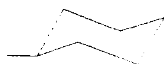 | 2-OMe | 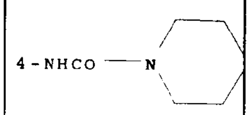 | 210 AcOEt |

(1) Composé préparé selon le mode opératoire décrit à l'EXEMPLE 1.

(2) Composé préparé selon le mode opératoire décrit à l'EXEMPLE 2.

(3) Composé préparé selon le mode opératoire décrit à l'EXEMPLE 8 en utilisant les amines appropriées.

(4) Composé préparé selon les modes opératoires décrits aux EXEMPLES 5, 6, 7 puis 8.

(5) Composé préparé selon le mode opératoire décrit à l'EXEMPLE 5.

(6) Composé préparé selon le mode opératoire décrit à l'EXEMPLE 6.

(7) Composé préparé selon le mode opératoire décrit à l'EXEMPLE 7.

(8) Composé préparé selon les modes opératoires décrits aux EXEMPLES 5 et 6.

(9) Composé préparé selon le mode opératoire suivant : on hydrogène à TA et à pression atmosphérique un mélange du composé nitré ($R_6$ = 4–$NO_2$) correspondant, en solution dans l'AcOEt, et en présence de palladium sur charbon à 5 %. On filtre le catalyseur sur Célite et évapore sous vide le filtrat.

(10) Composé préparé selon les modes opératoires décrits aux EXEMPLES 5, 6 puis 7.

(11) Composé préparé selon les modes opératoires décrits aux EXEMPLES 7 puis 8.

(a,e) : mélange d'isomères axial–équatorial.

(a) : isomère axial.

(e) : isomère équatorial.

Spectre de RMN à 200 MHz dans le DMSO du composé de l'EXEMPLE 18.

| | | |
|---|---|---|
| 1,8 à 2,3 ppm | : mt | : 10 H |
| 3,8 ppm | : 3 x s | : 9 H |
| 4,2 ppm | : mt | : 1 H |
| 4,95 ppm | : s | : 2 H |
| 6,6 à 7,1 ppm | : mt | : 6 H |

EXEMPLE 46

3-Cyclopentyl-5-éthoxy-1-[4-(N',N'-diéthyluréido)-2-méthoxybenzyl]-1,3-dihydro-2*H*-benzimidazol-2-one.

A) 3-Cyclopentyl-5-éthoxy-1,3-dihydro-1-(2-méthoxy-4-nitrobenzyl)-2*H*-benzimidazol-2-one.

On prépare ce composé selon le mode opératoire décrit à l'EXEMPLE 5 à partir de 1 g du composé obtenu à la Préparation 32 et 1,5 g de 1-bromométhyl-2-méthoxy-4-nitrobenzène. On obtient 1 g du produit attendu après cristallisation dans l'éther iso, F = 114°C.

B) 1-(4-Amino-2-méthoxybenzyl)-3-cyclopentyl-5-éthoxy-1,3-dihydro-2*H*-benzimidazol-2-one.

On hydrogène à TA, sous une pression de 2 bars, pendant 30 minutes un mélange de 1 g du composé obtenu à l'étape précédente dans 100 ml d'EtOH 95 et en présence de nickel de Raney®. On filtre le catalyseur sur Célite® et évapore sous vide le filtrat. On obtient 0,7 g du produit attendu, F = 164°C.

C) 3-Cyclopentyl-5-éthoxy-1,3-dihydro-1-[2-méthoxy-4-(phénoxycarboxamido)benzyl]-2*H*-benzimidazol-2-one.

On prépare ce composé selon le mode opératoire décrit à l'EXEMPLE 7 à partir de 0,7 g du composé obtenu à l'étape précédente et 1 ml de chloroformiate de phényle. On obtient 0,65 g du produit attendu, F = 140°C.

D) 3-Cyclopentyl-5-éthoxy-1-[4-(N',N'-diéthyluréido)-2-méthoxybenzyl]-1,3-dihydro-2*H*-benzimidazol-2-one.

On prépare ce composé selon le mode opératoire décrit à l'EXEMPLE 8 à partir de 0,33 g du composé obtenu à l'étape précédente et 2 ml de diéthylamine. On obtient 0,25 g du produit attendu après cristallisation dans le mélange AcOEt/éther iso (75/25; v/v), F = 158°C.

EXEMPLE 47

3-Cyclohexyl-5-éthoxy-1,3-dihydro-1-[2-méthoxy-4-[N-(2-diméthylamino-1,1-diméthyléthyl)carbamoyl]benzyl] -2*H*-benzimidazol-2-one.

On laisse 1 heure sous agitation à TA un mélange de 1,5 g du composé obtenu à l'EXEMPLE 15 étape B, 1 g de 2-diméthylamino-1,1-diméthyléthylamine (synthétisée selon J. Am. Chem. Soc., 1946, 68, 12-14), 1,6 g de BOP, 2 g de DIPEA et 20 ml de DCM. On concentre sous vide le mélange réactionnel, extrait le résidu à l'AcOEt, lave la phase organique par une solution de NaOH 1N, à l'eau, sèche sur $Na_2SO_4$ et évapore sous vide le solvant. On chromatographie le résidu sur silice en éluant par le mélange DCM/MeOH (98/2 ; v/v). On obtient 0,15 g du produit attendu après cristallisation dans l'éther iso, F = 161°C.

EXEMPLE 48

3-Cyclohexyl-5-éthoxy-1,3-dihydro-1-[4-[N-(1,1-diméthylpropyl)sulfamoyl] benzyl]-2*H*-benzimidazol-2-one.

A une solution de 0,5 g du composé obtenu à la Préparation 3 dans 10 ml de DMF, on ajoute à TA, par portions, 0,1 g d'hydrure de sodium à 60 % dans l'huile et laisse 30 minutes sous agitation. On refroidit au bain de glace, ajoute 0,6 g de N-(1,1-diméthylpropyl)-4-bromométhylbenzènesulfonamide et laisse trois heures sous agitation en laissant remonter la température à TA. On verse le mélange réactionnel dans un mélange eau/glace, extrait à l'AcOEt, lave la phase organique à l'eau, sèche sur $Na_2SO_4$ et évapore sous vide le solvant. On chromatographie le résidu sur silice en éluant par le mélange DCM/AcOEt (98,5/1,5 ; v/v) puis (97/3 ; v/v). On obtient 0,5 g du produit attendu après cristallisation dans l'éther iso, F = 165°C.

EXEMPLE 49

Chlorhydrate de 3-cyclohexyl-5-éthoxy-1,3-dihydro-1-[4-[6-(diméthylamino)hexyloxy]benzyl]-2*H*-benzimidazol-2-one.

A) 1-[4-(6-Bromohexyloxy)benzyl]-3-cyclohexyl-5-éthoxy-1,3-dihydro-2*H*-benzimidazol-2-one.

A une solution de 0,5 g du composé obtenu à la Préparation 3 dans 20 ml de THF, on ajoute à TA, par portions, 0,06 g d'hydrure de sodium à 80 % dans l'huile et laisse 30 minutes sous agitation. Puis on ajoute 1 g de bromure de 4-(6-bromohexyloxy)benzyle et laisse 20 heures sous agitation à TA. On concentre sous vide le mélange réactionnel, extrait le résidu à l'AcOEt, lave la phase organique à l'eau, sèche sur $Na_2SO_4$ et évapore sous vide le solvant. On chromatographie le résidu sur silice en éluant à l'hexane puis à l'éther iso. On obtient 1 g du produit attendu que l'on utilise tel quel.

B) Chlorhydrate de 3-cyclohexyl-5-éthoxy-1,3-dihydro-1-[4-[6-(diméthylamino) hexyloxy]benzyl]-2*H*-benzimidazol-2-one.

On laisse 20 heures sous agitation à TA un mélange de 0,5 g du composé obtenu à l'étape précédente, 10 ml d'une solution à 33 % de diméthylamine dans le MeOH et 20 ml de THF. On concentre sous vide le mélange réactionnel, reprend le résidu à l'AcOEt, extrait la phase organique par une solution d'HCl1N, alcalinise la phase aqueuse acide par ajouts de KOH en pastilles, extrait à l'AcOEt, lave la phase organique à l'eau, sèche sur $Na_2SO_4$ et évapore sous vide le solvant. On dissout le résidu dans du pentane, acidifie à pH = 1 par barbotage d'HCl gaz et concentre sous vide. On obtient 0,15 g du produit attendu après cristallisation dans le mélange AcOEt/éther, F = 90°C (déc.).

EXEMPLE 50

3-Cyclohexyl-5-éthoxy-1,3-dihydro-1-[2-méthoxy-4-(pyrrol-1-yl)benzyl]-2*H*-benzimidazol-2-one.

A) 3-Cyclohexyl-5-éthoxy-1,3-dihydro-1-[2-méthoxy-4-(Δ3-pyrrolin-1-yl)benzyl]-2*H*-benzimidazol-2-one.

On chauffe à reflux pendant 1 heure 30 minutes, sous atmosphère d'azote, un mélange de 1 g du composé obtenu à l'EXEMPLE 6, 1 g de cis-1,4-dichlorobut-2-ène, 1 ml de triéthylamine dans 20 ml de DMF. Après refroidissement on verse le mélange réactionnel dans un mélange eau/glace, extrait à l'AcOEt, lave la phase organique à l'eau, sèche sur $Na_2SO_4$ et évapore sous vide le solvant. On chromatographie le résidu sur silice en éluant au DCM. On obtient 0,5 g du produit attendu.

B) 3-Cyclohexyl-5-éthoxy-1,3-dihydro-1-[2-méthoxy-4-(pyrrol-1-yl)benzyl]-2*H*-benzimidazol-2-one.

On refroidit à 5-10°C une solution de 0,7 g de chlorure de benzoyle dans 2 ml de THF, ajoute goutte à goutte, 3 ml d'une solution à 30 % de peroxyde d'hydrogène dans l'eau et laisse 30 minutes sous agitation. On reprend le mélange réactionnel dans du chloroforme, lave la phase organique à l'eau et sèche sur $Na_2SO_4$. A la solution obtenue on ajoute une solution de 0,5 g du composé obtenu à l'étape précédente dans 10 ml de chloroforme et laisse une nuit sous agitation à TA et à l'obscurité. On lave le mélange réactionnel par une solution de NaOH 1N, à l'eau, sèche la phase organique sur $Na_2SO_4$ et évapore sous vide le solvant. On obtient 0,3 g du produit attendu après cristallisation dans l'éther iso, F = 115°C.

**Revendications**

1. Un composé de formule :

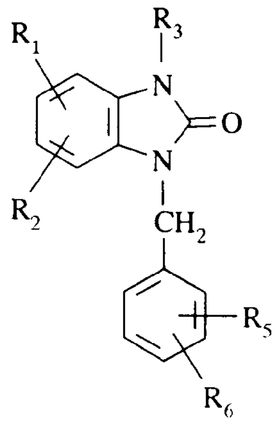

(I)

dans laquelle :

- $R_1$ représente un halogène ; un $(C_1-C_7)$alkyle ; un $(C_1-C_7)$alkylthio ; un phénylthio ; un trifluorométhyle ; un cyano ; un nitro ; un groupe $-NR_7R_8$ ; un hydroxy ; un $(C_1-C_7)$alcoxy ; un $(C_3-C_7)$cycloalkyloxy ; un $(C_3-C_7)$cycloalkylméthoxy ; un phénoxy ; un benzyloxy ; un ω-halogéno$(C_1-C_7)$alkyloxy ; un polyhalogéno$(C_1-C_7)$alkyloxy ; un ω-hydroxy$(C_2-C_7)$alkyloxy ; un ω-méthoxy$(C_2-C_7)$alkyloxy ;

- $R_2$ représente un hydrogène, un halogène, un $(C_1-C_7)$alkyle ;

- $R_3$ représente $R_4$ ; un groupe-$(CH_2)_p$-$R_4$ ; un indanyle ; un hexahydroindanyle ; un adamantyle ; un noradamantyle ; un norbornyle ; un $(C_1-C_8)$alkyle non substitué ou substitué par un $(C_1-C_4)$alcoxy ; un cyclohexyle substitué par un di$(C_1-C_7)$alkylamino, un carboxy, un $(C_1-C_4)$alcoxycarbonyle, un hydroxy, un tetrahydropyran-2-yloxy, un $(C_1-C_4)$alcoxy$(C_1-C_4)$alcoxy ou un phényl$(C_1-C_2)$alcoxy$(C_1-C_4)$alcoxy ;

- $R_4$ représente un groupe $-NR_9R_{10}$ ; un $(C_3-C_7)$cycloalkyle non substitué ou substitué une ou deux fois par un $(C_1-C_4)$alkyle ou un $(C_1-C_4)$alcoxy ; un furyle; un thiényle ; un pyrrolyle ; un triazolyle ; un tétrazolyle ; un pyridyle ; un pyridyle N-oxyde ; un pyrimidinyle ; un pyrazolyle ; un pyrazinyle ; un tétrahydropyran-4-yle ; un azétidin-3-yle substitué en position 1 par $R_{11}$ ; un pipérid-4-yle substitué en position 1 par $R_{11}$ ; un groupe Ar ;

- $R_5$ représente un hydrogène ; un $(C_1-C_7)$alkyle ; un $(C_1-C_7)$alcoxy ; un halogène ; un hydroxy ; un trifluorométhyle ;

- $R_6$ représente un cyano ; un groupe $-CH_2NR_7R_8$ ; un nitro ; un groupe $-NR_{12}R_{13}$ ; un groupe -NHOH ; un guanidino non substitué ou substitué en position 1 par un $(C_1-C_7)$alkyle et/ou en position 3 par un ou deux $(C_1-C_7)$ alkyles, un groupe Ar ou un groupe $-CH_2$-Ar et/ou en position 2 par un cyano ; un groupe $-OR_{14}$ ; un groupe $-SR_{14}$ ; un $(C_1-C_7)$alkylcarbonyle ; un groupe $-CONR_{15}R_{16}$ ; un thiocarbamoyle libre ou substitué par un ou deux $(C_1-C_7)$alkyles ; un carboxy; un $(C_1-C_7)$alcoxycarbonyle ; un groupe -COO-Ar ; un groupe $-COO-CH_2$-Ar ; un groupe $-CO-NH-CR_{17}R_{18}-COR_{19}$ ; un groupe $-SO_2NR_{20}R_{21}$ ; un groupe $-NHSO_2-(C_1-C_7)$alkyle ; un groupe $-NHSO_2$-Ar ; un groupe $-NHSO_2-CH_2$-Ar ; un diméthylaminosulfonamido ;

- $R_7$ et $R_8$ représentent chacun indépendamment un hydrogène ou un $(C_1-C_7)$alkyle ; $R_8$ peut de plus représenter un groupe protecteur ;

- $R_9$ et $R_{10}$ représentent chacun indépendamment un hydrogène ou un $(C_1-C_7)$alkyle ;

- ou bien $R_9$ et $R_{10}$ ensemble avec l'atome d'azote auquel ils sont liés constituent un hétérocycle choisi parmi la morpholine, la thiomorpholine, l'azétidine, la pyrrolidine, la pipéridine, la pipérazine substituée en position 4 par $R_{11}$ ou la perhydroazépine ;

- $R_{11}$ représente un hydrogène ; un $(C_1-C_7)$alkyle ; un phényle ; un benzyle ; un $(C_1-C_7)$alkylcarbonyle ; un benzoyle ; un $(C_1-C_7)$alcoxycarbonyle ; un phénoxycarbonyle ; un carbamoyle non substitué ou substitué par un ou deux $(C_1-C_7)$alkyles ;

- $R_{12}$ et $R_{13}$ représentent chacun indépendamment un hydrogène ; un $(C_1-C_7)$alkyle ; un groupe $-CH_2$-Ar ; $R_{13}$

peut de plus représenter un $(C_3-C_7)$cycloalkylméthyle ; un groupe Ar ; un groupe $-CH_2CH_2Ar$ ; un $(C_3-C_8)$ alcényle ; un $(C_1-C_7)$alkylcarbonyle ; un $(C_1-C_7)$alkylthiocarbonyle ; un $(C_3-C_7)$cycloalkylcarbonyle ; un $(C_3-C_7)$ cycloalkylthiocarbonyle ; un groupe -CO-Ar ; un groupe $-CO-CH_2-Ar$ ; un ω-$R_7R_8N(C_2-C_7)$alkylcarbonyle ; un ω-hydroxy$(C_1-C_7)$alkylcarbonyle ; un ω-benzyloxy$(C_1-C_7)$alkylcarbonyle ; un pyridylcarbonyle ; un méthylpyridylcarbonyle ; un thiénylcarbonyle ; un furylcarbonyle ; un pipérid-4-ylcarbonyle substitué en position 1 par $R_{11}$ ; un $(C_1-C_7)$alcoxycarbonyle ; un phénoxycarbonyle ; un phénoxythiocarbonyle ; un benzyloxycarbonyle ; un groupe $-CONR_{22}R_{23}$ ; un groupe $-CSNR_{22}R_{23}$ ; un groupe $-CO-CR_{17}R_{18}-NR_7R_8$ ; un groupe $-CR_{17}R_{18}COR_{19}$ ; un groupe $-(CH_2)_t-COR_{19}$ ; un groupe $-CO-(CH_2)_u-COR_{19}$ ;

- ou bien $R_{12}$ et $R_{13}$ ensemble avec l'atome d'azote auquel ils sont liés constituent l'hydantoïne, la N-méthylhydantoïne, ou un radical hétérocyclique choisi parmi le pyrrol-1-yle, le Δ3-pyrrolin-1-yle, la pyrrolidin-1-yle, l'isoindolin-2-yle dans lequel le noyau benzénique est non substitué ou substitué par un halogène, un $(C_1-C_7)$alkyle, un trifluorométhyle ou un $(C_1-C_7)$alcoxy ;

- $R_{14}$ représente l'hydrogène ; un $(C_1-C_7)$alkyle ; un phényle ; un benzyle ; un $(C_3-C_7)$cycloalkyle ; un $(C_3-C_7)$ alcényle ; un ω-halogéno$(C_2-C_7)$alkyle ; un polyhalogéno$(C_1-C_7)$alkyle ; un ω-hydroxy$(C_2-C_7)$alkyle ; un $(C_1-C_7)$alkylcarbonyle ; un benzoyle ; un ω-carboxy$(C_1-C_7)$alkyle ; un ω-$(C_1-C_4)$alcoxycarbonyl$(C_1-C_7)$alkyle ; un ω-benzyloxycarbonyl$(C_1-C_7)$alkyle ; un ω-$R_7R_8N(C_2-C_7)$alkyle ; un ω-carbamoyl$(C_1-C_7)$alkyle dans lequel le carbamoyle est libre ou substitué par un ou deux $(C_1-C_7)$alkyles ;

- $R_{15}$ et $R_{16}$ représentent chacun indépendamment un hydrogène ou un $(C_1-C_7)$alkyle ; $R_{16}$ peut de plus représenter un $(C_3-C_7)$cycloalkyle non substitué ou substitué par un $(C_1-C_4)$alkyle ; un groupe Ar ; un pyridyle ; un méthylpyridyle ; un pipérid-4-yle substitué en position 1 par $R_{11}$ ; un méthylpipérid-4-yle ; un pyrrolidin-1-yle ; un pipérid-1-yle ; un morpholin-4-yle ; un $(C_1-C_7)$alkyle substitué par un ou plusieurs halogènes ou par $R_{24}$ ;

- ou bien $R_{15}$ et $R_{16}$ ensemble avec l'atome d'azote auquel ils sont liés représentent un radical hétérocyclique $R_{25}$ ;

- $R_{17}$ et $R_{18}$ représentent chacun indépendamment l'hydrogène ; un $(C_1-C_7)$alkyle ; un benzyle ;

- ou bien $R_{17}$ et $R_{18}$ ensemble avec l'atome de carbone auquel ils sont liés constituent un $(C_3-C_7)$cycloalkyle ;

- $R_{19}$ représente un hydroxy ; un $(C_1-C_7)$alcoxy ; un amino libre ou substitué par un ou deux $(C_1-C_7)$alkyles ;

- $R_{20}$ et $R_{21}$ représentent chacun indépendamment l'hydrogène ; un $(C_1-C_7)$alkyle ; $R_{21}$ peut de plus représenter un $(C_3-C_7)$cycloalkyle ;

- ou bien $R_{20}$ et $R_{21}$ ensemble avec l'atome d'azote auquel ils sont liés constituent un radical hétérocyclique $R_{25}$ ;

- $R_{22}$ et $R_{23}$ représentent chacun indépendamment l'hydrogène ; un $(C_1-C_7)$alkyle ; $R_{23}$ peut de plus représenter un $(C_3-C_7)$cycloalkyle ; un adamantyle ; un groupe Ar ; un hydroxy ; un $(C_1-C_4)$alcoxy ; un $(C_1-C_7)$alkyle substitué par un groupe Ar, un pyridyle, un hydroxy, un $(C_1-C_7)$alcoxy, un groupe-$NR_7R_8$, un carboxy ou un $(C_1-C_7)$ alcoxycarbonyle ;

- ou bien $R_{22}$ et $R_{23}$ ensemble avec l'atome d'azote auquel ils sont liés constituent un radical hétérocyclique $R_{25}$ ;

- $R_{24}$ représente un hydroxy ; un $(C_1-C_7)$alcoxy ; un cyano ; un carboxy ; un $(C_1-C_7)$alcoxycarbonyle ; un groupe $-NR_7R_8$ ; un carbamoyle libre ou substitué par un ou deux $(C_1-C_7)$alkyles ; un benzyloxycarbonyle ; un groupe Ar ; un $(C_3-C_7)$cycloalkyle ; un adamantyle ; un pyrrolidin-1-ylcarbonyle ; un pipérid-1-ylcarbonyle ; un perhydroazépin-1-ylcarbonyle ; un radical hétérocyclique choisi parmi un pyridyle, un méthylpyridyle, un furanyle, un tétrahydrofuranyle, un thiényle, un méthylthiényle, un pyrrolin-1-yle, un pipérid-1-yle, un perhydroazépin-1-yle ;

- $R_{25}$ représente un morpholin-4-yle ; un thiomorpholine-4-yle ; un azétidin-1-yle non substitué ou substitué en position 2 par un carboxy, un $(C_1-C_4)$alcoxycarbonyle ou en position 3 par un groupe $-NR_7R_8$, un $(C_1-C_7)$alkyle, un phényle, un benzyle ou un $(C_1-C_7)$alkylcarbonyle ; un perhydroazépin-1-yle ; un pipérazin-1-yle non substitué ou substitué en position 4 par un $(C_1-C_7)$alkyle, un phényle, un benzyle, un $(C_1-C_7)$alkylcarbonyle, un $(C_1-C_7)$alcoxycarbonyle ou un benzyloxycarbonyle ; un pipérid-1-yle non substitué ou substitué en position 4

par un $(C_1-C_7)$alkyle, un phényle, un benzyle, un $(C_1-C_7)$alkylcarbonyle, un groupe $-NR_7R_8$ ; un cis-2,6-diméthylpipérid-1-yle ; un pyrrolidin-1-yle non substitué ou substitué par un $(C_1-C_7)$alkyle, un phényle, un benzyle, un $(C_1-C_7)$alkylcarbonyle, un hydroxyméthyle, un carboxy, un $(C_1-C_7)$alcoxycarbonyle, un carbamoyle non substitué ou substitué par un ou deux $(C_1-C_7)$alkyles ;

- Ar représente un phényle non substitué ou substitué une ou plusieurs fois par un substituant choisi parmi : un atome d'halogène, un $(C_1-C_7)$alkyle, un trifluorométhyle, un hydroxy, un $(C_1-C_7)$alcoxy, un carboxy, un $(C_1-C_7)$alcoxycarbonyle, un $(C_1-C_7)$alkylcarbonyloxy, un nitro, un cyano, un amino, un $(C_1-C_7)$alkylamino, un di$(C_1-C_7)$alkylamino, lesdits substituants étant identiques ou différents ;
- t représente un nombre entier qui peut varier de 2 à 5 ;
- u représente un nombre entier qui peut varier de 0 à 7 ;
- p représente un nombre entier qui peut varier de 1 à 8

ainsi que ses sels,
à la condition que lorsque $R_6$ représente un méthoxy, $R_5$ est autre que l'hydrogène.

**2.** Un composé selon la revendication 1 de formule :

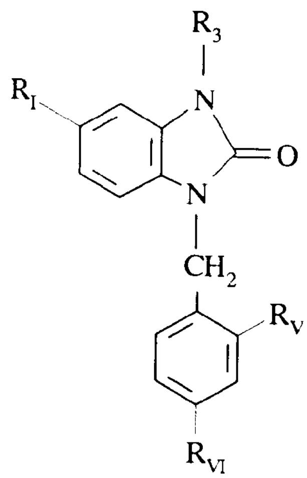

(Ia)

dans laquelle :

- $R_I$ représente un $(C_1-C_4)$alcoxy, un atome de chlore ou de fluor,
- $R_V$ représente l'hydrogène ou un méthoxy,
- $R_{VI}$ représente un $(C_1-C_7)$alkylcarboxamido, un groupe -NHCO-Ar, un groupe $-CONR_{15}R_{16}$, un groupe $-NR_{12}CONR_{22}R_{23}$ ;

et les substituants $R_3$, Ar, $R_{12}$, $R_{15}$, $R_{16}$, $R_{22}$, $R_{23}$ sont tels que définis dans la revendication 1 pour les composés de formule (I) ;
ainsi que ses sels.

**3.** Un composé selon la revendication 2 de formule (Ia) dans laquelle $R_3$ représente un cyclohexyle ou un groupe Ar.

**4.** Un procédé pour la préparation d'un composé selon la revendication 1 ou de l'un de ses sels, caractérisé en ce que :

1) on fait agir sur un composé de formule :

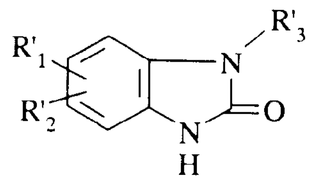

(II)

dans laquelle $R'_1$, $R'_2$ et $R'_3$ représentent, respectivement, soit $R_1$, $R_2$ et $R_3$ tels que définis pour (I) dans la revendication 1, soit des groupes précurseurs de $R_1$, $R_2$ et $R_3$, un halogénure de benzyle de formule :

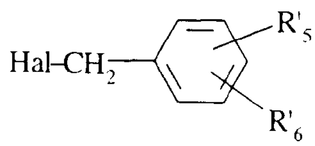

(III)

dans laquelle Hal représente un atome d'halogène, et $R'_5$ et $R'_6$ représentent, respectivement, soit $R_5$ et $R_6$ tels que définis pour (I) dans la revendication 1, soit des groupes précurseurs de $R_5$ et $R_6$ ; et,

2) soit, lorsque $R'_1 = R_1$, $R'_2 = R_2$, $R'_3 = R_3$, $R'_5 = R_5$ et $R'_6 = R_6$, on isole le composé de formule (I) ainsi obtenu ;

3) soit, lorsque l'un quelconque des groupes $R'_1$, $R'_2$, $R'_3$, $R'_5$ et/ou $R'_6$ représente respectivement un groupe précurseur de $R_1$, $R_2$, $R_3$, $R_5$ et/ou $R_6$, on soumet le composé obtenu, à l'étape 1, à un traitement ultérieur pour préparer le composé de formule (I) par transformation de l'un quelconque des groupes $R'_1$, $R'_2$, $R'_3$, $R'_5$ et/ou $R'_6$ en, respectivement, $R_1$, $R_2$, $R_3$, $R_5$ et/ou $R_6$ ;

4) éventuellement, on transforme le composé obtenu à l'étape 2/ ou à l'étape 3/, en l'un de sels

**5.** Composition pharmaceutique contenant à titre de principe actif, un composé selon l'une quelconque des revendications 1 à 3, ou un de ses sels pharmaceutiquement acceptables.

**6.** Composition pharmaceutique contenant un composé selon l'une quelconque des revendications 1 à 3 ou un de ses sels pharmaceutiquement acceptables, en association avec un autre principe actif.

**7.** Composition pharmaceutique contenant deux composés selon l'une quelconque des revendications 1 à 3, l'un étant un antagoniste spécifique du récepteur $V_1$ et l'autre étant un antagoniste spécifique du récepteur $V_2$.

**8.** Composition pharmaceutique contenant deux composés selon l'une quelconque des revendications 1 à 3, l'un étant un antagoniste spécifique du récepteur $V_1$ et l'autre étant un antagoniste spécifique de l'ocytocine.

Office européen des brevets

# RAPPORT DE RECHERCHE EUROPEENNE

Numero de la demande
EP 95 40 1599

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| A,D | WO-A-93 15051 (ELF SANOFI)<br>* page 2, ligne 18 - page 6, ligne 15 *<br>--- | 1,5 | C07D235/26<br>A61K31/415 |
| A,D | WO-A-91 05549 (OTSUKA PHARMACEUTICAL COMPANY, LIMITED)<br>* page 1 - page 7, alinéa 2 *<br>--- | 1,5 | |
| A,D | EP-A-0 477 819 (NEUROSEARCH A/S)<br>* page 2, colonne 2, ligne 5 - page 4, colonne 5, ligne 15 *<br>--- | 1,5 | |
| A,D | EP-A-0 051 827 (A. NATTERMANN & CIE, GMBH)<br>* page 1 - page 2, ligne 22 *<br>--- | 1,5 | |
| A,D | US-A-4 209 527 (REINHARD SARGES)<br>* colonne 1 - colonne 2, ligne 35 *<br>--- | 1,5 | |
| A,D | FR-A-1 573 739 (MANUFACTURES J. R. BOTTU)<br>* le document en entier *<br>----- | 1 | |

DOMAINES TECHNIQUES RECHERCHES (Int.Cl.6)

C07D

Le présent rapport a été établi pour toutes les revendications

| Lieu de la recherche | Date d'achèvement de la recherche | Examinateur |
|---|---|---|
| BERLIN | 29 Novembre 1995 | Kyriakakou, G |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)